(19) Europäisches Patentamt
European Patent Office
Office européen des brevets

(11) EP 4 803 550 A1

# (12) EUROPEAN PATENT APPLICATION
published in accordance with Art. 153(4) EPC

(43) Date of publication:
**09.09.2026 Bulletin 2026/37**

(21) Application number: **24884951.5**

(22) Date of filing: **01.11.2024**

(51) International Patent Classification (IPC):
***C07K 19/00*** (2006.01) ***A61K 39/095*** (2006.01)
***C12N 15/31*** (2006.01) ***C12N 1/21*** (2006.01)

(86) International application number:
**PCT/CN2024/129236**

(87) International publication number:
**WO 2025/092953 (08.05.2025 Gazette 2025/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.11.2023 CN 202311462742**

(71) Applicants:
- **Yantai Patronus Biotech Co., Ltd.
Yantai, Shandong 264670 (CN)**
- **Guangzhou Patronus Biotech Co., Ltd.
Guangzhou, Guangdong 510000 (CN)**

(72) Inventors:
- **TIAN, Siyu
Guangzhou, Guangdong 510000 (CN)**
- **JIN, Jing
Guangzhou, Guangdong 510000 (CN)**
- **LI, Yuanyuan
Guangzhou, Guangdong 510000 (CN)**
- **ZHANG, Senyan
Guangzhou, Guangdong 510000 (CN)**

(74) Representative: **karo IP
Patentanwälte PartG mbB
Steinstraße 16-18
40212 Düsseldorf (DE)**

Remarks:
The complete document including Reference Table(s) and the Sequence Listing(s) can be downloaded from the EPO website

## (54) IMMUNE COMPOSITION PRODUCT FOR PREVENTING OR TREATING DISEASES RELATED TO NEISSERIA MENINGITIDIS GROUP B, AND PREPARATION METHOD THEREFOR

(57) An immune composition product for preventing or treating diseases related to Neisseria meningitidis group B, and a preparation method therefor. The immune composition product comprises an fHbp fusion protein, wherein the fusion protein is constructed by means of disassembling two domains of three fHbp variants and then recombining same to construct a chimeric protein, and then on the basis of the chimeric protein, constructing a fusion protein containing characteristic amino acid sequences of the three fHbp variants. Furthermore, a nanoparticle vaccine is prepared by means of using such fusion protein as a vaccine antigen. The fusion protein has better immunological effects than those brought about by simply mixing the three fHbp variants.

RD012-1M
RD012-2M
260 kDa
140 kDa
100 kDa
70 kDa
50 kDa
40 kDa
35 kDa
25 kDa
15 kDa
RD012M
NPM

FIG. 1B

EP 4 803 550 A1

## Description

## TECHNICAL FIELD

The present disclosure relates to the field of biopharmaceutical technology, and in particular to an immune composition product for preventing or treating a disease related to *Neisseria meningitidis* group B and a method for preparing same.

## BACKGROUND

Meningococcal meningitis is an infectious disease caused by *N. meningitidis*. It spreads through the respiratory tract, features acute meningitis and septicemia as the main symptoms, and belongs to the class B infectious diseases specified in China. Based on the characteristics of the capsular polysaccharide, *N. meningitidis* can be divided into 13 serogroups: A, B, C, Y, W135, etc. The currently approved tetravalent meningococcal polysaccharide or polysaccharide-protein conjugate vaccines can effectively prevent pathogenic serogroups A, C, Y, and W135 and reduce the development of invasive meningococcal diseases.

*N. meningitidis* group B (MenB) is commonly known as meningococcus group B, which currently predominates in China due to the lack of effective prevention and control means. Unlike the capsular polysaccharides of serogroups A, C, Y, and W135, the major component of MenB capsular polysaccharides is structurally similar to a human central nervous system antigen N-acetylneuraminic acid polymer, which may lead to immune tolerance in humans to the MenB capsular polysaccharides and risks of inducing autoimmune diseases. Therefore, MenB capsular polysaccharides are improper for use as a candidate vaccine antigen.

Current attempts at MenB vaccine development mainly focus on non-capsular polysaccharide immunogens. Factor H-binding protein (fHBP), a lipoprotein expressed on the cell membrane of almost all MenB strains, is currently the most promising vaccine antigen. An important function of fHBP is to bind to human complement factor H, allowing *N. meningitidis* to evade the bactericidal effect in the host and improving its survival in the blood. Using fHBP as a MenB vaccine antigen, the elicited anti-fHBP antibodies can either directly activate the classical complement pathway to lyse the bacterium, or prevent fH from binding to the bacterial surface. fHBP is an antigen on the surface with highly diverse sequences and can be divided into 3 antigen variant groups: variant 1 (V1), variant 2 (V2), and variant 3 (V3), and the variants further include a large number of subvariants. The fHBP protein phylogenetic tree shows that the V1 variant accounts for about 70% among the isolates, the variants V2 and V3 account for about 30%, and there is no significant cross-protection reaction between the variant V1 and the other two variants V2 and V3. In addition, the definition of the fHBP protein structure varies with the assay method. MASCIONI et al. determined the full-length molecular structure of non-esterified fHBP protein in aqueous solutions by nuclear magnetic resonance, and revealed that the fHBP protein consists of 2 domains: a C-terminal anti-parallel β-barrel structure and a "taco-shaped" N-terminal β-sheet, which are connected via a 5’ residue flexible linker.

There are two predominant MenB vaccines approved overseas at present, Bexsero (GSK) and Trumenba (Pfizer). Bexsero (also known as C4MenB) a formulation comprising an outer membrane vesicle (OMV) from a prevalent strain of *N. meningitidis* group B in New Zealand, NZ98/254, together with five meningococcal antigens: Neisserial heparin-binding protein A (NHBA), factor H-binding protein (fHBP) variant 1.1, Neisserial adhesin A (NadA), and accessory proteins GNA1030 and GNA2091. Trumenba comprises two lipidated MenB fHBP antigens, A05 and B01, adsorbed on aluminum phosphate. The fHBP proteins in the 2 approved MenB protein vaccines above are all expressed in the form of a single variant or a fusion with a non-fHBP protein. The products target MenB strains prevalent in Europe and America, but do not provide extensive protection against other variant strains. The fHBP protein sequences in the approved MenB protein vaccines overseas are mainly directed against V1 and V3, while the fHBP type of the prevalent strains in China is mainly V2. Therefore, the approved MenB vaccines overseas cannot cover the prevalent strains in China.

Nanoparticle vaccines use nanomaterials as antigen carriers. Compared with conventional vaccines, nanoparticle vaccines have good antigen encapsulation effects and stable structures, and possess advantages in antigen assembly, antigen presentation, and the like. Nanoparticle vaccines mainly include 4 types: virus-like particle nanovaccines, self-assembled protein nanovaccines, polymer particle nanovaccines, and inorganic particle nanovaccines. In the field of self-assembled protein nanovaccines, researchers have recently found, based on the computational design of icosahedral nanocages, that the nanoparticle protein mi3 can spontaneously form highly ordered 60-subunit dodecahedral nanoparticles, and display SpyCatcher on the surface of the nanoparticles and link it to the antigen protein. The results confirm that this protein nanoparticle vaccine that is completely based on computational design can also cause a potent antibody response and is prospective in the preparation of novel self-assembled protein nanovaccines.

At present, no meningococcus group B vaccine is available in China, and very few *N. meningitidis* group B nanoparticle vaccine developments are ongoing. Since the two approved vaccines are mainly directed against MenB

strains prevalent in Europe and America, it is of great significance to develop MenB vaccines that are extensively protective, safer and more effective, and cost-efficient.

## SUMMARY

In order to solve the problem that the current *N. meningitidis* group B vaccine technology and supplied types are insufficient, particularly the lack of *N. meningitidis* group B nanoparticle vaccines, the present disclosure provides an *N. meningitidis* group B nanoparticle vaccine and a method for preparing same.

According to the present disclosure, structural alterations are made to the sequence of the fHBP antigen protein of MenB. Specifically, the 2 domains of three fHBP variants are disassembled and then recombined to construct a chimeric protein, and a fusion protein comprising characteristic amino acid sequences of the three fHBP variants is constructed on the basis of the chimeric protein. Using the fusion protein as the vaccine antigen, an extensively protective and effective MenB nanoparticle vaccine is prepared.

The nanoparticle vaccine of the present disclosure is a vaccine formed on the basis of the nanoparticle protein. The nanoparticle protein is mainly used for antigen display.

### A. Factor H-binding protein (fHBP) variants

The factor H-binding protein (fHBP), also known as GNA1870, GNA 1870, ORF2086, LP2086 (lipoprotein 2086), and "741" in the literature, refers to a class of *N. meningitidis* polypeptides that are lipoproteins on the surface of bacteria in nature. *N. meningitidis* strains are subdivided according to amino acid sequence variability and immunological cross-reactivity into three populations of fHBP variants: variant 1 (V1), variant 2 (V2), and variant (V3), which are further divided into subvariants fHbp-1.x, fHbp-2.x, and fHbp-3.x, wherein x represents a specific peptide subvariant, and chimeric variants such as v1-2,3.x are also present (see Structural characterization of a cross-protective natural chimera of factor H-binding protein from meningococcal serogroup B strain NL096, Computational and Structural Biotechnology Journal, vol. 20, 2070-2081, 18 April 2022; US9266942B2).

The present disclosure provides an fHBP variant 1, abbreviated as fHBP V1, comprising the amino acid sequence of amino acids 1-259 of SEQ ID NO: 7.

The present disclosure provides an fHBP variant 2, abbreviated as fHBP V2, comprising the amino acid sequence of amino acids 1-253 of SEQ ID NO: 8.

The present disclosure provides an fHBP variant 3, abbreviated as fHBP V3, comprising the amino acid sequence of amino acids 1-260 of SEQ ID NO: 9.

### B. fHBP chimeric proteins

The two fHBP domains involved in the present disclosure are designated as domain 1 and domain 2, wherein domain 1 corresponds to the N-terminal domain of fHBP known in the art, and domain 2 corresponds to the C-terminal domain of fHBP known in the art.

The present disclosure provides an fHBP chimeric protein, comprising different domains of different fHBP variants, wherein the domain is selected from domain 1 or domain 2 of fHBP V1, domain 1 or domain 2 of fHBP V2, or domain 1 or domain 2 of fHBP V3.

The fHBP V1 domain 1 provided herein has the following three forms:

1. fHBP V1 domain 1:

Amino acid sequence:

SSGGGGSGGGGVTADIGTGLADALTAPLDHKDKGLKSLTLEDSISQNGTLTLSAQGAEKTYGNGDSL NTGKLKNDKVSRFDFIRQIEVDGQLITLESGEFQVYKQSHSALTALQTEQEQDPEHSEKMVAKRRFRI GDIA (SEQ ID NO: 54);

Nucleotide sequence:

TCTAGCGGAGGTGGTGGTTCCGGTGGTGGCGGTGTTACCGCGGACATCGGTACCGGTTTGGCGGATGCGCTTACGGCACCGCTGGATCACAAAGACAAGGGTCTGAAGTCCTTGACCTTGGAGGACTCCATTTCGCAAAACGGCACCCTGACGCTTAGCGCTCAAGGCGCAGAAAAGACCTACGGCAATGGTGACTCACTGAACACCGGTAAACTGAAGAACGACAAAGTTAGCCGCTTCGACTTTATCCGTCAGATTGAGGTTGATGGTCAGCTGATTACCCTGGAAAGCGGGGAGTTTCAGGTATATAAGCAATCCCATTCCGCGCTGACTGCCCTGCAGACCGAACAAGAACAGGACCCGGAGCACTCCGAAAAAATGGTTGCAAAGCGCCGTTTTCGTATTGGTGACATTGCG (SEQ ID NO: 65);

2. fHBP V1 domain 1 truncated construct 1 (or fHBP V1 domain $1_{T1}$):

Amino acid sequence:

GSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIA (SEQ ID NO: 55);

Nucleotide sequence:

GGCAGCGGAGGTGGCGGTGTTACCGCGGACATTGGTACTGGTTTGGCGGACGCTCTGACTGCGCCACTGGACCACAAAGATAAGGGCTTGAAGTCATTGACCTTGGAGGATAGCATTAGCCAAAATGGCACCCTGACACTGTCTGCGCAGGGTGCCGAGAAGACCTACGGTAATGGGGACTCTCTGAACACTGGCAAACTTAAAAACGATAAGGTGAGCCGCTTCGATTTTATCCGTCAAATTGAAGTGGACGGTCAGCTGATTACGCTGGAGAGCGGCGAGTTTCAGGTTTATAAACAAAGCCATTCCGCTCTGACGGCTTTGCAAACCGAACAAGAACAGGACCCGGAGCACTCTGAAAAGATGGTGGCTAAACGTCGTTTCCGTATTGGCGACATTGCA (SEQ ID NO: 66);

3. fHBP V1 domain 1 truncated construct 2 (or fHBP V1 domain $1_{T2}$):

Amino acid sequence:

VTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIA (SEQ ID NO: 56);

Nucleotide sequence:

GTGACCGCGGACATCGGCACTGGTCTGGCTGATGCCCTCACTGCGCCTCTGGACCACAAGGATAAGGGTTTGAAGTCCCTCACCCTGGAGGACAGCATCAGCCAAAATGGTACGCTGACCCTGAGCGCTCAGGGCGCGGAGAAGACCTACGGTAATGGTGATTCTCTGAATACCGGTAAGCTGAAGAACGACAAGGTGTCTCGCTTCGACTTCATCCGTCAGATTGAGGTTGACGGCCAGCTGATTACCTTGGAAAGCGGTGAGTTCCAGGTTTATAAACAAAGCCATTCCGCGCTGACCGCGCTGCAAACCGAACAGGAACAGGATCCGGAGCACAGCGAAAAAATGGTCGCGAAGCGCCGCTTTCGTATCGGTGATATCGCA (SEQ ID NO: 67).

The amino acid and nucleotide sequences of fHBP V1 domain 2 provided herein are as follows:

Amino acid sequence:

GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQ (SEQ ID NO: 57);

Nucleotide sequence:

GGCGAACACACCAGCTTTGATAAGCTGCCAAAAGATGTCATGGCAACCTACCGTGGTACGGCGTTCGGCTCGGACGACGCGGGTGGCAAATTAACTTACACCATTGACTTTGCAGCGAAGCAGGGCCATGGCAAAATCGAGCACCTGAAGTCCCCGGAACTGAACGTGGACCTGGCAGTGGCGTACATCAAACCGGACGAAAAGCATCATGCTGTGATCAGCGGTTCGGTGCTGTATAACCAAGATGAGAAAGGCAGCTATAGCCTGGGTATTTTTGGCGAGAAGGCGCAGGAGGTGGCGGGTTCCGCCGAGGTTGAAACCGCGAATGGCATCCATCACATTGGGTTGGCGGCAAAACAG (SEQ ID NO: 68).

The amino acid and nucleotide sequences of fHBP V2 domain 1 provided herein are as follows:

Amino acid sequence:

SSGGGGVAADIGAGLADALTAPLDHKDKSLQSLTLDQSVRKNEKLKLAAQGAEKTYGNGDSLNTGKLKNDKVSRFDFIRQIEVDGQLITLESGEFQIYKQDHSAVVALQIEKINNPDKIDSLINQRSFLVSGLG (SEQ ID NO: 58);

Nucleotide sequence:

AGTTCAGGTGGAGGGGGAGTAGCAGCTGACATCGGTGCAGGCCTCGCAGACGCGCTGACCGCGCCGCTTGATCATAAGGATAAGAGCCTGCAGAGCCTGACATTGGATCAGTCCGTGCGTAAAAATGAAAAGTTGAAGCTAGCGGCCCAAGGTGCGGAAAAGACCTACGGCAACGGCGACTCCTTGAACACCGGTAAGCTGAAGAACGACAAGGTGAGCAGATTCGACTTCATCCGCCAGATCGAAGTCGACGGTCAGCTGATTACCTTGGAATCTGGCGAGTTTCAGATCTATAAGCAGGACCACAGCGCGGTCGTGGCCCTGCAGATTGAGAAGATCAACAACCCGGATAAGATCGATAGCTTGATCAATCAACGTAGCTTCCTGGTTAGTGGTTTGGGC (SEQ ID NO: 69).

The amino acid and nucleotide sequences of fHBP V2 domain 2 provided herein are as follows:

Amino acid sequence:

GEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQ (SEQ ID NO: 59);

Nucleotide sequence:

GGCGAGCACACCGCGTTCAACCAGCTGCCTGATGGTAAAGCCGAGTATCACGGCAAGGCCTTCAGCAGCGATGACGCCGGGGGTAAGTTAACGTACACCATTGACTTTGCCGCGAAGCAAGGTCACGGGAAAATCGAGCACCTGAAGACACCGGAGCAAAATGTCGAGTTGGCTGCGGCAGAGCTGAAGGCTGACGAAAAGAGCCATGCAGTCATCCTGGGCGATACCCGTTATGGTTCTGAAGAAAAGGGTACGTACCACCTGGCCTTGTTCGGTGATCGTGCCCAAGAGATCGCGGGCTCTGCTACCGTTAAAATCGGCGAAAAAGTGCATGAAATCGGTATCGCGGGTAAACAG (SEQ ID NO: 70).

The fHBP V3 domain 1 provided herein has the following four forms:

1. fHBP V3 domain 1:

Amino acid sequence:

SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG (SEQ ID NO: 60);

Nucleotide sequence:

TCTAGCGGTTCAGGAAGTGGCGGAGGGGGGGTGGCAGCTGACATCGGCACTGGTCTGGCGGACGCTCTGACCGCTCCGCTGGACCACAAGGACAAAGGTTTGAAAAGCCTGACTTTGGAAGATTCGATCTCTCAGAATGGTACGCTGACCCTCAGCGCGCAAGGCGCGGAGAAGACCTTTAAAGTTGGTGATA

AGGACAACAGCTTGAACACCGGTAAACTGAAAAACGATAAGATTAGCCGCTTCGACTTCGTGCAGAAAATCGAAGTGGACGGTCAGACCATTACCCTTGCGAGCGGCGAGTTCCAGATTTACAAGCAGGATCATAGTGCGGTTGTCGCATTACAAATCGAAAAAATCAATAATCCGGATAAGATTGATTCTCTCATCAACCAACGTAGCTTCTTGGTTAGCGGCCTGGGT (SEQ ID NO: 71).

2. fHBP V3 domain 1 truncated construct 1 (or fHBP V3 domain $1_{T1}$):

Amino acid sequence:

GSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG (SEQ ID NO: 61);

Nucleotide sequence:

GGTTCGGGTGGTGGCGGGGTGGCAGCCGATATCGGCACCGGGCTTGCTGACGCGCTCACGGCTCCGCTGGACCACAAAGACAAGGGTTTAAAGAGCCTGACCCTGGAAGATAGCATTTCCCAGAACGGCACCCTGACCCTGTCTGCACAAGGTGCGGAGAAAACCTTTAAAGTAGGTGATAAGGACAACAGCCTAAACACCGGTAAGCTGAAAAATGACAAGATCTCTCGTTTCGACTTTGTTCAGAAAATCGAGGTGGATGGTCAGACCATTACCTTGGCTAGCGGCGAGTTCCAGATCTATAAACAAGACCATAGCGCGGTCGTGGCGTTACAGATCGAGAAGATTAACAACCCGGATAAGATCGATTCGCTGATTAACCAGCGTAGCTTTCTGGTTTCCGGACTCGGT (SEQ ID NO: 72).

3. fHBP V3 domain 1 truncated construct 2 (or fHBP V3 domain $1_{T2}$):

Amino acid sequence:

VAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG (SEQ ID NO: 62);

Nucleotide sequence:

GTGGCAGCTGACATCGGCACTGGTCTGGCGGACGCTCTGACCGCTCCGCTGGACCACAAGGACAAAGGTTTGAAAAGCCTGACTTTGGAAGATTCGATCTCTCAGAATGGTACGCTGACCCTCAGCGCGCAAGGCGCGGAGAAGACCTTTAAAGTTGGTGATAAGGACAACAGCTTGAACACCGGTAAACTGAAAAACGATAAGATTAGCCGCTTCGACTTCGTGCAGAAAATCGAAGTGGACGGTCAGACCATTACCCTTGCGAGCGGCGAGTTCCAGATTTACAAGCAGGATCATAGTGCGGTTGTCGCATTACAAATCGAAAAAATCAATAATCCGGATAAGATTGATTCTCTCATCAACCAACGTAGCTTCTTGGTTAGCGGCCTGGGT (SEQ ID NO: 73).

4. fHBP V3 domain 1 truncated construct 3 (or fHBP V3 domain $1_{T3}$):

Amino acid sequence:

LNTGKLKNDKISRFDFVQKIEVDGQTITLASGEFQIYKQDHSAVVALQIEKINNPDKIDSLINQRSFLVSGLG (SEQ ID NO: 63);

Nucleotide sequence:

TTGAACACCGGTAAACTGAAAAACGATAAGATTAGCCGCTTCGACTTCGTGCAGAAAATCGAAGTGGACGGTCAGACCATTACCCTTGCGAGCGGCGAGTTCCAGATTTACAAGCAGGATCATAGTGCGGTTGTCGCATTACAAATCGAAAAAATCAATAATCCGGATAAGATTGATTCTCTCATCAACCAACGTAGCTTCTTGGTTAGCGGCCTGGGT (SEQ ID NO: 74).

The amino acid and nucleotide sequences of fHBP V3 domain 2 provided herein are as follows:

Amino acid sequence:

GEHTAFNQLPSGKAEYHGKAFSSDDAGGKLTYTIDFAAKQGHGKIEHLKTPEQNVELASAELKADEKSHAVILGDTRYGSEEKGTYHLALFGDRAQEIAGSATVKIREKVHEIGIAGKQ (SEQ ID NO: 64);

Nucleotide sequence:

GGCGAACACACCGCGTTCAACCAGCTGCCGAGCGGTAAGGCTGAGTATCACGGCAAGGCGTTTAGCAGCGACGACGCAGGTGGTAAGCTGACGTACACCATTGACTTCGCCGCGAAACAAGGTCATGGTAAGATTGAGCATCTGAAAACTCCGGAGCAAAATGTTGAATTGGCGAGCGCTGAGCTGAAGGCAGATGAAAAGAGCCACGCAGTGATTCTGGGTGACACCCGTTATGGTTCGGAGGAAAAAGGCACCTACCATCTGGCATTGTTTGGCGACCGCGCACAGGAGATCGCGGGTTCGGCGACCGTTAAAATTCGTGAAAAAGTTCATGAAATCGGCATCGCTGGCAAGCAG (SEQ ID NO: 75).

In some embodiments, the amino acid sequence of fHBP V1 domain 1 provided herein has 40% or higher, 50% or higher, 60% or higher, 70% or higher, 80% or higher, or 90% or higher identity to SEQ ID NO: 54, 55, or 56, and is derived from an fHBP V1 subvariant.

In some embodiments, the amino acid sequence of fHBP V1 domain 2 provided herein has 40% or higher, 50% or higher, 60% or higher, 70% or higher, 80% or higher, or 90% or higher identity to SEQ ID NO: 57, and is derived from an fHBP V1 subvariant.

Preferably, the fHBP V1 subvariant is selected from v1.1, v1.4, v1.13, v1.15, v1.14, v1.10, v1.260, v1.510, v1.90, v1.275, v1.697, v1.226, v1.110, v1.249, v1.108, v1.227, v1.215, and v1-2,3.x, or other known fHBPV1 subvariants.

In some embodiments, the amino acid sequence of fHBP V2 domain 1 provided herein has 40% or higher, 50% or higher, 60% or higher, 70% or higher, 80% or higher, or 90% or higher identity to SEQ ID NO: 58, and is derived from an fHBP V2 subvariant.

In some embodiments, the amino acid sequence of fHBP V2 domain 2 provided herein has 40% or higher, 50% or higher, 60% or higher, 70% or higher, 80% or higher, or 90% or higher identity to SEQ ID NO: 59, and is derived from an fHBP V2 subvariant.

Preferably, the fHBP V2 subvariant is selected from v2.16, v2.19, v2.21, v2.22, v2.24, and v1-2,3.x, or other known fHBPV2 subvariants.

In some embodiments, the amino acid sequence of fHBP V3 domain 1 provided herein has 40% or higher, 50% or higher, 60% or higher, 70% or higher, 80% or higher, or 90% or higher identity to SEQ ID NO: 60, 61, 62, or 63, and is derived

from an fHBP V3 subvariant.

In some embodiments, the amino acid sequence of fHBP V3 domain 2 provided herein has 40% or higher, 50% or higher, 60% or higher, 70% or higher, 80% or higher, or 90% or higher identity to SEQ ID NO: 64, and is derived from an fHBP V3 subvariant.

Preferably, the fHBP V3 subvariant is selected from v3.116, v3.28, v3.31, v3.45, v3.42, and v1-2,3.x, or other known fHBPV3 subvariants.

In some embodiments, the fHBP chimeric protein provided herein comprises, from the N-terminus to the C-terminus, fHBP V3 domain 1 and fHBP V1 domain 2.

In some embodiments, the fHBP chimeric protein provided herein comprises, from the N-terminus to the C-terminus, fHBP V1 domain 1 and fHBP V2 domain 2.

In some embodiments, the fHBP chimeric protein provided herein comprises, from the N-terminus to the C-terminus, fHBP V2 domain 1 and fHBP V1 domain 2.

In some embodiments, the fHBP chimeric protein provided herein comprises, from the N-terminus to the C-terminus, fHBP V1 domain 1 and fHBP V3 domain 2.

In some embodiments, the fHBP chimeric protein provided herein can induce the production of a neutralizing antibody, a protective antibody, or a bactericidal antibody.

In some embodiments, the fHBP chimeric protein provided herein is used to prepare a neutralizing antibody, a protective antibody, or a bactericidal antibody.

The neutralizing antibody, the protective antibody, or the bactericidal antibody described above refers to an antibody, preferably IgG, that can bind to a corresponding living bacterium and then activate the complement system, resulting in the lysis and death of the bacterium.

C. **fHBP** fusion proteins

The present disclosure provides an fHBP fusion protein, comprising two different fHBP chimeric proteins, wherein the two fHBP chimeric proteins may be linked in tandem via a linker, and the fHBP fusion protein may simultaneously induce antibodies against fHBP V1, V2, and V3.

In some embodiments, the fHBP fusion protein provided herein is formed by connecting two fHBP chimeric proteins in tandem, and the specific structure is fHBP V3 domain 1-fHBP V1 domain 2-fHBP V1 domain 1-fHBP V2 domain 2.

Preferably, the structure of the fHBP fusion protein is fHBP V3 domain 1-fHBP V1 domain 2-linker-fHBP V1 domain 1-fHBP V2 domain 2.

In some embodiments, the fHBP fusion protein provided herein is formed by connecting two fHBP chimeric proteins in tandem, and the specific structure is fHBP V1 domain 1-fHBP V2 domain 2-fHBP V3 domain 1-fHBP V1 domain 2.

Preferably, the structure of the fHBP fusion protein is fHBP V1 domain 1-fHBP V2 domain 2-linker-fHBP V3 domain 1-fHBP V1 domain 2.

In some embodiments, the fHBP fusion protein provided herein is formed by connecting two fHBP chimeric proteins in tandem, and the specific structure is fHBP V2 domain 1-fHBP V1 domain 2-fHBP V1 domain 1-fHBP V3 domain 2.

Preferably, the structure of the fHBP fusion protein is fHBP V2 domain 1-fHBP V1 domain 2-linker-fHBP V1 domain 1-fHBP V3 domain 2.

The linker described above is any linker peptide commonly used in the art, such as a flexible linker peptide, a rigid linker peptide, or a semi-rigid linker peptide, including but not limited to, the amino acid sequence of $G_n$, GSGGGG, $(EAAAK)_n$, or GGSGGEAAAK, wherein n may be an integer greater than 0 and less than or equal to 10, and preferably, n is 1, 2, 3, or 4.

In some embodiments, the fHBP fusion protein provided herein comprises the amino acid sequence set forth in any one of (1) to (9) below:

(1) the amino acid sequence at positions 1-520 of SEQ ID NO: 1;
(2) the amino acid sequence at positions 1-514 of SEQ ID NO: 2;
(3) the amino acid sequence at positions 1-518 of SEQ ID NO: 17;
(4) the amino acid sequence at positions 1-507 of SEQ ID NO: 18;
(5) the amino acid sequence at positions 1-523 of SEQ ID NO: 19;
(6) the amino acid sequence at positions 1-528 of SEQ ID NO: 20;
(7) the amino acid sequence at positions 1-511 of SEQ ID NO: 21;
(8) the amino acid sequence at positions 1-516 of SEQ ID NO: 22; or
(9) the amino acid sequence at positions 1-521 of SEQ ID NO: 23.

In some embodiments, the fHBP fusion protein provided herein can induce the production of a neutralizing antibody, a protective antibody, or a bactericidal antibody.

In some embodiments, the fHBP fusion protein provided herein is used to prepare a neutralizing antibody, a protective antibody, or a bactericidal antibody.

The neutralizing antibody, the protective antibody, or the bactericidal antibody described above refers to an antibody, preferably IgG, that can bind to a corresponding living bacterium and then activate the complement system, resulting in the lysis and death of the bacterium.

**D. Immunogenic complex**

The present disclosure provides an immunogenic complex, comprising a protein formed by a covalent binding reaction between an antigenic component and a particle protein.

The antigenic component of the immunogenic complex of the present disclosure comprises an fHBP protein selected from the fHBP chimeric protein, fHBP fusion protein, or fHBP variant described above, wherein the fHBP variant is selected from fHBP V1, fHBP V2, or fHBP V3.

The present disclosure provides an immunogenic complex, comprising:

(1) an antigenic component, comprising an fHBP protein; and
(2) a particle protein component, comprising a nanoparticle protein.

The present disclosure provides an immunogenic complex, comprising:

(1) an antigenic component, comprising an fHBP protein and a binding peptide 1; and
(2) a particle protein component, comprising a nanoparticle protein and a binding peptide 2.

The present disclosure provides an immunogenic complex, comprising:

(1) an antigenic component, comprising an fHBP protein, a linker peptide 1, and a binding peptide 1; and
(2) a particle protein component, comprising a nanoparticle protein, a linker peptide 2, and a binding peptide 2.

In some embodiments, in any one of the immunogenic complexes provided herein, the antigenic component is formed by fusing the fHBP protein, at the C-terminus, with the binding peptide 1 via the linker peptide 1.

In some embodiments, in any one of the immunogenic complexes provided herein, the particle protein component is formed by fusing the nanoparticle protein, at the N-terminus, with the binding peptide 2 via the linker peptide 2.

In some embodiments, the antigenic component and the particle protein component are covalently bound to each other via the binding peptide 1 and the binding peptide 2 to form the immunogenic complex.

In some embodiments, in any one of the immunogenic complexes provided herein, the binding peptide 1 comprises the amino acid sequence set forth in AHIVMVDAYKPTK (SEQ ID NO: 47), which is referred to as "4T" hereinafter.

In some embodiments, in any one of the immunogenic complexes provided herein, the binding peptide 2 comprises the amino acid sequence set forth in DSATHIKFSKRDEDGKELAGATMELRDSSGKTISTWISDGQVKDFYL YPGKYTFVETAAPDGYEVATAITFTVNEQGQVTVNGKATKGDAHI (SEQ ID NO: 48), which is referred to as "4C" hereinafter.

In some embodiments, both the antigenic component and the particle protein component comprise a histidine tag.

In some embodiments, in any one of the immunogenic complexes provided herein, the particle protein may be selected from a nanoparticle protein, and further may be selected from a virus-like particle protein; both the antigenic component and the particle protein component can self-assemble by binding to form a particle structure. In some embodiments, the nanoparticle used in the present disclosure that can self-assemble includes: an NPM particle, a ferritin particle, a virus-like particle formed by a viral structural protein, an I53-50 particle, and the like. The viral structural protein includes bacteriophage capsid protein AP205 and the like.

In some embodiments, in any one of the immunogenic complexes provided herein, the fHBP protein comprises the amino acid sequence set forth in any one of SEQ ID NO: 1-23.

Preferably, in any one of the immunogenic complexes provided herein, the fHBP protein comprises the amino acid sequence set forth in any one of SEQ ID NO: 1, 2, 5-9, 12, 13, and 17-33.

Table 1: Structures of antigenic components (including control molecules) and particle protein components of the present disclosure

| Molecule species | Molecule No. | Molecular designation | Description | Polypeptide sequence /nucleic acid sequence |
|---|---|---|---|---|
| Antigenic component | RD012-1 | fHBP V3 domain 1-fHBP V1 domain 2-(G)-fHBP V1 domain 1-fHBP V2 domain 2-(GSG)2-4T-His | Fusion protein comprising characteristic | SEQ ID NO:1 / SEQ ID NO:24 |
| | RD012-2 | fHBP V3 domain 1-fHBP V1 domain 2-fHBP V1 domain 1T1-fHBP V2 domain 2-(GSG)2-4T-His | amino acid sequences of three fHBP variants | SEQ ID NO:2 / SEQ ID NO:25 |
| | RD012-3 | His-fHBP V3 domain 1-fHBP V1 domain 2-(G)-fHBP V1 domain 1-fHBP V2 domain 2 | RD012-1 fusion protein with the removal of 4T sequence and transfer of His tag from C-terminus to N-terminus | SEQ ID NO:3 / SEQ ID NO:26 |
| | RD012-4 | His-fHBP V3 domain 1-fHBP V1 domain 2-(GSG) 4-4T-(G)-fHBP V1 domain 1-fHBP V2 domain 2 | With 4T sequence between two chimeric proteins | SEQ ID NO:4 / SEQ ID NO:27 |
| | RD012-5 | fHBP V3 domain 1-fHBP V1 domain 2 -(GSG)2-4T-His | Chimeric protein constructed by separation and recombination of fHBP domains | SEQ ID NO:5 / SEQ ID NO:28 |
| | RD012-6 | fHBP V1 domain 1-fHBP V2 domain 2 -(GSG)2-4T-His | | SEQ ID NO:6 / SEQ ID NO:29 |
| | RD012-7 | fHBP V1-(GSG) 2-4T-His | Single fHBP variant protein | SEQ ID NO:7 / SEQ ID NO:30 |
| | RD012-8 | fHBP V2-(GSG) 2-4T-His | | SEQ ID NO:8 / SEQ ID NO:31 |
| | RD012-9 | fHBP V3-(GSG) 2-4T-His | | SEQ ID NO:9 / SEQ ID NO:32 |
| | RD012-13 | His-fHBP V1 domain 1-fHBP V2 domain 2 E121G | Equal mixture of RD012-13 and RD012-14 as control molecule | SEQ ID NO:10 / SEQ ID NO:33 |
| | RD012-14 | His-fHBP V1 domain 1-fHBP V2 domain 2 E121G, A174K, K180R | | SEQ ID NO:11 / SEQ ID NO:34 |

(continued)

| Molecule species | Molecule No. | Molecular designation | Description | Polypeptide sequence /nucleic acid sequence |
|---|---|---|---|---|
| | RD012-15 | fHBP V1 domain 1-fHBP V2 domain 2-(GG)-fHBP V3 domain 1-fHBP V1 domain 2-(GSG) 2-4T-His | RD012-1 fusion protein with changed order of chimeric proteins | SEQ ID NO:12 / SEQ ID NO:35 |
| | RD012-16 | fHBP V1 domain 1-fHBP V2 domain 2-fHBP V3 domain 1T1-fHBP V1 domain 2-(GSG)2-4T-His | RD012-2 fusion protein with changed order of chimeric proteins | SEQ ID NO:13 / SEQ ID NO:36 |
| | RD012-17 | fHBP V3 domain 1T2-fHBP V1 domain 2-(G)-fHBP V1 domain 1-fHBP V2 domain 2-(GSG) 2-4T-His | RD012-1 fusion protein with N-terminal amino acid sequence truncation | SEQ ID NO:14 / SEQ ID NO:37 |
| | RD012-18 | fHBP V3 domain 1T3-fHBP V1 domain 2-(G)-fHBP V1 domain 1-fHBP V2 domain 2-(GSG) 2-4T-His | RD012-1 fusion protein with N-terminal split amino acid sequence truncation | SEQ ID NO:15 / SEQ ID NO:38 |
| | RD012-19 | fHBP V3 domain 1-fHBP V1 domain 2-GGSGGEAAAK-fHBP V1 domain 1 T2-fHBP V2 domain 2-(GSG)2-4T-His | RD012-1 fusion protein with replacement of flexible linker between two chimeric proteins by semi-rigid linker: GGSGGEAAAK | SEQ ID NO:16 / SEQ ID NO:39 |
| | RD012-20 | fHBP V3 domain 1-fHBP V1 domain 2-(EAAAK)2-fHBP V1 domain 1 T2-fHBP V2 domain 2-(GSG)2-4T-His | RD012-1 fusion protein with replacement of flexible linker between two chimeric proteins by rigid linker | SEQ ID NO:17 / SEQ ID NO:40 |
| | RD012-21 | fHBP V2 domain 1-fHBP V1 domain 2-fHBP V1 domain 1T1-fHBP V3 domain 2-(GSG)2-4T-His | RD012-2 chimeric protein with respective replacement of fHBP V3 domain 1 and fHBP V2 domain 2 by fHBP V2 domain 1 and fHBP V3 domain 2 | SEQ ID NO:18 / SEQ ID NO:41 |

(continued)

| Molecule species | Molecule No. | Molecular designation | Description | Polypeptide sequence /nucleic acid sequence |
|---|---|---|---|---|
| | RD012-22 | fHBP V3 domain 1-fHBP V1 domain 2-(EAAAK)3-fHBP V1 domain 1 T2-fHBP V2 domain 2-(GSG)2-4T-His | RD012-1 fusion protein with replacement of flexible linker between two chimeric proteins by rigid linker | SEQ ID NO:19 / SEQ ID NO:42 |
| | RD012-23 | fHBP V3 domain 1-fHBP V1 domain 2-(EAAAK)4-fHBP V1 domain 1 T2-fHBP V2 domain 2-(GSG)2-4T-His | | SEQ ID NO:20 / SEQ ID NO:43 |
| | RD012-24 | fHBP V2 domain 1-fHBP V1 domain 2-(EAAAK)2-fHBP V1 domain 1 T2-fHBP V3 domain 2-(GSG)2-4T-His | RD012-21 fusion protein with replacement of flexible linker between two chimeric proteins by rigid linker | SEQ ID NO:21 / SEQ ID NO:44 |
| | RD012-25 | fHBP V2 domain 1-fHBP V1 domain 2-(EAAAK)3-fHBP V1 domain 1 T2-fHBP V3 domain 2-(GSG)2-4T-His | | SEQ ID NO:22 / SEQ ID NO:45 |
| | RD012-26 | fHBP V2 domain 1-fHBP V1 domain 2-(EAAAK)4-fHBP V1 domain 1 T2-fHBP V3 domain 2-(GSG)2-4T-His | | SEQ ID NO:23 / SEQ ID NO:46 |

(continued)

| Molecule species | Molecule No. | Molecular designation | Description | Amino acid sequence of immunogenic complex (antigenic component + particle protein component) |
|---|---|---|---|---|
| Immunogenic complex | RD012-1M | fHBP V3 domain 1-fHBP V1 domain 2-(G)-fHBP V1 domain 1-fHBP V2 domain 2-NPM | The antigenic component is conjugated to the NPM particle to construct the nanoparticle vaccines, and the amino acid sequence of the particle protein component used is set forth in SEQ ID NO: 52 | SEQ ID NO:1+ SEQ ID NO:52 |
| | RD012-2M | fHBP V3 domain 1-fHBP V1 domain 2-fHBP V1 domain 1T1-fHBP V2 domain 2-NPM | | SEQ ID NO:2+ SEQ ID NO:52 |
| | RD012-4M | His-fHBP V3 domain 1-fHBP V1 domain 2-(GSG)4-NPM-(G)-fHBP V1 domain 1-fHBP V2 domain 2 | | SEQ ID NO:4+ SEQ ID NO:52 |
| | RD012-5M | fHBP V3 domain 1-fHBP V1 domain 2-(GSG)2-NPM | | SEQ ID NO:5+ SEQ ID NO:52 |
| | RD012-6M | fHBP V1 domain 1-fHBP V2 domain 2-(GSG)2-NPM | | SEQ ID NO:6+ SEQ ID NO:52 |
| | RD012-7M | fHBP V1-(GSG)2-NPM | | SEQ ID NO:7+ SEQ ID NO:52 |
| | RD012-8M | fHBP V2-(GSG)2-NPM | | SEQ ID NO:8+ SEQ ID NO:52 |
| | RD012-9M | fHBP V3-(GSG)2-NPM | | SEQ ID NO:9+ SEQ ID NO:52 |
| | RD012-15M | fHBP V1 domain 1-fHBP V2 domain 2-(GG)-fHBP V3 domain 1-fHBP V1 domain 2-NPM | | SEQ ID NO:12+ SEQ ID NO:52 |
| | RD012-16M | fHBP V1 domain 1-fHBP V2 domain 2-fHBP V3 domain 1 T1-fHBP V1 domain 2-NPM | | SEQ ID NO:13+ SEQ ID NO:52 |

(continued)

| Molecule species | Molecule No. | Molecular designation | Description | Amino acid sequence of immunogenic complex (antigenic component + particle protein component) |
|---|---|---|---|---|
| | RD012-17M | fHBP V3 domain 1 T2-fHBP V1 domain 2-(G)-fHBP V1 domain 1-fHBP V2 domain 2-NPM | | SEQ ID NO:14+ SEQ ID NO:52 |
| | RD012-18M | fHBP V3 domain 1 T3-fHBP V1 domain 2-(G)-fHBP V1 domain 1-fHBP V2 domain 2-NPM | | SEQ ID NO:15+ SEQ ID NO:52 |
| | RD012-19M | fHBP V3 domain 1-fHBP V1 domain 2-GGSGGEAAAK-fHBP V1 domain 1 T2-fHBP V2 domain 2-NPM | | SEQ ID NO:16+ SEQ ID NO:52 |
| | RD012-20M | fHBP V3 domain 1-fHBP V1 domain 2-(EAAAK)2-fHBP V1 domain 1 T2-fHBP V2 domain 2-NPM | | SEQ ID NO:17+ SEQ ID NO:52 |
| | RD012-21M | fHBP V2 domain 1-fHBP V1 domain 2-fHBP V1 domain 1 T1-fHBP V3 domain 2-NPM | | SEQ ID NO:18+ SEQ ID NO:52 |
| | RD012-22M | fHBP V3 domain 1-fHBP V1 domain 2-(EAAAK)3-fHBP V1 domain 1 T2-fHBP V2 domain 2-NPM | | SEQ ID NO:19+ SEQ ID NO:52 |
| | RD012-23M | fHBP V3 domain 1-fHBP V1 domain 2-(EAAAK)4-fHBP V1 domain 1 T2-fHBP V2 domain 2-NPM | | SEQ ID NO:20+ SEQ ID NO:52 |
| | RD012-24M | fHBP V2 domain 1-fHBP V1 domain 2-(EAAAK)2-fHBP V1 domain 1 T2-fHBP V3 domain 2-NPM | | SEQ ID NO:21+ SEQ ID NO:52 |

(continued)

| Molecule species | Molecule No. | Molecular designation | Description | Amino acid sequence of immunogenic complex (antigenic component + particle protein component) |
|---|---|---|---|---|
| | RD012-25M | fHBP V2 domain 1-fHBP V1 domain 2-(EAAAK)3-fHBP V1 domain 1 T2-fHBP V3 domain 2-NPM | | SEQ ID NO:22+ SEQ ID NO:52 |
| | RD012-26M | fHBP V2 domain 1-fHBP V1 domain 2-(EAAAK)4-fHBP V1 domain 1 T2-fHBP V3 domain 2-NPM | | SEQ ID NO:23+ SEQ ID NO:52 |

The nucleic acid sequence corresponding to the amino acid sequence set forth in any one of SEQ ID NOs: 1-23 described above is set forth in any one of SEQ ID NOs: 24-46, and the amino acid sequences set forth in SEQ ID NOs: 1-23 are listed in Table 2. The nucleic acid sequences set forth in SEQ ID NOs: 24-46 are listed in Table 3.

Table 2: Amino acid sequences set forth in SEQ ID NOs: 1-23 (**RD012-1 to RD012-9 and RD012-13 to RD012-26 molecules**)

| Polypeptide name | Sequence |
|---|---|
| **RD012-1** | **SSGSGSGGGGVAADIGTGLADALTAPLDHKDKGLKSLTLEDSISQNGTLTLSAQGAEKTFKVGDKD**<br>**NSLNTGKLKNDKISRFDFVQKIEVDGQTITLASGEFQIYKQDHSAVVALQIEKINNPDKIDSLINQRS**<br>**FLVSGLG**GEHTSFDKLPKDVMATYRGTAFGSDDAGGKLTYTIDFAAKQGHGKIEHLKSPELNVDLAVA<br>YIKPDEKHHAVISGSVLYNQDEKGSYSLGIFGEKAQEVAGSAEVETANGIHHIGLAAKQGSSGGGGSGG<br>GGVTADIGTGLADALTAPLDHKDKGLKSLTLEDSISQNGTLTLSAQGAEKTYGNGDSLNTGKLKNDKVS<br>RFDFIRQIEVDGQLITLESGEFQVYKQSHSALTALQTEQEQDPEHSEKMVAKRRFRIGDIAGEHTAFNQLP<br>DGKAEYHGKAFSSDDAGGKLTYTIDFAAKQGHGKIEHLKTPEQNVELAAAELKADEKSHAVILGDTRY<br>GSEEKGTYHLALFGDRAQEIAGSATVKIGEKVHEIGIAGKQGSGGSGAHIVMVDAYKPTKHHHHHH<br>(SEQ ID NO:1) |
| **RD012-2** | **SSGSGSGGGGVAADIGTGLADALTAPLDHKDKGLKSLTLEDSISQNGTLTLSAQGAEKTFKVGDKD**<br>**NSLNTGKLKNDKISRFDFVQKIEVDGQTITLASGEFQIYKQDHSAVVALQIEKINNPDKIDSLINQRS**<br>**FLVSGLG**GEHTSFDKLPKDVMATYRGTAFGSDDAGGKLTYTIDFAAKQGHGKIEHLKSPELNVDLAVA<br>YIKPDEKHHAVISGSVLYNQDEKGSYSLGIFGEKAQEVAGSAEVETANGIHHIGLAAKQGSGGGGVTADI<br>GTGLADALTAPLDHKDKGLKSLTLEDSISQNGTLTLSAQGAEKTYGNGDSLNTGKLKNDKVSRFDFIRQI<br>EVDGQLITLESGEFQVYKQSHSALTALQTEQEQDPEHSEKMVAKRRFRIGDIAGEHTAFNQLPDGKAEY<br>HGKAFSSDDAGGKLTYTIDFAAKQGHGKIEHLKTPEQNVELAAAELKADEKSHAVILGDTRYGSEEKGT<br>YHLALFGDRAQEIAGSATVKIGEKVHEIGIAGKQGSGGSGAHIVMVDAYKPTKHHHHHH (SEQ ID NO:2) |

(continued)

| Polypeptide name | Sequence |
|---|---|
| **RD012-3** | HHHHHH**SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGSSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQ (SEQ ID NO:3) |
| **RD012-4** | HHHHHH**SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGGSGGSGGSGGSAHIVMVDAYKPTKGSSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQ (SEQ ID NO:4) |
| **RD012-5** | **SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGSGGSGAHIVMVDAYKPTKHHHHHH (SEQ ID NO:5) |

(continued)

| Polypeptide name | Sequence |
|---|---|
| **RD012-6** | SSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGSGGSGAHIVMVDAYKPTKHHHHHH(SEQ ID NO:6) |
| **RD012-7** | SSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGSGGSGAHIVMVDAYKPTKHHHHHH(SEQ ID NO:7) |
| **RD012-8** | SSGGGGVAADIGAGLADALTAPLDHKDKSLQSLTLDQSVRKNEKLKLAAQGAEKTYGNGDSLNTGKLKNDKVSRFDFIRQIEVDGQLITLESGEFQIYKQDHSAVVALQIEKINNPDKIDSLINQRSFLVSGLGGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGSGGSGAHIVMVDAYKPTKHHHHHH (SEQ ID NO:8) |
| **RD012-9** | SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLGGEHTAFNQLPSGKAEYHGKAFSSDDAGGKLTYTIDFAAKQGHGKIEHLKTPEQNVELASAELKADEKSHAVILGDTRYGSEEKGTYHLALFGDRAQEIAGSATVKIREKVHEIGIAGKQGSGGSGAHIVMVDAYKPTKHHHHHH (SEQ ID NO:9) |

(continued)

| Polypeptide name | Sequence |
|---|---|
| **RD012-13** | HHHHHHSSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHS**G**KMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQ (SEQ ID NO:10) |
| **RD012-14** | HHHHHHSSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHS**G**KMVAKRRFRIGDIAGEHTAFNQLPDGKAEYHGKAFSSDDAGGKLTYTIDFA**K**KQGHG**R**IEHLKTPEQNVELAAAELKADEKSHAVILGDTRYGSEEKGTYHLALFGDRAQEIAGSATVKIGEKVHEIGIAGKQ (SEQ ID NO:11) |
| **RD012-15** | SSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGG**SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGSGGSGAHIVMVDAYKPTKHHHHHH (SEQ ID NO:12) |
| **RD012-16** | SSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQ**GSGGGGVAADIGTGLAD** |

(continued)

| Polypeptide name | Sequence |
|---|---|
| | **ALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGSGGSGAHIVMVDAYKPTKHHHHHH (SEQ ID NO:13) |
| **RD012-17** | **VAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGSSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGSGGSGAHIVMVDAYKPTKHHHHHH (SEQ ID NO:14) |
| **RD012-18** | **LNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGSSGGGGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGSGGSGAHIVMVDAYKPTKHHHHHH (SEQ ID NO:15) |

(continued)

| Polypeptide name | Sequence |
|---|---|
| **RD012-19** | **SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGGSGGEAAAKVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGSGGSGAHIVMVDAYKPTKHHHHHH (SEQ ID NO:16) |
| **RD012-20** | **SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQEAAAKEAAAKVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGSGGSGAHIVMVDAYKPTKHHHHHH (SEQ ID NO:17) |

(continued)

| Polypeptide name | Sequence |
|---|---|
| **RD012-21** | **SSGGGGVAADIGAGLADALTAPLDHKDKSLQSLTLDQSVRKNEKLKLAAQGAEKTYGNGDSLNTGKLKNDKVSRFDFIRQIEVDGQLITLESGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQGSGGGGVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPSGKAEYHGKAFSSDDAGGKLTYTIDFAAKQGHGKIEHLKTPEQNVELASAELKADEKSHAVILGDTRYGSEEKGTYHLALFGDRAQEIAGSATVKIREKVHEIGIAGKQGSGGSGAHIVMVDAYKPTKHHHHHH (SEQ ID NO:18) |
| **RD012-22** | **SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQEAAAKEAAAKEAAAKVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGSGGSGAHIVMVDAYKPTKHHHHHH (SEQ ID NO:19) |

(continued)

| Polypeptide name | Sequence |
|---|---|
| **RD012-23** | **SSGSGSGGGGVAADIGTGLADALTAPLDHKDKGLKSLTLEDSISQNGTLTLSAQGAEKTFKVGDKDNSLNTGKLKNDKISRFDFVQKIEVDGQTITLASGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQEAAAKEAAAKEAAAKEAAAKVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPDGKAEYHGKAFSSDDAGGKLTYTIDFAAKQGHGKIEHLKTPEQNVELAAAELKADEKSHAVILGDTRYGSEEKGTYHLALFGDRAQEIAGSATVKIGEKVHEIGIAGKQGSGGSGAHIVMVDAYKPTKHHHHHH (SEQ ID NO:20) |
| **RD012-24** | **SSGGGGVAADIGAGLADALTAPLDHKDKSLQSLTLDQSVRKNEKLKLAAQGAEKTYGNGDSLNTGKLKNDKVSRFDFIRQIEVDGQLITLESGEFQIYKQDHSAVVALQIEKINNPDKIDSLINQRSFLVSGLG**GEHTSFDKLPKDVMATYRGTAFGSDDAGGKLTYTIDFAAKQGHGKIEHLKSPELNVDLAVAYIKPDEKHHAVISGSVLYNQDEKGSYSLGIFGEKAQEVAGSAEVETANGIHHIGLAAKQEAAAKEAAAKVTADIGTGLADALTAPLDHKDKGLKSLTLEDSISQNGTLTLSAQGAEKTYGNGDSLNTGKLKNDKVSRFDFIRQIEVDGQLITLESGEFQVYKQSHSALTALQTEQEQDPEHSEKMVAKRRFRIGDIAGEHTAFNQLPSGKAEYHGKAFSSDDAGGKLTYTIDFAAKQGHGKIEHLKTPEQNVELASAELKADEKSHAVILGDTRYGSEEKGTYHLALFGDRAQEIAGSATVKIREKVHEIGIAGKQGSGGSGAHIVMVDAYKPTKHHHHHH (SEQ ID NO:21) |
| **RD012-25** | **SSGGGGVAADIGAGLADALTAPLDHKDKSLQSLTLDQSVRKNEKLKLAAQGAEKTYGNGDSLNTG** |

(continued)

| Polypeptide name | Sequence |
|---|---|
| | **KLKNDKVSRFDFIRQIEVDGQLITLESGEFQIYKQDHSAVVALQIEKINNPDKIDSLINQRSFLVSGL**<br>**G**GEHTSFDKLPKDVMATYRGTAFGSDDAGGKLTYTIDFAAKQGHGKIEHLKSPELNVDLAVAYIKPDEK<br>HHAVISGSVLYNQDEKGSYSLGIFGEKAQEVAGSAEVETANGIHHIGLAAKQEAAAKEAAAKEAAAKV<br>TADIGTGLADALTAPLDHKDKGLKSLTLEDSISQNGTLTLSAQGAEKTYGNGDSLNTGKLKNDKVSRFD<br>FIRQIEVDGQLITLESGEFQVYKQSHSALTALQTEQEQDPEHSEKMVAKRRFRIGDIAGEHTAFNQLPSGK<br>AEYHGKAFSSDDAGGKLTYTIDFAAKQGHGKIEHLKTPEQNVELASAELKADEKSHAVILGDTRYGSEE<br>KGTYHLALFGDRAQEIAGSATVKIREKVHEIGIAGKQGSGGSGAHIVMVDAYKPTKHHHHHH (SEQ ID<br>NO:22) |
| **RD012-26** | **SSGGGGVAADIGAGLADALTAPLDHKDKSLQSLTLDQSVRKNEKLKLAAQGAEKTYGNGDSLNTG**<br>**KLKNDKVSRFDFIRQIEVDGQLITLESGEFQIYKQDHSAVVALQIEKINNPDKIDSLINQRSFLVSGL**<br>**G**GEHTSFDKLPKDVMATYRGTAFGSDDAGGKLTYTIDFAAKQGHGKIEHLKSPELNVDLAVAYIKPDEK<br>HHAVISGSVLYNQDEKGSYSLGIFGEKAQEVAGSAEVETANGIHHIGLAAKQEAAAKEAAAKEAAAKE<br>AAAKVTADIGTGLADALTAPLDHKDKGLKSLTLEDSISQNGTLTLSAQGAEKTYGNGDSLNTGKLKND<br>KVSRFDFIRQIEVDGQLITLESGEFQVYKQSHSALTALQTEQEQDPEHSEKMVAKRRFRIGDIAGEHTAFN<br>QLPSGKAEYHGKAFSSDDAGGKLTYTIDFAAKQGHGKIEHLKTPEQNVELASAELKADEKSHAVILGDT<br>RYGSEEKGTYHLALFGDRAQEIAGSATVKIREKVHEIGIAGKQGSGGSGAHIVMVDAYKPTKHHHHHH<br>(SEQ ID NO:23) |

Table 3: Nucleic acid sequences set forth in SEQ ID NOs: 24-46 (encoding **RD012-1 to RD012-9 and RD012-13 to RD012-26 molecules**)

| Polypeptide name | Nucleic acid sequence |
|---|---|
| **RD012-1** | TCTAGCGGTTCAGGAAGTGGCGGAGGGGGGTGGCAGCTGACATCGGCACTGGTCTGGCGGACG CTCTGACCGCTCCGCTGGACCACAAGGACAAAGGTTTGAAAAGCCTGACTTTGGAAGATTCGATC TCTCAGAATGGTACGCTGACCCTCAGCGCGCAAGGCGCGGAGAAGACCTTTAAAGTTGGTGATAA GGACAACAGCTTGAACACCGGTAAACTGAAAAACGATAAGATTAGCCGCTTCGACTTCGTGCAGA AAATCGAAGTGGACGGTCAGACCATTACCCTTGCGAGCGGCGAGTTCCAGATTTACAAGCAGGAT CATAGTGCGGTTGTCGCATTACAAATCGAAAAAATCAATAATCCGGATAAGATTGATTCTCTCAT CAACCAACGTAGCTTCTTGGTTAGCGGCCTGGGTGGCGAACACACCAGCTTTGATAAGCTGCCAA AAGATGTCATGGCAACCTACCGTGGTACGGCGTTCGGCTCGGACGACGCGGGTGGCAAATTAACT TACACCATTGACTTTGCAGCGAAGCAGGGCCATGGCAAAATCGAGCACCTGAAGTCCCCGGAACT GAACGTGGACCTGGCAGTGGCGTACATCAAACCGGACGAAAAGCATCATGCTGTGATCAGCGGTT CGGTGCTGTATAACCAAGATGAGAAAGGCAGCTATAGCCTGGGTATTTTTGGCGAGAAGGCGCAG GAGGTGGCGGGTTCCGCCGAGGTTGAAACCGCGAATGGCATCCATCACATTGGGTTGGCGGCAAA ACAGGGCTCTAGCGGAGGTGGTGGTTCCGGTGGTGGCGGTGTTACCGCGGACATCGGTACCGGTT TGGCGGATGCGCTTACGGCACCGCTGGATCACAAAGACAAGGGTCTGAAGTCCTTGACCTTGGAG GACTCCATTTCGCAAAACGGCACCCTGACGCTTAGCGCTCAAGGCGCAGAAAAGACCTACGGCAA TGGTGACTCACTGAACACCGGTAAACTGAAGAACGACAAAGTTAGCCGCTTCGACTTTATCCGTC |

(continued)

| Polypeptide name | Nucleic acid sequence |
|---|---|
| | AGATTGAGGTTGATGGTCAGCTGATTACCCTGGAAAGCGGGGAGTTTCAGGTATATAAGCAATCC CATTCCGCGCTGACTGCCCTGCAGACCGAACAAGAACAGGACCCGGAGCACTCCGAAAAAATGG TTGCAAAGCGCCGTTTTCGTATTGGTGACATTGCGGGCGAGCACACCGCGTTCAACCAGCTGCCT GATGGTAAAGCCGAGTATCACGGCAAGGCCTTCAGCAGCGATGACGCCGGGGGTAAGTTAACGT ACACCATTGACTTTGCCGCGAAGCAAGGTCACGGGAAAATCGAGCACCTGAAGACACCGGAGCA AAATGTCGAGTTGGCTGCGGCAGAGCTGAAGGCTGACGAAAAGAGCCATGCAGTCATCCTGGGC GATACCCGTTATGGTTCTGAAGAAAAGGGTACGTACCACCTGGCCTTGTTCGGTGATCGTGCCCA AGAGATCGCGGGCTCTGCTACCGTTAAAATCGGCGAAAAAGTGCATGAAATCGGTATCGCGGGTA AACAGGGTTCTGGCGGCTCTGGCGCCCATATTGTTATGGTGGACGCATATAAACCGACCAAACAT CATCACCACCACCACTAATGA (SEQ ID NO:24) |

(continued)

| Polypeptide name | Nucleic acid sequence |
|---|---|
| **RD012-2** | TCTAGCGGTTCAGGAAGTGGAGGCGGGGGGGTGGCGGCGGACATCGGTACCGGTTTAGCGGACG CTCTCACCGCACCGCTGGATCATAAGGACAAAGGCCTAAAGTCCCTGACCCTGGAAGACAGCATT AGCCAGAATGGCACCCTGACCCTGAGCGCCCAGGGCGCAGAGAAGACCTTTAAAGTTGGCGACA AGGACAACTCCTTGAACACGGGCAAGCTGAAGAACGACAAGATCTCCCGCTTCGATTTCGTGCAG AAAATCGAAGTTGATGGCCAGACCATCACCTTGGCGAGCGGTGAGTTCCAGATTTATAAACAGGA CCACAGCGCGGTCGTGGCTCTGCAGATCGAAAAAATTAACAACCCGGACAAAATCGACTCGCTGA TCAACCAGCGTAGCTTTCTGGTTTCCGGTCTGGGCGGTGAGCACACCAGTTTTGATAAACTGCCTA AAGATGTTATGGCAACCTACCGCGGTACGGCGTTTGGTTCGGATGATGCGGGCGGGAAATTGACC TATACGATCGATTTCGCCGCGAAGCAGGGTCATGGTAAGATCGAACACCTAAAGTCCCCGGAACT TAACGTGGACCTGGCGGTGGCGTATATCAAGCCGGATGAAAAGCATCACGCAGTCATCAGCGGCT CCGTGCTGTACAATCAAGACGAAAAGGGTTCTTATAGCTTAGGTATCTTCGGCGAGAAAGCCCAA GAGGTCGCAGGTTCCGCGGAGGTCGAGACGGCCAATGGTATTCATCACATTGGTCTTGCGGCAAA ACAAGGCAGCGGAGGTGGCGGTGTTACCGCGGACATTGGTACTGGTTTGGCGGACGCTCTGACTG CGCCACTGGACCACAAAGATAAGGGCTTGAAGTCATTGACCTTGGAGGATAGCATTAGCCAAAAT GGCACCCTGACACTGTCTGCGCAGGGTGCCGAGAAGACCTACGGTAATGGGGACTCTCTGAACAC TGGCAAACTTAAAAACGATAAGGTGAGCCGCTTCGATTTTATCCGTCAAATTGAAGTGGACGGTC AGCTGATTACGCTGGAGAGCGGCGAGTTTCAGGTTTATAAACAAAGCCATTCCGCTCTGACGGCT TTGCAAACCGAACAAGAACAGGACCCGGAGCACTCTGAAAAGATGGTGGCTAAACGTCGTTTCC GTATTGGCGACATTGCAGGTGAGCACACCGCGTTTAACCAGCTGCCGGATGGCAAGGCCGAGTAT CACGGTAAGGCCTTCAGCAGCGATGACGCTGGCGGTAAATTGACGTACACCATTGATTTCGCCGC GAAACAAGGGCATGGTAAGATCGAGCATTTGAAGACTCCGGAACAAAATGTTGAACTGGCGGCC GCGGAGCTGAAGGCGGACGAAAAATCTCATGCAGTTATTCTGGGCGACACCCGTTACGGTAGCGA AGAGAAAGGTACCTACCACCTGGCTCTGTTTGGTGACAGAGCACAGGAGATCGCTGGTAGCGCGA CCGTAAAAATTGGTGAAAAAGTTCATGAAATCGGTATCGCAGGCAAGCAAGGCTCAGGCGGCTCT GGCGCGCATATTGTTATGGTGGATGCGTACAAGCCGACAAAACACCACCATCACCACCACTAATG A (SEQ ID NO:25) |

(continued)

| Polypeptide name | Nucleic acid sequence |
|---|---|
| **RD012-3** | CACCATCACCACCACCATTCATCTGGAAGTGGTTCAGGTGGCGGTGGCGTGGCGGCTGACATCGG<br>CACCGGTCTTGCTGACGCTTTGACGGCTCCGCTGGATCATAAAGACAAAGGTCTGAAAAGCCTAA<br>CCTTGGAAGATAGCATCTCTCAGAATGGCACTCTGACGCTGAGCGCTCAGGGCGCAGAGAAAACC<br>TTTAAAGTGGGTGACAAGGACAACAGCCTGAACACCGGTAAGCTGAAGAATGATAAAATCAGTA<br>GATTCGATTTTGTTCAGAAAATTGAAGTTGATGGCCAGACGATTACCTTGGCCAGCGGTGAGTTCC<br>AGATCTACAAGCAAGATCACAGCGCGGTAGTTGCCCTGCAAATTGAAAAGATCAACAACCCGGA<br>TAAAATCGATTCTCTGATAAACCAGCGTAGCTTTCTGGTTTCTGGCTTAGGCGGTGAACACACCAG<br>CTTTGATAAGTTGCCGAAAGATGTGATGGCAACCTACCGTGGTACGGCCTTCGGCTCGGACGATG<br>CTGGGGGCAAGCTGACGTACACCATTGACTTCGCAGCGAAACAGGGTCACGGTAAAATCGAGCA<br>CCTTAAGTCGCCGGAATTGAATGTGGACCTGGCGGTTGCGTATATTAAACCGGATGAAAAACATC<br>ACGCAGTGATCTCCGGTAGCGTTCTGTATAATCAGGATGAGAAGGGTAGCTATAGCCTGGGCATT<br>TTTGGTGAAAAGGCGCAGGAGGTCGCGGGTTCTGCGGAGGTGGAGACAGCGAATGGTATTCATC<br>ATATTGGCCTGGCGGCGAAGCAAGGTTCCAGCGGCGGTGGAGGCAGCGGTGGCGGCGGCGTGAC<br>GGCTGACATCGGCACCGGTCTGGCGGACGCGCTCACTGCGCCACTGGACCACAAAGACAAAGGC<br>CTCAAGAGCTTGACCTTGGAGGACTCGATTAGCCAAAACGGCACTTTGACTTTGAGCGCTCAGGG<br>TGCAGAGAAGACCTACGGTAATGGCGACTCCCTCAACACCGGTAAACTGAAGAACGATAAAGTT<br>AGTCGCTTCGATTTCATCCGCCAGATCGAGGTGGACGGCCAACTGATTACCCTTGAGAGCGGTGA<br>GTTCCAAGTTTATAAGCAATCCCACTCGGCTCTGACCGCGCTGCAGACCGAACAAGAACAGGACC<br>CGGAGCACAGCGAAAAGATGGTTGCGAAGCGCCGTTTTCGTATCGGAGACATCGCTGGCGAGCA<br>CACCGCGTTCAACCAACTGCCGGACGGTAAAGCGGAGTATCATGGTAAGGCATTCAGCTCCGACG<br>ACGCAGGTGGCAAACTGACTTACACCATTGACTTTGCGGCAAAACAAGGCCACGGCAAAATCGA<br>GCACCTGAAAACCCCGGAACAAAACGTGGAACTGGCAGCGGCGGAACTGAAGGCCGACGAAAA<br>GTCCCATGCCGTCATCCTGGGTGACACCCGTTACGGCTCTGAAGAAAAAGGTACCTACCATCTGG<br>CACTGTTTGGTGATCGTGCCCAGGAGATCGCCGGCTCTGCGACCGTCAAAATCGGTGAGAAGGTT<br>CATGAAATTGGTATTGCGGGGAAGCAATAATGA (SEQ ID NO:26) |

(continued)

| Polypeptide name | Nucleic acid sequence |
|---|---|
| **RD012-4** | CACCATCACCACCACCATTCATCTGGAAGTGGTTCCGGTGGTGGTGGTGTTGCCGCGGACATTGG<br>CACTGGACTGGCCGACGCGCTGACCGCGCCACTGGACCACAAGGACAAGGGTCTGAAAAGCCTG<br>ACATTGGAGGATAGCATTAGCCAGAACGGTACGTTGACTTTATCTGCCCAAGGCGCGGAAAAAAC<br>CTTTAAAGTCGGTGACAAGGACAACAGCCTCAATACCGGCAAGCTGAAAAACGATAAAATCAGT<br>AGATTCGACTTTGTCCAGAAAATCGAAGTGGACGGTCAGACGATCACCTTGGCTAGCGGTGAATT<br>TCAGATTTATAAACAGGACCACTCCGCTGTCGTGGCACTGCAGATTGAAAAGATCAACAACCCGG<br>ACAAGATCGATAGCCTAATCAATCAACGTAGCTTTCTGGTTAGCGGCCTGGGCGGTGAGCACACG<br>AGCTTCGATAAACTCCCGAAAGATGTTATGGCAACCTACCGCGGTACCGCGTTCGGCTCTGATGA<br>TGCTGGTGGCAAGCTGACGTACACCATCGACTTCGCGGCTAAGCAAGGCCATGGCAAAATCGAGC<br>ACCTGAAGTCCCCGGAACTGAACGTTGACCTGGCCGTGGCATACATTAAACCGGATGAGAAACAT<br>CACGCCGTCATCTCTGGCTCTGTTCTGTATAACCAGGATGAAAAAGGTTCATATAGCCTGGGTATT<br>TTTGGTGAAAAGGCACAAGAGGTGGCGGGTTCCGCGGAGGTGGAAACCGCAAATGGCATCCATC<br>ACATTGGTTTGGCTGCGAAGCAAGGTGGAAGTGGCGGCTCGGGGGGTTCTGGCGGCTCCGCGCAT<br>ATTGTTATGGTTGATGCGTATAAACCGACCAAAGGTTCGAGCGGTGGGGGTGGCTCTGGCGGTGG<br>GGGCGTGACCGCGGACATCGGCACCGGCCTGGCGGACGCCTTGACCGCTCCGCTGGATCACAAAG |

(continued)

| Polypeptide name | Nucleic acid sequence |
|---|---|
| | ATAAGGGCCTGAAGTCCTTAACTCTCGAAGATTCCATCTCCCAGAATGGTACCCTGACGCTTAGC GCGCAAGGCGCGGAGAAAACCTACGGAAACGGTGACAGCCTGAATACTGGCAAGCTGAAGAACG ATAAAGTGAGCCGTTTTGATTTCATTCGTCAAATCGAGGTGGACGGCCAGCTGATTACCCTGGAA AGCGGCGAGTTCCAGGTGTACAAGCAAAGCCATAGCGCATTGACCGCGCTGCAAACCGAACAAG AACAGGACCCGGAACACTCGGAGAAGATGGTGGCGAAGCGCCGTTTTCGTATTGGTGACATCGCC GGCGAGCACACCGCCTTCAACCAGCTGCCGGATGGTAAAGCTGAGTATCACGGTAAAGCGTTCAG CAGCGACGACGCCGGTGGTAAGTTGACGTACACCATCGACTTCGCTGCGAAACAAGGTCACGGCA AAATCGAGCACCTGAAGACCCCGGAGCAGAATGTTGAACTGGCGGCAGCGGAGCTGAAAGCTGA CGAGAAGTCGCATGCGGTGATTCTGGGTGATACCCGTTATGGTTCCGAAGAAAAGGGCACCTACC ATCTGGCGTTGTTCGGTGACCGCGCACAGGAGATCGCAGGCTCTGCGACCGTTAAAATTGGTGAG AAAGTTCATGAAATTGGTATTGCAGGCAAGCAGTAATGA (SEQ ID NO:27) |

(continued)

| Polypeptide name | Nucleic acid sequence |
| --- | --- |
| **RD012-5** | TCTAGCGGAAGTGGTTCAGGGGGAGGCGGGGTGGCTGCGGACATCGGCACGGGTCTGGCTGATG CACTGACTGCGCCACTGGATCATAAAGACAAGGGTCTGAAAAGCCTGACCTTGGAAGATTCCATT TCGCAAAACGGTACTTTGACCCTGAGCGCTCAGGGCGCAGAAAAAACCTTTAAAGTTGGTGATAA GGACAATAGCTTGAACACCGGTAAACTGAAGAATGATAAAATCTCTCGTTTTGACTTCGTGCAGA AAATCGAGGTCGATGGTCAGACCATTACCTTGGCGAGCGGCGAATTTCAGATTTATAAACAAGAC CATAGCGCGGTTGTTGCGCTGCAGATTGAGAAGATCAACAACCCGGACAAGATCGACAGCCTGAT TAATCAACGTTCGTTCCTGGTTAGCGGGCTGGGTGGCGAGCATACCAGCTTCGACAAGCTGCCGA AAGATGTTATGGCAACCTACCGCGGTACGGCGTTTGGCAGCGATGATGCGGGTGGCAAGCTGACG TACACCATCGACTTCGCCGCGAAACAAGGTCATGGTAAAATCGAGCACCTGAAGTCCCCGGAACT GAATGTTGACCTGGCGGTGGCCTACATTAAACCGGACGAGAAGCACCACGCGGTGATCTCAGGTT CCGTGCTGTATAACCAGGATGAGAAAGGCAGCTATAGCTTAGGTATCTTCGGCGAAAAGGCCCAA GAAGTCGCCGGTTCTGCAGAGGTGGAAACCGCGAACGGCATTCATCACATCGGCCTCGCGGCAAA GCAGGGTTCCGGCGGTTCTGGCGCTCATATTGTTATGGTGGACGCTTACAAGCCGACCAAACATC ACCACCACCACCACTAATGA (SEQ ID NO:28) |

(continued)

| Polypeptide name | Nucleic acid sequence |
|---|---|
| **RD012-6** | TCATCTGGTGGAGGGGGAAGTGGCGGAGGGGGTGTTACCGCCGACATCGGGACGGGTCTGGCTG ACGCCTTGACTGCGCCACTGGATCATAAAGATAAAGGTCTTAAGTCGCTGACGTTGGAGGACAGT ATTAGCCAGAACGGTACCCTGACCCTGTCTGCTCAGGGCGCAGAGAAAACCTACGGCAATGGAG ACAGCCTGAACACCGGTAAACTGAAGAACGATAAGGTGAGCCGTTTTGATTTCATCCGTCAGATT GAAGTTGACGGCCAGCTGATTACCTTGGAGAGCGGCGAATTTCAGGTGTATAAGCAAAGCCACTC CGCGTTGACTGCGTTACAAACCGAACAAGAACAGGACCCGGAACACTCGGAAAAAATGGTCGCC AAGCGCAGATTCCGCATTGGCGACATCGCGGGCGAGCACACCGCGTTCAACCAGCTGCCGGATGG CAAGGCGGAGTATCACGGCAAGGCCTTCAGCTCCGATGATGCCGGCGGTAAATTGACGTACACCA TTGATTTTGCGGCGAAACAAGGTCATGGCAAGATCGAGCACCTCAAGACCCCGGAACAGAATGTT GAGCTGGCAGCTGCTGAGCTGAAGGCGGACGAAAAGAGCCACGCTGTGATCCTGGGGGACACCC GTTATGGTTCCGAGGAAAAAGGCACGTACCATCTGGCGTTATTTGGTGACCGTGCACAAGAGATC GCGGGTAGCGCAACCGTTAAAATTGGTGAGAAAGTACATGAAATCGGTATCGCGGGTAAGCAAG GCTCCGGCGGTTCTGGTGCACATATTGTCATGGTGGACGCGTACAAACCGACCAAACACCATCAC CACCACCATTAATGA (SEQ ID NO:29) |

(continued)

| Polypeptide name | Nucleic acid sequence |
|---|---|
| **RD012-7** | TCATCTGGTGGAGGGGGAAGTGGCGGAGGGGGCGTGACCGCGGACATCGGTACAGGCTTAGCGG<br>ACGCTCTGACGGCTCCGCTGGATCATAAGGACAAAGGCTTGAAGTCGTTGACCCTTGAGGACAGC<br>ATCAGCCAGAACGGCACTCTGACCCTCTCGGCTCAAGGTGCGGAAAAGACCTACGGCAACGGCG<br>ACAGCCTGAATACCGGTAAGCTGAAGAACGATAAAGTTTCTCGTTTCGACTTCATTCGTCAAATC<br>GAAGTTGATGGTCAGCTGATCACCTTGGAGAGCGGAGAGTTCCAGGTGTATAAACAAAGCCATAG<br>TGCACTGACGGCGCTGCAGACCGAACAAGAACAGGACCCGGAGCACAGCGAAAAGATGGTTGCT<br>AAACGTCGCTTTCGTATTGGCGACATCGCCGGTGAGCACACCAGCTTTGATAAACTACCGAAAGA<br>CGTCATGGCAACCTACCGCGGTACTGCCTTTGGTTCCGATGATGCGGGTGGTAAGCTGACGTACA<br>CCATTGATTTTGCGGCGAAGCAAGGTCATGGTAAAATTGAGCACCTGAAGTCCCCGGAGCTGAAC<br>GTGGACCTGGCGGTCGCATACATCAAACCGGACGAAAAACACCACGCCGTTATTAGCGGCTCTGT<br>TCTGTATAACCAGGATGAGAAGGGTAGCTATTCCCTGGGTATCTTCGGTGAAAAGGCGCAGGAGG<br>TGGCCGGTTCTGCGGAAGTAGAAACCGCGAATGGCATTCACCACATCGGTTTGGCTGCAAAGCAA<br>GGCAGCGGTGGCTCCGGCGCACATATCGTGATGGTTGACGCGTACAAACCAACGAAACATCATCA<br>CCACCATCACTAATGA (SEQ ID NO:30) |

(continued)

| Polypeptide name | Nucleic acid sequence |
|---|---|
| **RD012-8** | TCAAGTGGTGGAGGAGGGGTAGCAGCTGACATCGGTGCTGGCCTGGCGGACGCGCTGACGGCTC<br>CGCTGGACCATAAAGATAAATCCTTACAGAGCTTAACTCTGGACCAATCAGTTCGCAAAAACGAA<br>AAATTGAAGTTGGCCGCGCAGGGTGCGGAAAAAACGTACGGAAACGGCGACAGCTTGAATACCG<br>GTAAGCTCAAGAACGATAAGGTCTCTCGTTTTGATTTTATCCGTCAGATTGAGGTGGACGGTCAGC<br>TGATTACCCTGGAAAGCGGCGAGTTCCAGATTTACAAGCAAGATCATAGCGCTGTGGTGGCATTG<br>CAAATCGAGAAGATCAATAACCCGGACAAAATCGACAGCCTTATCAACCAGCGTAGCTTCCTGGT<br>GTCCGGTCTGGGCGGTGAGCACACCGCGTTTAACCAGTTGCCAGACGGCAAGGCTGAGTATCACG<br>GCAAAGCGTTCAGCAGCGATGATGCGGGCGGTAAGCTGACCTACACCATCGACTTCGCGGCGAA<br>ACAAGGTCATGGCAAAATTGAGCACCTGAAGACCCCGGAACAGAATGTTGAACTGGCTGCGGCG<br>GAGCTGAAGGCGGATGAAAAGTCCCATGCGGTCATTCTGGGTGATACCCGTTATGGCTCGGAAGA<br>GAAAGGTACATACCATCTGGCCTTGTTTGGTGATCGCGCACAAGAGATCGCCGGTAGCGCCACCG<br>TTAAAATCGGCGAGAAGGTGCATGAAATTGGTATTGCAGGTAAGCAAGGCTCTGGCGGGTCTGGT<br>GCCCATATTGTTATGGTTGACGCATATAAACCGACGAAACACCACCACCACCACCACTAATGA<br>(SEQ ID NO:31) |
| **RD012-9** | TCTAGCGGAAGTGGTTCAGGAGGCGGGGGGGTGGCTGCTGATATTGGTACCGGCCTGGCCGACGC<br>TCTCACGGCGCCACTGGATCACAAAGATAAGGGCTTGAAATCGTTGACCCTGGAAGATTCAATTA<br>GCCAGAACGGTACTTTGACCCTATCTGCGCAAGGTGCGGAAAAGACGTTTAAAGTTGGTGACAAG<br>GACAACAGCCTTAATACTGGTAAGCTGAAGAACGACAAAATCAGTCGTTTTGATTTCGTGCAGAA<br>AATCGAGGTAGATGGTCAAACCATTACCTTGGCGAGCGGTGAGTTCCAGATCTACAAACAAGACC<br>ACAGCGCTGTGGTTGCATTACAGATTGAGAAGATCAACAACCCGGATAAGATCGACTCCCTGATT<br>AATCAACGCAGCTTCCTGGTTAGCGGCCTGGGCGGCGAGCACACCGCGTTCAACCAGCTGCCGAG |

(continued)

| Polypeptide name | Nucleic acid sequence |
|---|---|
| | CGGCAAGGCCGAGTATCATGGCAAGGCGTTTTCCAGCGATGATGCAGGTGGCAAGCTGACGTACA<br>CCATTGACTTTGCCGCGAAACAGGGTCATGGTAAGATCGAGCACCTGAAAACCCCGGAACAGAAT<br>GTCGAGTTGGCGTCCGCGGAACTGAAAGCAGACGAGAAAAGCCACGCAGTCATCCTGGGGGACA<br>CCCGTTATGGATCTGAAGAGAAAGGTACATACCATCTGGCACTGTTCGGCGACCGCGCGCAAGAG<br>ATTGCCGGTTCGGCTACCGTTAAAATCCGTGAAAAGGTGCATGAAATCGGAATCGCGGGTAAGCA<br>GGGCAGCGGTGGCTCTGGTGCCCATATTGTTATGGTGGACGCGTATAAACCGACCAAACATCACC<br>ACCACCATCACTAATGA (SEQ ID NO:32) |
| **RD012-13** | CACCATCACCACCACCATTCAAGTGGAGGGGGCGGTTCTGGTGGTGGCGGAGTTACCGCGGACAT<br>CGGCACGGGCCTGGCGGACGCCTTGACGGCTCCGCTGGATCATAAAGACAAAGGTCTGAAATCCT<br>TGACCTTGGAGGACTCCATTAGCCAGAATGGTACCCTGACCTTAAGCGCTCAGGGTGCAGAGAAG<br>ACCTACGGCAACGGCGACTCCTTGAATACGGGCAAGTTGAAGAACGATAAAGTCAGCAGATTTG<br>ACTTCATCCGCCAGATTGAAGTTGATGGTCAGCTTATCACCCTGGAAAGCGGTGAGTTTCAGGTTT<br>ATAAACAGTCTCACAGCGCGCTCACTGCGCTGCAGACCGAACAAGAGCAAGATCCAGAACACTCT<br>GGCAAGATGGTGGCAAAACGCCGTTTTCGTATTGGCGACATCGCGGGCGAGCACACCGCGTTCAA<br>CCAGCTGCCGGATGGCAAAGCGGAGTATCATGGTAAGGCGTTCAGCAGCGATGATGCAGGTGGT<br>AAGCTGACGTACACCATTGATTTCGCCGCGAAACAAGGTCACGGCAAGATCGAGCACCTGAAAA<br>CCCCGGAGCAAAACGTGGAACTGGCGGCAGCTGAGCTGAAGGCGGACGAAAAGAGCCACGCTGT<br>GATTCTGGGTGACACCCGTTATGGTTCGGAAGAAAAGGGTACTTACCATCTGGCGCTGTTTGGTG<br>ACCGTGCACAAGAGATCGCCGGCTCGGCTACCGTTAAAATCGGCGAGAAAGTGCATGAAATCGG<br>CATTGCCGGCAAGCAATAATGA (SEQ ID NO:33) |

(continued)

| Polypeptide name | Nucleic acid sequence |
|---|---|
| **RD012-14** | CACCACCATCACCACCATTCAAGTGGAGGGGGCGGTTCGGGCGGTGGCGGGGTGACCGCGGACA TCGGTACGGGTCTGGCTGACGCCCTCACGGCTCCGCTGGATCATAAAGATAAAGGTTTGAAGTCC CTGACCCTGGAAGATAGTATTTCGCAAAATGGTACGTTAACCCTGAGCGCGCAGGGTGCAGAGAA GACCTACGGAAACGGCGACAGCCTGAACACCGGTAAGCTGAAGAACGACAAAGTCAGCCGTTTC GATTTTATCCGCCAGATTGAAGTGGATGGTCAGCTTATCACCTTGGAGAGCGGTGAATTTCAGGTT TATAAACAAAGCCACAGCGCGTTGACTGCCCTACAGACCGAACAAGAGCAAGATCCAGAACACT CCGGCAAGATGGTGGCAAAACGTCGTTTCCGCATTGGCGACATCGCCGGCGAGCACACCGCGTTC AACCAGCTTCCGGATGGTAAAGCAGAGTATCATGGTAAGGCGTTTTCTAGCGATGATGCAGGCGG CAAACTGACGTACACCATTGACTTCGCGAAAAAACAAGGTCATGGTCGTATCGAGCACCTGAAAA CTCCGGAGCAGAATGTTGAACTGGCGGCTGCGGAGCTGAAGGCCGACGAAAAGTCCCACGCTGTT ATTCTGGGTGACACCCGTTATGGTAGCGAAGAAAAGGGCACCTACCACCTGGCGTTGTTTGGTGA CCGCGCACAGGAGATCGCTGGCTCTGCGACCGTTAAAATCGGAGAGAAGGTGCATGAAATCGGC ATTGCGGGCAAGCAATAATGA (SEQ ID NO:34) |
| **RD012-15** | TCATCTGGTGGGGGGGGAAGTGGCGGAGGCGGGGTGACCGCAGATATCGGCACCGGTCTGGCTG ACGCGCTGACTGCGCCTCTGGATCACAAAGACAAGGGCTTGAAGTCCCTGACCCTGGAAGATTCG ATCAGCCAGAACGGCACCTTGACGCTGAGCGCGCAGGGTGCCGAAAAAACCTACGGAAACGGTG ACAGCCTAAACACCGGTAAACTGAAGAATGATAAAGTGTCTCGTTTCGACTTCATTCGTCAGATT |

(continued)

| Polypeptide name | Nucleic acid sequence |
|---|---|
| | GAAGTGGACGGCCAGCTGATCACCTTGGAGAGCGGCGAGTTCCAAGTTTATAAACAAAGCCATA<br>GCGCGTTGACGGCGCTGCAAACCGAACAGGAGCAGGATCCGGAACACAGCGAAAAAATGGTTGC<br>TAAGCGCCGTTTTCGTATTGGCGACATAGCAGGCGAGCACACCGCGTTTAATCAACTGCCGGACG<br>GCAAGGCCGAGTATCATGGCAAAGCCTTCAGCAGCGACGACGCTGGTGGTAAATTGACGTACACC<br>ATTGACTTCGCCGCCAAACAGGGCCACGGCAAGATCGAGCACCTGAAGACTCCGGAACAGAACG<br>TGGAGCTGGCAGCAGCGGAGCTGAAGGCTGACGAGAAGAGCCACGCCGTGATTCTGGGCGATAC<br>CCGTTATGGTTCTGAAGAAAAAGGTACATACCATCTGGCGCTGTTTGGTGATCGTGCACAAGAGA<br>TCGCGGGTTCGGCTACCGTTAAGATCGGTGAAAAGGTGCATGAGATCGGGATCGCCGGTAAGCAA<br>GGCGGCTCGAGCGGTTCCGGTTCTGGCGGCGGCGGCGTTGCGGCGGACATCGGTACCGGCCTGGC<br>GGATGCGCTGACTGCGCCACTGGATCATAAAGACAAAGGTTTGAAGTCCTTGACGCTTGAGGACA<br>GCATTTCACAAAACGGTACGTTAACCCTTAGTGCGCAAGGTGCTGAGAAGACCTTTAAAGTTGGT<br>GACAAAGATAATTCCCTGAACACCGGTAAGCTCAAGAACGATAAGATCAGCCGCTTCGACTTTGT<br>TCAGAAAATCGAGGTGGATGGTCAGACCATTACCCTGGCTAGCGGTGAGTTCCAGATTTATAAAC<br>AGGACCACTCTGCGGTCGTGGCCTTACAGATCGAAAAGATCAACAACCCGGATAAGATTGATTCG<br>CTGATTAACCAACGTAGCTTCCTGGTTAGCGGTTTAGGTGGTGAGCACACCAGCTTCGACAAATT<br>GCCGAAAGACGTGATGGCAACCTACCGCGGTACTGCGTTCGGCTCTGATGATGCGGGTGGTAAGT<br>TGACGTACACGATCGACTTTGCAGCGAAACAAGGTCATGGCAAAATCGAACACCTGAAATCCCCG<br>GAACTGAATGTTGATCTGGCAGTTGCATACATCAAACCGGATGAAAAGCACCACGCAGTCATCAG<br>CGGTTCCGTGCTGTATAATCAGGATGAGAAAGGCTCCTATAGCCTGGGTATTTTTGGCGAGAAGG<br>CGCAAGAAGTAGCTGGTAGCGCGGAAGTTGAAACCGCTAATGGCATTCATCACATCGGCCTCGCT<br>GCGAAGCAGGGTTCTGGTGGGTCGGGCGCACATATTGTTATGGTGGACGCGTACAAGCCGACCAA<br>ACATCACCACCACCATCATTAATGA (SEQ ID NO:35) |

(continued)

| Polypeptide name | Nucleic acid sequence |
|---|---|
| **RD012-16** | TCATCTGGGGGAGGCGGAAGTGGTGGGGGGGGCGTTACCGCGGACATCGGTACTGGCCTGGCCG<br>ACGCCCTGACGGCACCGCTGGATCACAAAGACAAGGGCTTGAAATCCTTGACTCTTGAGGATTCT<br>ATTTCGCAAAACGGCACCCTGACTTTGAGTGCGCAGGGTGCAGAGAAGACCTATGGTAATGGCGA<br>CAGCTTGAACACCGGTAAGCTGAAGAACGATAAGGTGAGCCGCTTCGATTTCATCCGCCAGATAG<br>AAGTAGATGGTCAACTGATTACCCTGGAAAGCGGTGAATTTCAAGTTTATAAACAGAGCCATTCC<br>GCCTTGACCGCGCTGCAAACCGAACAAGAGCAAGATCCGGAACATTCGGAAAAGATGGTTGCCA<br>AACGTCGTTTCCGTATTGGCGACATCGCAGGTGAGCACACCGCGTTTAATCAGCTGCCGGACGGC<br>AAAGCGGAGTATCACGGCAAGGCGTTCAGCAGCGACGACGCGGGCGGTAAACTGACGTACACCA<br>TTGATTTCGCTGCGAAACAAGGTCATGGTAAGATCGAACACCTGAAAACACCGGAACAAAACGT<br>GGAATTGGCGGCTGCGGAGCTTAAGGCGGACGAGAAGTCCCATGCGGTCATCCTGGGTGACACCC<br>GTTACGGCTCCGAAGAAAAGGGGACGTACCACTTGGCGTTGTTTGGTGATAGAGCACAGGAGATC<br>GCGGGCAGCGCGACCGTTAAAATCGGCGAGAAGGTTCATGAAATTGGTATCGCCGGTAAGCAGG<br>GTTCGGGTGGTGGCGGGGTGGCAGCCGATATCGGCACCGGGCTTGCTGACGCGCTCACGGCTCCG<br>CTGGACCACAAAGACAAGGGTTTAAAGAGCCTGACCCTGGAAGATAGCATTTCCCAGAACGGCA<br>CCCTGACCCTGTCTGCACAAGGTGCGGAGAAAACCTTTAAAGTAGGTGATAAGGACAACAGCCTA<br>AACACCGGTAAGCTGAAAAATGACAAGATCTCTCGTTTCGACTTTGTTCAGAAAATCGAGGTGGA |

(continued)

| Polypeptide name | Nucleic acid sequence |
|---|---|
| | TGGTCAGACCATTACCTTGGCTAGCGGCGAGTTCCAGATCTATAAACAAGACCATAGCGCGGTCG TGGCGTTACAGATCGAGAAGATTAACAACCCGGATAAGATCGATTCGCTGATTAACCAGCGTAGC TTTCTGGTTTCCGGACTCGGTGGCGAGCACACCAGCTTCGACAAGCTGCCAAAAGACGTGATGGC AACCTACCGCGGTACGGCTTTCGGTTCTGATGATGCTGGTGGTAAACTGACCTACACGATCGACTT CGCCGCAAAACAAGGCCACGGCAAGATTGAGCACCTGAAATCCCCGGAACTGAATGTTGATCTG GCGGTTGCGTATATTAAACCGGATGAAAAACACCACGCAGTCATTTCTGGCAGCGTGCTGTACAA TCAGGATGAGAAGGGTAGCTATAGCCTGGGCATTTTTGGTGAGAAGGCGCAGGAGGTTGCCGGTA GCGCCGAAGTGGAAACCGCTAATGGTATTCACCACATCGGTCTGGCCGCTAAACAGGGCAGCGGT GGCTCCGGCGCACATATCGTGATGGTGGACGCATACAAACCGACGAAACATCATCATCACCACCA CTAATGA (SEQ ID NO:36) |

(continued)

| Polypeptide name | Nucleic acid sequence |
|---|---|
| **RD012-17** | GTGGCAGCTGACATCGGCACTGGTCTGGCGGACGCTCTGACCGCTCCGCTGGACCACAAGGACAA AGGTTTGAAAAGCCTGACTTTGGAAGATTCGATCTCTCAGAATGGTACGCTGACCCTCAGCGCGC AAGGCGCGGAGAAGACCTTTAAAGTTGGTGATAAGGACAACAGCTTGAACACCGGTAAACTGAA AAACGATAAGATTAGCCGCTTCGACTTCGTGCAGAAAATCGAAGTGGACGGTCAGACCATTACCC TTGCGAGCGGCGAGTTCCAGATTTACAAGCAGGATCATAGTGCGGTTGTCGCATTACAAATCGAA AAAATCAATAATCCGGATAAGATTGATTCTCTCATCAACCAACGTAGCTTCTTGGTTAGCGGCCTG GGTGGCGAACACACCAGCTTTGATAAGCTGCCAAAAGATGTCATGGCAACCTACCGTGGTACGGC GTTCGGCTCGGACGACGCGGGTGGCAAATTAACTTACACCATTGACTTTGCAGCGAAGCAGGGCC ATGGCAAAATCGAGCACCTGAAGTCCCCGGAACTGAACGTGGACCTGGCAGTGGCGTACATCAA ACCGGACGAAAAGCATCATGCTGTGATCAGCGGTTCGGTGCTGTATAACCAAGATGAGAAAGGC AGCTATAGCCTGGGTATTTTTGGCGAGAAGGCGCAGGAGGTGGCGGGTTCCGCCGAGGTTGAAAC CGCGAATGGCATCCATCACATTGGGTTGGCGGCAAAACAGGGCTCTAGCGGAGGTGGTGGTTCCG GTGGTGGCGGTGTTACCGCGGACATCGGTACCGGTTTGGCGGATGCGCTTACGGCACCGCTGGAT CACAAAGACAAGGGTCTGAAGTCCTTGACCTTGGAGGACTCCATTTCGCAAAACGGCACCCTGAC GCTTAGCGCTCAAGGCGCAGAAAAGACCTACGGCAATGGTGACTCACTGAACACCGGTAAACTG AAGAACGACAAAGTTAGCCGCTTCGACTTTATCCGTCAGATTGAGGTTGATGGTCAGCTGATTAC CCTGGAAAGCGGGGAGTTTCAGGTATATAAGCAATCCCATTCCGCGCTGACTGCCCTGCAGACCG AACAAGAACAGGACCCGGAGCACTCCGAAAAAATGGTTGCAAAGCGCCGTTTTCGTATTGGTGAC ATTGCGGGCGAGCACACCGCGTTCAACCAGCTGCCTGATGGTAAAGCCGAGTATCACGGCAAGGC CTTCAGCAGCGATGACGCCGGGGGTAAGTTAACGTACACCATTGACTTTGCCGCGAAGCAAGGTC ACGGGAAAATCGAGCACCTGAAGACACCGGAGCAAAATGTCGAGTTGGCTGCGGCAGAGCTGAA GGCTGACGAAAAGAGCCATGCAGTCATCCTGGGCGATACCCGTTATGGTTCTGAAGAAAAGGGTA CGTACCACCTGGCCTTGTTCGGTGATCGTGCCCAAGAGATCGCGGGCTCTGCTACCGTTAAAATCG GCGAAAAAGTGCATGAAATCGGTATCGCGGGTAAACAGGGTTCTGGCGGCTCTGGCGCCCATATT GTTATGGTGGACGCATATAAACCGACCAAACATCATCACCACCACCACTAATGA (SEQ ID NO:37) |

(continued)

| Polypeptide name | Nucleic acid sequence |
|---|---|
| **RD012-18** | TTGAACACCGGTAAACTGAAAAACGATAAGATTAGCCGCTTCGACTTCGTGCAGAAAATCGAAGT GGACGGTCAGACCATTACCCTTGCGAGCGGCGAGTTCCAGATTTACAAGCAGGATCATAGTGCGG TTGTCGCATTACAAATCGAAAAAATCAATAATCCGGATAAGATTGATTCTCTCATCAACCAACGT AGCTTCTTGGTTAGCGGCCTGGGTGGCGAACACACCAGCTTTGATAAGCTGCCAAAAGATGTCAT GGCAACCTACCGTGGTACGGCGTTCGGCTCGGACGACGCGGGTGGCAAATTAACTTACACCATTG ACTTTGCAGCGAAGCAGGGCCATGGCAAAATCGAGCACCTGAAGTCCCCGGAACTGAACGTGGA CCTGGCAGTGGCGTACATCAAACCGGACGAAAAGCATCATGCTGTGATCAGCGGTTCGGTGCTGT ATAACCAAGATGAGAAAGGCAGCTATAGCCTGGGTATTTTTGGCGAGAAGGCGCAGGAGGTGGC GGGTTCCGCCGAGGTTGAAACCGCGAATGGCATCCATCACATTGGGTTGGCGGCAAAACAGGGCT CTAGCGGAGGTGGTGGTTCCGGTGGTGGCGGTGTTACCGCGGACATCGGTACCGGTTTGGCGGAT GCGCTTACGGCACCGCTGGATCACAAAGACAAGGGTCTGAAGTCCTTGACCTTGGAGGACTCCAT TTCGCAAAACGGCACCCTGACGCTTAGCGCTCAAGGCGCAGAAAAGACCTACGGCAATGGTGACT CACTGAACACCGGTAAACTGAAGAACGACAAAGTTAGCCGCTTCGACTTTATCCGTCAGATTGAG GTTGATGGTCAGCTGATTACCCTGGAAAGCGGGGAGTTTCAGGTATATAAGCAATCCCATTCCGC GCTGACTGCCCTGCAGACCGAACAAGAACAGGACCCGGAGCACTCCGAAAAAATGGTTGCAAAG CGCCGTTTTCGTATTGGTGACATTGCGGGCGAGCACACCGCGTTCAACCAGCTGCCTGATGGTAA AGCCGAGTATCACGGCAAGGCCTTCAGCAGCGATGACGCCGGGGGTAAGTTAACGTACACCATTG ACTTTGCCGCGAAGCAAGGTCACGGGAAAATCGAGCACCTGAAGACACCGGAGCAAAATGTCGA GTTGGCTGCGGCAGAGCTGAAGGCTGACGAAAAGAGCCATGCAGTCATCCTGGGCGATACCCGTT ATGGTTCTGAAGAAAAGGGTACGTACCACCTGGCCTTGTTCGGTGATCGTGCCCAAGAGATCGCG GGCTCTGCTACCGTTAAAATCGGCGAAAAAGTGCATGAAATCGGTATCGCGGGTAAACAGGGTTC TGGCGGCTCTGGCGCCCATATTGTTATGGTGGACGCATATAAACCGACCAAACATCATCACCACC ACCACTAATGA (SEQ ID NO:38) |

(continued)

| Polypeptide name | Nucleic acid sequence |
|---|---|
| **RD012-19** | TCTAGCGGAAGTGGGTCAGGAGGCGGTGGTGTTGCGGCTGACATCGGAACCGGCCTGGCAGACG<br>CGTTGACCGCGCCGTTGGACCACAAAGACAAGGGTTTGAAAAGCCTGACCTTGGAAGACTCAATT<br>TCGCAGAATGGTACCCTCACGCTGAGCGCTCAGGGTGCGGAGAAAACGTTTAAAGTTGGTGACAA<br>GGACAACAGCCTGAACACCGGTAAATTGAAGAACGATAAGATTAGCCGTTTTGATTTCGTGCAGA<br>AAATCGAGGTGGACGGCCAAACCATTACCCTGGCGAGCGGCGAGTTCCAGATCTACAAGCAAGA<br>CCATTCCGCTGTCGTGGCCTTACAGATTGAAAAAATCAACAACCCGGATAAGATTGACTCCCTGA<br>TTAATCAGCGTAGCTTCCTGGTTAGCGGTCTGGGTGGCGAGCACACCAGCTTCGATAAGCTGCCA<br>AAAGATGTTATGGCAACCTACCGCGGTACTGCGTTCGGTTCTGATGACGCGGGCGGTAAGTTGAC<br>CTACACCATCGACTTCGCCGCGAAACAAGGCCACGGCAAGATCGAGCACCTGAAATCTCCGGAGC<br>TGAATGTGGATCTGGCAGTGGCGTACATTAAACCGGACGAGAAGCACCACGCGGTTATTTCCGGC<br>AGTGTTCTGTATAACCAAGATGAAAAGGGTAGCTATAGCCTGGGCATCTTTGGTGAGAAGGCTCA<br>AGAAGTGGCGGGCTCTGCCGAAGTTGAAACCGCTAATGGCATTCATCACATTGGTTTGGCTGCGA<br>AACAAGGTGGTTCGGGCGGTGAGGCAGCGGCTAAAGTCACCGCTGACATCGGCACGGGCTTGGC<br>GGACGCCCTCACGGCTCCGCTGGATCACAAGGATAAGGGCCTGAAGTCCCTAACGCTGGAAGATT<br>CCATTTCACAGAACGGCACATTGACCCTGTCCGCGCAGGGCGCAGAGAAGACCTACGGCAATGG<br>AGACAGCCTGAACACCGGTAAACTGAAAAACGATAAAGTGAGCCGCTTCGACTTCATCCGTCAGA<br>TCGAGGTAGACGGTCAGCTTATTACCCTTGAAAGCGGGGAGTTTCAGGTGTATAAACAAAGCCAT<br>TCTGCACTGACTGCGCTGCAGACCGAACAGGAGCAGGATCCGGAACACTCGGAGAAAATGGTGG |

(continued)

| Polypeptide name | Nucleic acid sequence |
| --- | --- |
| | CAAAACGTCGTTTCCGCATTGGTGATATCGCGGGCGAACACACTGCGTTTAATCAACTGCCTGAC GGCAAGGCGGAATATCATGGTAAGGCCTTCAGCAGCGATGACGCGGGTGGTAAGTTAACTTACAC CATCGATTTTGCAGCGAAACAAGGTCACGGTAAGATCGAGCACCTGAAAACCCCGGAACAAAAC GTGGAACTGGCAGCGGCTGAGCTGAAGGCGGATGAGAAGAGCCATGCGGTTATTCTGGGTGACA CGCGTTATGGTAGCGAAGAAAAGGGTACGTACCATCTGGCGTTATTTGGTGATCGTGCGCAAGAG ATCGCAGGCTCCGCCACCGTTAAAATCGGTGAAAAGGTTCATGAAATTGGGATCGCCGGTAAGCA GGGCTCTGGCGGCTCTGGCGCACATATCGTGATGGTCGACGCCTATAAACCGACCAAACATCACC ATCACCATCACTAATGA (SEQ ID NO:39) |

(continued)

| Polypeptide name | Nucleic acid sequence |
|---|---|
| **RD012-20** | TCTAGCGGGTCAGGAAGTGGCGGAGGTGGAGTTGCGGCAGATATAGGCACGGGCTTGGCCGACG<br>CCCTGACAGCTCCACTGGACCACAAGGATAAAGGGCTCAAGTCTTTAACGCTTGAGGATTCCATT<br>AGCCAAAACGGCACCCTGACTCTGTCCGCTCAGGGTGCGGAAAAAACTTTTAAAGTTGGTGACAA<br>AGACAACTCTTTGAACACCGGCAAACTCAAAAACGATAAGATTAGCCGTTTTGACTTCGTGCAGA<br>AAATTGAAGTCGACGGCCAAACCATTACCCTGGCGTCAGGCGAGTTCCAAATTTATAAACAAGAC<br>CACTCGGCGGTGGTGGCGCTGCAAATTGAGAAGATCAACAACCCGGATAAAATTGATAGCTTGAT<br>TAATCAGCGTTCGTTCCTGGTTAGCGGTCTGGGTGGTGAGCACACCTCCTTTGATAAGTTGCCGAA<br>AGACGTGATGGCAACCTACAGAGGTACCGCCTTCGGCTCGGATGATGCGGGTGGTAAATTGACGT<br>ATACCATCGATTTCGCCGCGAAGCAAGGTCATGGTAAGATCGAGCACCTGAAGTCCCCGGAACTG<br>AACGTGGACCTGGCTGTTGCATACATCAAACCGGACGAGAAACACCACGCGGTCATCTCCGGTTC<br>AGTTCTGTACAATCAGGATGAAAAGGGTAGCTATAGCCTGGGCATATTTGGTGAAAAAGCGCAAG<br>AAGTAGCAGGCAGCGCGGAGGTGGAAACCGCTAATGGCATTCATCACATCGGCTTGGCTGCAAA<br>GCAGGAGGCGGCAGCGAAAGAAGCAGCTGCGAAGGTGACCGCGGACATCGGCACTGGTCTGGCT<br>GATGCCCTCACTGCGCCTCTGGACCACAAGGATAAGGGTTTGAAGTCCCTCACCCTGGAGGACAG<br>CATCAGCCAAAATGGTACGCTGACCCTGAGCGCTCAGGGCGCGGAGAAGACCTACGGTAATGGT<br>GATTCTCTGAATACCGGTAAGCTGAAGAACGACAAGGTGTCTCGCTTCGACTTCATCCGTCAGATT<br>GAGGTTGACGGCCAGCTGATTACCTTGGAAAGCGGTGAGTTCCAGGTTTATAAACAAAGCCATTC<br>CGCGCTGACCGCGCTGCAAACCGAACAGGAACAGGATCCGGAGCACAGCGAAAAAATGGTCGCG<br>AAGCGCCGCTTTCGTATCGGTGATATCGCAGGTGAGCACACCGCCTTTAACCAGCTGCCGGATGG<br>CAAAGCGGAGTATCATGGTAAGGCGTTTAGCAGCGATGACGCGGGAGGTAAGTTGACCTACACG<br>ATCGACTTCGCAGCGAAGCAGGGTCACGGTAAGATCGAGCACCTGAAGACCCCGGAGCAAAACG<br>TTGAATTGGCCGCGGCTGAACTGAAAGCGGACGAGAAATCTCATGCAGTGATTCTGGGGGACACC<br>CGTTATGGCAGCGAGGAAAAAGGTACGTACCATCTGGCGTTGTTTGGCGATCGTGCCCAAGAAAT<br>CGCCGGTAGCGCGACCGTTAAAATCGGCGAAAAAGTTCATGAGATCGGCATTGCCGGCAAGCAG<br>GGTAGTGGTGGCAGCGGTGCTCATATCGTGATGGTAGACGCTTACAAACCGACCAAACACCATCA<br>TCACCACCACTAATGA (SEQ ID NO:40) |

(continued)

| Polypeptide name | Nucleic acid sequence |
|---|---|
| **RD012-21** | AGTTCAGGTGGAGGGGGAGTAGCAGCTGACATCGGTGCAGGCCTCGCAGACGCGCTGACCGCGC CGCTTGATCATAAGGATAAGAGCCTGCAGAGCCTGACATTGGATCAGTCCGTGCGTAAAAATGAA AAGTTGAAGCTAGCGGCCCAAGGTGCGGAAAAGACCTACGGCAACGGCGACTCCTTGAACACCG GTAAGCTGAAGAACGACAAGGTGAGCAGATTCGACTTCATCCGCCAGATCGAAGTCGACGGTCA GCTGATTACCTTGGAATCTGGCGAGTTTCAGATCTATAAGCAGGACCACAGCGCGGTCGTGGCCC TGCAGATTGAGAAGATCAACAACCCGGATAAGATCGATAGCTTGATCAATCAACGTAGCTTCCTG GTTAGTGGTTTGGGCGGCGAGCACACCTCATTTGACAAACTGCCAAAAGATGTTATGGCTACCTA CCGCGGTACGGCTTTCGGCTCCGACGACGCCGGTGGCAAGCTGACTTACACCATTGATTTCGCAG CAAAACAAGGTCACGGTAAAATCGAGCACCTGAAAAGCCCGGAGCTGAACGTGGATCTGGCGGT AGCTTACATCAAACCGGATGAGAAACATCACGCCGTCATCAGCGGTTCTGTTCTGTATAACCAAG ATGAGAAAGGTTCCTATAGCCTGGGCATTTTTGGTGAGAAAGCGCAAGAAGTTGCGGGTTCTGCG GAAGTCGAAACCGCCAATGGTATTCATCACATCGGCCTGGCGGCAAAGCAAGGCAGCGGCGGTG GCGGGGTGACCGCTGATATCGGCACGGGTTTAGCGGACGCCCTCACGGCTCCGCTGGATCATAAA GACAAAGGTCTGAAGTCTTTGACCCTGGAGGATTCAATTAGCCAGAACGGTACTCTGACGCTGAG CGCGCAGGGGGCCGAAAAGACCTACGGAAATGGTGATTCTTTGAATACCGGCAAACTGAAAAAC GATAAAGTTTCTCGCTTCGACTTTATCCGTCAGATTGAGGTGGACGGCCAGCTGATTACTCTCGAG TCGGGCGAGTTCCAGGTTTATAAACAATCCCATTCCGCGCTGACCGCGCTGCAAACCGAACAAGA GCAGGATCCGGAACACAGCGAAAAGATGGTTGCCAAACGTCGTTTTCGTATTGGTGACATCGCGG GCGAACACACCGCGTTCAACCAGCTGCCGAGCGGTAAGGCTGAGTATCACGGCAAGGCGTTTAGC AGCGACGACGCAGGTGGTAAGCTGACGTACACCATTGACTTCGCCGCGAAACAAGGTCATGGTA AGATTGAGCATCTGAAAACTCCGGAGCAAAATGTTGAATTGGCGAGCGCTGAGCTGAAGGCAGA TGAAAAGAGCCACGCAGTGATTCTGGGTGACACCCGTTATGGTTCGGAGGAAAAAGGCACCTACC ATCTGGCATTGTTTGGCGACCGCGCACAGGAGATCGCGGGTTCGGCGACCGTTAAAATTCGTGAA AAAGTTCATGAAATCGGCATCGCTGGCAAGCAGGGCAGCGGTGGCTCCGGAGCGCATATCGTGAT GGTGGACGCGTACAAGCCGACCAAACACCACCACCACCACCACTAATGA (SEQ ID NO:41) |

(continued)

| Polypeptide name | Nucleic acid sequence |
|---|---|
| **RD012-22** | TCTAGCGGGTCAGGAAGTGGCGGAGGTGGGGTGGCGGCCGACATCGGCACTGGTTTGGCGGACG<br>CGCTGACGGCTCCGTTGGACCACAAAGACAAAGGTTTGAAGTCCCTGACGTTAGAGGACTCGATC<br>TCTCAGAACGGTACTCTAACGCTGAGCGCGCAAGGTGCAGAGAAGACCTTTAAAGTGGGTGACA<br>AGGACAACAGCTTGAATACCGGTAAACTGAAGAACGACAAGATCAGCAGATTCGACTTCGTCCA<br>GAAAATCGAGGTGGACGGCCAGACCATTACCCTGGCGAGCGGTGAGTTCCAGATTTATAAACAA<br>GATCATAGCGCTGTCGTGGCGCTGCAGATTGAGAAGATCAACAACCCGGATAAGATCGACAGCCT<br>GATTAACCAGCGTAGCTTTCTGGTTAGCGGTCTTGGCGGCGAACACACCAGCTTTGATAAATTACC<br>GAAAGATGTGATGGCAACCTACCGCGGCACTGCGTTTGGTTCTGATGATGCAGGCGGTAAACTGA<br>CCTACACCATCGACTTCGCAGCCAAGCAAGGCCACGGTAAGATCGAGCACCTGAAGAGCCCGGA<br>ATTAAATGTCGACCTGGCGGTGGCATACATTAAACCAGACGAAAAGCACCACGCTGTTATTTCTG<br>GTAGTGTTCTGTATAACCAGGATGAGAAGGGCTCATATTCCCTGGGCATCTTCGGTGAGAAGGCG<br>CAGGAGGTGGCCGGTTCTGCGGAGGTGGAAACCGCAAATGGCATTCATCACATCGGTCTGGCGGC<br>TAAGCAAGAAGCAGCTGCGAAAGAAGCGGCGGCGAAAGAAGCCGCGGCCAAGGTCACCGCGGA<br>CATCGGCACTGGCCTGGCTGATGCTCTGACAGCACCGCTGGATCACAAAGACAAAGGCTTGAAGT<br>CGCTGACCTTGGAAGATAGCATCTCGCAAAACGGCACCCTCACGTTGAGCGCGCAGGGTGCGGAA<br>AAGACGTACGGCAATGGTGACTCCCTCAACACCGGCAAATTGAAGAATGATAAGGTGAGCCGTTT<br>TGACTTCATCCGTCAGATTGAAGTTGACGGTCAGCTGATTACCCTGGAGAGCGGTGAATTTCAGG |

(continued)

| Polypeptide name | Nucleic acid sequence |
|---|---|
| | TTTATAAACAAAGCCATTCTGCGTTAACGGCTCTGCAAACCGAACAAGAGCAGGATCCGGAACAC AGCGAAAAGATGGTTGCGAAGCGCCGTTTTCGTATTGGCGATATCGCCGGTGAGCACACCGCGTT TAATCAACTGCCGGACGGCAAAGCGGAATATCATGGGAAAGCATTCAGCAGCGATGATGCGGGT GGCAAGTTGACCTACACCATCGATTTCGCCGCGAAGCAGGGTCATGGTAAGATTGAGCACCTGAA AACCCCGGAACAAAACGTTGAGCTGGCGGCGGCGGAGTTAAAAGCGGACGAGAAAAGCCACGCC GTGATTCTGGGTGATACCCGTTATGGCTCCGAGGAAAAGGGTACGTACCATCTGGCCCTCTTCGG CGATCGCGCTCAGGAGATTGCTGGTTCGGCGACCGTTAAAATCGGTGAGAAAGTTCATGAAATCG GCATTGCAGGTAAACAAGGCTCCGGCGGTTCCGGCGCACATATTGTTATGGTAGATGCGTACAAG CCGACCAAACATCACCACCATCACCATTAATGA (SEQ ID NO:42) |

(continued)

| Polypeptide name | Nucleic acid sequence |
|---|---|
| **RD012-23** | TCTAGCGGGTCAGGAAGTGGCGGAGGTGGGGTGGCGGCGGACATCGGCACCGGTCTTGCGGATG CGCTGACCGCTCCACTGGATCATAAAGACAAAGGTCTGAAGTCTCTGACCTTGGAGGATTCTATT AGCCAGAACGGTACGTTGACTCTGAGCGCGCAGGGTGCTGAAAAGACTTTTAAAGTGGGTGACA AGGATAATAGCTTGAACACCGGTAAGCTCAAGAACGACAAGATCTCCCGCTTCGACTTTGTTCAG AAAATCGAGGTGGACGGCCAGACGATTACCCTGGCGAGCGGTGAGTTCCAGATTTATAAACAAG ATCATTCCGCGGTTGTTGCACTGCAGATCGAGAAGATTAACAACCCGGATAAGATCGATTCTCTG ATCAATCAGCGTAGCTTTCTGGTTAGCGGTCTCGGTGGTGAGCACACTTCATTTGACAAGCTGCCG AAAGACGTGATGGCCACGTACCGCGGTACAGCCTTCGGCAGCGACGACGCTGGCGGTAAACTGA CGTACACCATTGACTTCGCCGCTAAACAGGGTCACGGTAAAATCGAGCACCTGAAAAGCCCGGAA CTGAATGTTGATCTGGCAGTGGCGTATATTAAACCGGACGAGAAACACCACGCGGTCATCTCGGG CTCTGTTCTGTACAATCAGGACGAAAAGGGTTCCTATAGCTTGGGTATTTTTGGTGAGAAAGCCCA AGAAGTGGCGGGCTCTGCTGAGGTCGAAACCGCGAATGGCATTCATCACATCGGCCTGGCTGCGA AGCAAGAGGCTGCAGCCAAAGAAGCCGCCGCGAAAGAGGCGGCTGCGAAAGAAGCCGCGGCGA AGGTCACCGCGGATATCGGCACGGGTCTGGCTGACGCGCTTACCGCTCCGCTGGACCACAAGGAC AAAGGTTTGAAGTCCTTAACCCTGGAAGATTCCATCAGCCAAAACGGTACCTTGACCTTAAGCGC GCAAGGTGCGGAAAAGACCTACGGAAACGGCGACAGCCTGAATACCGGCAAGTTGAAAAACGAT AAGGTGAGCAGATTCGACTTCATTCGTCAGATTGAGGTTGATGGTCAGCTGATTACCCTGGAGAG CGGCGAGTTTCAAGTCTATAAACAATCCCATAGCGCATTGACTGCGCTACAAACCGAACAAGAAC AGGATCCGGAGCACAGCGAAAAAATGGTTGCGAAACGTCGTTTCCGCATTGGCGACATCGCCGGT GAGCACACCGCATTCAACCAGCTGCCGGATGGCAAGGCCGAATATCATGGTAAGGCGTTTAGCAG CGATGATGCAGGCGGTAAATTGACGTACACCATCGACTTCGCTGCAAAGCAGGGTCACGGCAAG ATCGAGCACCTGAAGACCCCTGAACAAAACGTGGAGCTGGCGGCAGCCGAACTGAAGGCTGATG AAAAAAGTCATGCAGTTATCCTGGGCGACACCCGTTATGGTTCGGAGGAAAAGGGCACGTACCAC CTGGCGTTGTTTGGCGATCGTGCACAGGAGATTGCAGGTTCTGCGACCGTAAAAATCGGTGAAAA GGTGCATGAAATTGGTATCGCAGGTAAGCAAGGCTCAGGAGGCTCTGGCGCGCATATCGTGATGG TTGATGCATACAAACCGACCAAACATCATCACCACCACCACTAATGA (SEQ ID NO:43) |

(continued)

| Polypeptide name | Nucleic acid sequence |
|---|---|
| **RD012-24** | AGTTCAGGTGGAGGGGGAGTAGCAGCTGACATCGGTGCTGGCTTGGCGGATGCCCTGACCGCGCC TCTGGATCACAAAGACAAAAGCCTGCAGTCGCTGACCCTGGACCAGAGCGTTCGTAAAAATGAA AAGTTGAAGTTGGCTGCCCAGGGCGCCGAGAAAACCTACGGCAACGGCGATTCGCTTAACACCG |

(continued)

| Polypeptide name | Nucleic acid sequence |
| --- | --- |
| | GTAAGTTGAAGAACGACAAAGTCTCACGTTTCGATTTCATCCGTCAGATTGAAGTGGACGGTCAACTGATCACCCTGGAATCTGGCGAGTTCCAGATCTATAAACAGGACCACTCTGCTGTGGTTGCACTGCAAATCGAGAAGATCAACAACCCGGATAAAATCGACAGCCTGATTAACCAGCGTAGCTTCTTGGTTTCTGGTCTGGGTGGTGAGCACACTTCGTTTGATAAACTGCCGAAGGATGTTATGGCTACCTACCGCGGTACAGCGTTCGGCTCCGACGACGCGGGCGGTAAACTCACGTACACCATTGACTTTGCGGCTAAGCAAGGTCATGGTAAGATTGAGCACCTGAAGAGCCCGGAGCTGAACGTGGACCTGGCGGTGGCATACATTAAACCGGATGAGAAGCACCATGCGGTGATCAGCGGTTCCGTGCTGTATAACCAGGACGAGAAAGGCTCCTACAGCCTCGGTATTTTTGGCGAAAAGGCGCAGGAGGTGGCCGGTAGCGCTGAGGTTGAAACCGCGAATGGAATCCATCACATCGGCCTGGCGGCAAAACAGGAGGCTGCTGCAAAAGAAGCAGCGGCGAAGGTCACCGCAGACATTGGTACGGGTTTAGCTGATGCCCTGACTGCGCCACTGGATCACAAAGATAAGGGTCTTAAGTCCCTGACGTTAGAAGATTCCATCAGCCAGAACGGTACGCTGACGCTGAGCGCGCAAGGCGCAGAGAAAACCTACGGTAATGGCGACTCTTTAAATACCGGTAAGCTGAAGAATGATAAAGTTTCTCGCTTCGACTTCATACGCCAGATTGAGGTCGACGGCCAACTGATTACCCTGGAGAGCGGAGAATTTCAAGTGTATAAACAAAGCCATAGCGCTTTGACCGCGCTCCAAACCGAACAAGAGCAAGATCCGGAACACTCCGAAAAAATGGTGGCGAAACGTCGTTTTCGTATTGGCGACATTGCGGGTGAGCACACGGCCTTCAACCAGCTTCCGAGCGGCAAGGCGGAATATCATGGTAAAGCGTTTAGCAGCGATGATGCGGGTGGTAAATTGACTTACACCATCGACTTCGCAGCGAAGCAAGGTCATGGTAAGATCGAGCACCTGAAGACCCCGGAACAGAATGTTGAATTGGCGAGCGCGGAGCTGAAAGCAGACGAGAAGTCCCATGCTGTGATCCTGGGCGACACCCGTTATGGCAGCGAGGAAAAGGGCACCTACCATCTGGCGTTGTTTGGTGATAGAGCCCAAGAGATCGCAGGCTCTGCTACCGTAAAAATCCGCGAAAAGGTTCATGAAATTGGGATCGCTGGCAAGCAGGGTAGCGGTGGCAGTGGCGCGCATATTGTTATGGTTGACGCGTATAAGCCGACCAAACACCACCACCATCACCACTAATGA (SEQ ID NO:44) |

(continued)

| Polypeptide name | Nucleic acid sequence |
|---|---|
| **RD012-25** | TCAAGTGGTGGAGGAGGGGTAGCAGCTGACATCGGTGCGGGATTGGCAGATGCGCTTACGGCGC<br>CACTGGACCACAAAGATAAAAGCCTCCAATCGCTGACCTTAGACCAGTCCGTTCGTAAAAACGAA<br>AAGCTCAAGCTGGCCGCCCAAGGTGCAGAGAAGACGTACGGAAACGGCGACTCATTGAATACCG<br>GTAAGCTGAAGAACGATAAAGTGTCCCGCTTCGACTTCATCCGTCAGATTGAAGTGGACGGCCAG<br>CTGATCACCTTGGAGTCAGGCGAGTTCCAGATTTACAAGCAAGATCATAGCGCGGTCGTGGCTCT<br>TCAAATTGAGAAAATCAACAACCCGGACAAGATCGACTCCCTGATCAATCAGCGTAGCTTTCTGG<br>TTTCCGGTCTGGGCGGTGAACATACCAGCTTCGATAAATTGCCGAAAGACGTTATGGCTACCTAC<br>CGCGGTACGGCCTTCGGTTCCGACGATGCGGGTGGTAAACTGACCTACACCATTGATTTCGCAGC<br>AAAACAGGGCCACGGCAAGATTGAGCACCTGAAGAGTCCGGAACTGAACGTCGACCTGGCGGTG<br>GCGTACATTAAACCGGATGAAAAGCACCACGCCGTCATTAGCGGTTCCGTGCTGTATAACCAAGA<br>CGAGAAAGGTTCTTATAGCCTAGGTATCTTTGGTGAGAAGGCTCAAGAGGTTGCTGGTTCTGCTG<br>AGGTGGAAACCGCTAATGGTATCCATCACATTGGCCTAGCGGCTAAGCAGGAGGCCGCAGCCAA<br>AGAGGCTGCCGCGAAAGAAGCAGCTGCGAAAGTCACCGCTGATATCGGTACTGGTCTGGCAGAT<br>GCGTTGACCGCACCGCTGGATCACAAGGACAAGGGTCTTAAGTCGCTGACTCTGGAAGATTCGAT<br>TAGCCAGAATGGCACCCTGACGCTGAGCGCGCAAGGTGCAGAAAAAACCTACGGCAACGGCGAC |

(continued)

| Polypeptide name | Nucleic acid sequence |
|---|---|
| | TCTTTGAATACCGGTAAGCTGAAGAACGACAAAGTTTCCAGATTCGACTTTATCCGCCAGATCGA GGTTGATGGTCAACTGATTACCCTGGAGAGCGGTGAGTTCCAGGTTTATAAGCAGAGCCACAGCG CGTTGACGGCGCTCCAAACCGAACAGGAGCAGGATCCGGAACACTCTGAAAAGATGGTGGCTAA ACGTCGTTTTCGTATCGGCGACATCGCGGGCGAACACACCGCTTTTAATCAGCTGCCGAGCGGGA AAGCGGAGTATCATGGCAAAGCGTTTAGCAGCGATGATGCGGGTGGTAAACTGACGTACACCATT GACTTCGCGGCCAAACAGGGTCATGGCAAGATCGAGCATCTGAAGACTCCGGAACAAAACGTGG AACTGGCGAGCGCGGAACTGAAAGCGGACGAGAAAAGCCATGCGGTGATCTTGGGTGATACCCG TTATGGCAGCGAGGAAAAGGGTACGTACCATCTGGCTTTGTTTGGTGATCGTGCACAGGAGATCG CCGGTTCTGCGACCGTTAAAATCCGCGAAAAGGTGCATGAAATAGGCATTGCAGGCAAGCAAGG CTCTGGCGGCAGCGGCGCGCATATTGTTATGGTTGACGCATATAAACCGACCAAACACCACCACC ACCACCATTAATGA (SEQ ID NO:45) |

(continued)

| Polypeptide name | Nucleic acid sequence |
|---|---|
| **RD012-26** | AGTTCAGGTGGAGGGGGAGTAGCAGCTGACATCGGCGCTGGCCTTGCTGATGCGCTCACGGCTCC GCTGGATCACAAAGACAAGTCCCTGCAATCCTTGACGCTCGACCAGAGCGTTCGTAAAAACGAAA AGTTGAAGCTGGCGGCACAGGGCGCGGAGAAGACTTACGGCAACGGTGACTCCTTGAACACCGG CAAGCTGAAGAATGATAAAGTATCCCGTTTTGACTTTATACGTCAAATCGAGGTGGACGGTCAGC TGATTACCCTTGAGAGCGGCGAGTTCCAAATCTACAAACAAGACCATAGCGCAGTTGTTGCCCTG CAGATTGAGAAGATCAACAACCCGGATAAGATCGATAGCCTGATCAATCAACGTAGCTTCCTGGT GTCTGGTCTGGGTGGTGAACACACCAGCTTTGACAAACTTCCGAAAGATGTTATGGCAACCTACC GCGGTACTGCCTTCGGCTCTGATGATGCGGGCGGTAAATTAACCTATACCATTGACTTTGCTGCGA AACAAGGTCATGGTAAGATCGAGCACCTGAAGTCTCCGGAATTGAACGTTGATCTGGCGGTCGCG TACATCAAACCGGATGAAAAACACCACGCAGTCATCTCTGGGTCCGTGCTGTATAACCAGGATGA GAAAGGCTCTTATAGCCTGGGTATTTTTGGTGAGAAGGCCCAAGAGGTTGCGGGTTCCGCGGAGG TGGAAACCGCTAATGGTATTCATCACATTGGCTTGGCAGCCAAACAAGAAGCAGCGGCGAAAGA AGCGGCGGCGAAAGAGGCCGCGGCCAAAGAAGCAGCGGCTAAAGTCACCGCGGACATTGGCACC GGTCTGGCGGATGCGCTGACTGCACCACTGGACCATAAAGACAAGGGTTTGAAGAGCTTGACGCT GGAAGATAGCATCAGCCAGAACGGCACCTTGACCTTATCAGCGCAGGGCGCGGAGAAAACCTAC GGTAATGGCGACAGCCTGAACACTGGCAAGCTGAAGAACGATAAGGTGAGCCGTTTCGACTTCAT CCGCCAAATTGAGGTGGACGGCCAGCTGATTACCCTGGAAAGCGGTGAGTTCCAGGTGTATAAGC AGAGCCATAGTGCATTAACGGCGCTGCAGACCGAACAAGAACAGGATCCGGAGCACTCGGAAAA GATGGTTGCCAAACGTCGTTTCAGAATTGGCGATATCGCCGGCGAGCATACCGCCTTTAATCAGC TGCCAAGCGGTAAGGCGGAGTATCACGGCAAAGCGTTCAGCAGCGACGACGCAGGCGGCAAATT GACCTACACCATTGACTTCGCCGCTAAACAGGGTCATGGTAAAATCGAGCACTTGAAGACCCCGG AGCAGAATGTTGAACTGGCTAGCGCTGAGCTGAAGGCCGACGAAAAGAGCCATGCAGTGATTCT GGGTGATACCCGTTATGGTTCTGAAGAAAAGGGTACGTACCATCTGGCGCTGTTTGGTGACCGCG CACAGGAGATCGCTGGTTCGGCGACCGTTAAAATTCGCGAAAAGGTGCACGAAATCGGCATCGC AGGCAAGCAAGGTTCCGGTGGCTCCGGCGCGCATATCGTGATGGTTGACGCGTACAAACCGACGA AACACCACCACCACCACCACTAATGA (SEQ ID NO:46) |

In some embodiments, in any one of the immunogenic complexes provided herein, the linker peptide 1 comprises the amino acid sequence of $(GGGGS)_n$, $(EAAAK)_n$, $(GSGGSG)_n$, or $(GGS)_n$, wherein n may be an integer greater than 0 and less than or equal to 5. In some embodiments, in any one of the immunogenic complexes provided herein, the linker peptide 1 is preferably GSGGSG (SEQ ID NO: 53).

In some embodiments, in any one of the immunogenic complexes provided herein, the linker peptide 2 comprises the amino acid sequence of (GGS)n, (GGGGS)n, (EAAAK)n, or (GSGGSG)n, wherein n may be an integer greater than 0 and less than or equal to 10. In some embodiments, in any one of the immunogenic complexes provided herein, the linker peptide 2 is preferably GGSGGSGGS (SEQ ID NO: 49) or GGSGGSGGSGGS (SEQ ID NO: 50).

In some embodiments, in any one of the above immunogenic complexes provided herein, the particle protein component is a fusion protein formed by fusing the nanoparticle protein, at the N-terminus, with the binding peptide 2 via the linker peptide 2; preferably, the nanoparticle protein is NPM, AP205 capsid protein 3 (AP205), or ferritin protein. Specifically, in some optional embodiments, the binding peptide 2 (designated as "4C") is linked to the coding gene of the nanoparticle protein via the linker peptide 2, and the construct is inserted into a prokaryotic expression vector (such as pET-28a(+) or pET-30a(+)) for expression in *E. coli* cells, so as to obtain a fusion protein of the binding peptide 2 and the nanoparticle protein. The fusion protein may be purified by chromatography (e.g., anion exchange chromatography and hydrophobic chromatography) to obtain a product. The nanoparticle protein is preferably NPM, AP205, or ferritin; the formed particle protein components are designated as NPM-4C, AP205-4C, and ferritin-4C.

Preferably, in the immunogenic complex provided herein, the amino acid sequence of the antigenic component is set forth in any one of SEQ ID NOs: 1, 2, and 17-23, and the amino acid sequence of the particle protein component is set forth in SEQ ID NO: 52.

Specifically, in some optional embodiments, any one of the above antigenic components is subjected to a conjugation binding reaction with the particle protein component under suitable reaction conditions (e.g., at room temperature), and the coupling is achieved by the covalent bonding of the binding peptide 1 of the antigenic component and the binding peptide 2 of the particle protein component, thereby forming the immunogenic complex. Immunogenic complexes formed with different nanoparticle proteins, such as NPM, AP205, or ferritin, are designated fHBP-NPM, fHBP-AP205, or fHBP-ferritin, respectively.

In some embodiments, the present disclosure provides a method for a covalent binding reaction of an antigenic component and a particle protein component, wherein the antigenic component and the particle protein component are mixed in a protein concentration ratio of 6:1 as determined by the BCA method, a 50% sucrose stock solution is added at a final sucrose concentration of about 25%, and a 1 M Tris-HCl stock solution pH 7.4 is added at 10% of the total reaction volume to stabilize the pH. The mixture is left to react at 22 °C for 24 h. All endotoxin measurements are below 100 EU/mL, meeting the requirements for mass production.

In some embodiments, the present disclosure provides an immunogenic complex, comprising:

(1) an antigenic component, comprising an fHBP protein, a linker peptide 1, and a binding peptide 1; and
(2) a particle protein component, comprising a nanoparticle protein, a linker peptide 2, and a binding peptide 2.

The linker peptide 1 is any linker peptide (e.g., a flexible linker peptide or a rigid linker peptide) commonly used in the art, including but not limited to, the amino acid sequences of $(GGS)_n$, $(GSGGSG)_n$, $(GGGGS)_n$, and $(EAAAK)_n$. The linker peptide 1 is preferably GSGGSG (SEQ ID NO: 53).

The linker peptide 2 is any linker peptide commonly used in the art (e.g., a flexible linker peptide or a rigid linker peptide), including, but not limited to, the amino acid sequences of $(GGS)_n$, (GGGGS)n, (EAAAK)n, and (GSGGSG)n, wherein n may be an integer greater than 0 and less than or equal to 10, preferably, GGSGGSGGS (SEQ ID NO: 49) or GGSGGSGGSGGS (SEQ ID NO: 50); the nanoparticle protein is NPM, AP205, or ferritin. In some embodiments, in any one of the immunogenic complexes provided herein, the nanoparticle protein NPM comprises the amino acid sequence set forth in SEQ ID NO: 51.

Preferably, in any one of the immunogenic complexes provided herein, the particle protein component comprises NPM-4C, as set forth in SEQ ID NO: 52, which comprises a fusion protein obtained by linking the binding peptide 2 set forth in SEQ ID NO: 48 to the nanoparticle protein NPM set forth in SEQ ID NO: 51 via the linker peptide 2.

Further, the present disclosure provides a method for preparing an immunogenic complex, comprising:

(1) separately ligating coding genes of an fHBP antigenic component and a particle protein component into expression vectors to construct recombinant expression plasmids;
(2) constructing a recombinant strain capable of expressing the fHBP antigenic component and the particle protein component in the host cell;
(3) expressing fusion proteins using the recombinant strain and purifying the recombinant fusion proteins; and
(4) subjecting the antigenic component and the particle protein component described above to a covalent binding reaction, so as to obtain the immunogenic complex.

Preferably, the immunogenic complex obtained in step (4) above is purified to obtain a vaccine drug substance. Preferably, in step (1) of the method for preparing the immunogenic complex for preventing or treating the disease related to *N. meningitidis* group B, the plasmid expressing the *N. meningitidis* group B antigenic component may be selected from pcDNA3.4, and the vector expressing the nanoparticle may be selected from pET-28a(+) or pET-30a(+).

In some embodiments, the immunogenic complex provided herein can induce the production of a neutralizing antibody, a protective antibody, or a bactericidal antibody.

In some embodiments, the immunogenic complex provided herein can be used for preparing a neutralizing antibody, a protective antibody, or a bactericidal antibody.

The neutralizing antibody, the protective antibody, or the bactericidal antibody described above refers to an antibody, preferably IgG, that can bind to a corresponding living bacterium and then activate the complement system, resulting in the lysis and death of the bacterium.

In step (2) of the method for preparing the immunogenic complex for preventing or treating the disease related to *N. meningitidis* group B disclosed herein, the host cell expressing the *N. meningitidis* group B antigen is CHO, and the host cell expressing the particle protein vector is *E. coli*.

In step (3) of the method for preparing the immunogenic complex for preventing or treating the disease related to *N. meningitidis* group B disclosed herein, reference can be made to Patent No. CN114395015B for the method for purifying the particle protein.

**E. Nucleic acid**

The present disclosure provides coding nucleotides for the fHBP proteins and the antigenic components described above, and the specific nucleotide sequences are readily available to those skilled in the art by conventional means such as codon tables.

The present disclosure provides coding nucleotides for the particle protein components and the nanoparticle proteins described above, and the specific nucleotide sequences are readily available to those skilled in the art by conventional means such as codon tables.

Preferably, the nucleotide sequences of the fHBP proteins provided herein are set forth in SEQ ID NOs: 24-46, as specifically shown in Table 3.

The present disclosure also provides vectors comprising the nucleotide sequences of the present disclosure, including cloning or expression vectors, and host cells transformed with the vectors.

In some embodiments, the vector used in the present disclosure includes pcDNA3.4, pET-28a(+), and pET-30a(+).

In some embodiments, the host cell expressing the antigenic component vector is CHO, and the host cell expressing the particle protein vector is *E. coli*.

**F. Immune composition**

The present disclosure provides an immune composition, comprising the fHBP protein or the immunogenic complex of the present disclosure.

Optionally, the immune composition of the present disclosure further comprises a pharmaceutically acceptable carrier.

Preferably, the pharmaceutically acceptable carrier comprises a stabilizer, an excipient, a surfactant, a buffering agent, and a pH regulator; the stabilizer is sucrose and/or arginine, the excipient is mannitol, the surfactant is Tween 80, the buffering agent is disodium hydrogen phosphate dihydrate and/or sodium dihydrogen phosphate dihydrate, and the pH regulator is hydrochloric acid.

In some embodiments, the immune composition provided herein is an injection or a lyophilized formulation, preferably a lyophilized formulation.

In some embodiments, the immune composition described herein comprises one or more of the fHBP proteins described above.

In some embodiments, the present disclosure provides an immune composition, wherein the immune composition is obtained by mixing the fHBP chimeric proteins described above, and the mixture has immunogenicity superior to that of a simple mixture of the three fHBP variants (i.e., V1, V2, and V3). Preferably, the immune composition of the present disclosure is obtained by mixing the fHBP chimeric proteins described above in equal mass ratios.

In some embodiments, the immune composition provided herein comprises chimeric protein fHBP V3 domain 1-fHBP V1 domain 2 and chimeric protein fHBP V1 domain 1-fHBP V2 domain 2.

In some embodiments, the immune composition provided herein comprises chimeric protein fHBP V2 domain 1-fHBP V1 domain 2 and chimeric protein fHBP V1 domain 1-fHBP V3 domain 2.

In some embodiments, the immune composition described herein comprises fHBP V1, fHBP V2, and fHBP V3

described above.

Preferably, the immune composition is obtained by mixing fHBP V1, fHBP V2, and fHBP V3 described above in equal mass ratios.

In some embodiments, the immune composition described herein comprises one or more of the immunogenic complexes described above. Preferably, the immune composition disclosed herein comprises the following two immunogenic complexes: (1) an immunogenic complex formed by the antigenic component set forth in SEQ ID NO: 5 and the particle protein component set forth in SEQ ID NO: 52; and (2) an immunogenic complex formed by the antigenic component set forth in SEQ ID NO: 6 and the particle protein component set forth in SEQ ID NO: 52.

Preferably, the immune composition disclosed herein comprises the following three immunogenic complexes: (1) an immunogenic complex formed by the antigenic component set forth in SEQ ID NO: 7 and the particle protein component set forth in SEQ ID NO: 52; (2) an immunogenic complex formed by the antigenic component set forth in SEQ ID NO: 8 and the particle protein component set forth in SEQ ID NO: 52; and (3) an immunogenic complex formed by the antigenic component set forth in SEQ ID NO: 9 and the particle protein component set forth in SEQ ID NO: 52.

In some embodiments, the immune composition of the present disclosure can induce the production of a neutralizing antibody, a protective antibody, or a bactericidal antibody.

In some embodiments, the immune composition of the present disclosure can be used for preparing a neutralizing antibody, a protective antibody, or a bactericidal antibody.

The neutralizing antibody, the protective antibody, or the bactericidal antibody described above refers to an antibody, preferably IgG, that can bind to a corresponding living bacterium and then activate the complement system, resulting in the lysis and death of the bacterium.

The protective efficacy against *N. meningitidis* can be measured by conventional means in the art, e.g., epidemiological measurements in clinical trials, or by indirect measures to confirm that the immunogenic composition elicits a serum bactericidal antibody (SBA) response in recipients. In an SBA assay, sera from recipients of the composition are incubated with target bacterium (*N. meningitidis* in the present disclosure) in the presence of complements (preferably human complements, although baby rabbit complements are often used instead), and the killing against the bacterium is assessed at various dilutions of the serum to determine the SBA activity. The results observed in the SBA assay can be corroborated by performing a competitive SBA assay to provide further indirect evidence of the immunogenic activity of the target antigen. In a competitive SBA assay, the serum from a recipient of an immunogenic composition comprising one or more antigens is pre-incubated with the one or more antigens and then incubated with target bacterium in the presence of human complements. The killing of the bacterium is then assessed, and if bactericidal antibodies in the serum of the recipient bind to the target antigen during the pre-incubation period and thus cannot bind to surface antigens on the bacterium, the killing of the bacterium will be reduced or abolished.

### G. Vaccines

The present disclosure provides a vaccine, comprising the immune composition described herein and an adjuvant.

In some embodiments, the adjuvant contained in the vaccine of the present disclosure is selected from at least one of an aluminum salt adjuvant, Freund's complete adjuvant, a propolis adjuvant, a water/oil adjuvant, a cytokine, CpGDNA, flagellin, a genetically engineered toxoid, an immune-stimulating complex, a liposome, a saponin, and a Poly(I:C) adjuvant.

The aluminum salt adjuvant in the present disclosure is an aluminum hydroxide adjuvant, specifically Alhydrogel.

The adjuvant of the present disclosure comprises 3%-5% of squalene, 0.4%-1% of Span 85, 0.4%-1% of Tween 80, 10 mM of citrate or 0.1%-0.5% of sodium citrate, and 0.01%-0.05% of citric acid (w/v). The amount of squalene is preferably 3.5% to 4.5%, more preferably 3.5%, 3.6%, 3.7%, 3.8%, 3.9%, 4.1%, 4.2%, 4.3%, 4.4%, or 4.5% (w/v). The squalene-based adjuvant may be the commercial adjuvant MF59 or SWE. MF59 is a water-in-oil emulsion comprising 4.3% of squalene, 0.5% of Tween 80, and 0.5% of Span 85, and SWE is an oil-in-water emulsion similar in composition to MF59.

The squalene-based water/oil adjuvant used in the specific embodiments of the present disclosure comprises the following components: 0.5% of Span 85, 0.5% of Tween 80, 4.2% of squalene, 0.264% of sodium citrate, and 0.016% of citric acid.

In some embodiments, the vaccine provided herein can induce the production of a neutralizing antibody, a protective antibody, or a bactericidal antibody.

In some embodiments, the vaccine provided herein can be used for preparing a neutralizing antibody, a protective antibody, or a bactericidal antibody.

The neutralizing antibody, the protective antibody, or the bactericidal antibody described above refers to an antibody, preferably IgG, that can bind to a corresponding living bacterium and then activate the complement system, resulting in the lysis and death of the bacterium.

The present disclosure provides a method for preparing the vaccine for preventing or treating the disease related to *N. meningitidis* group B, comprising: subjecting the purified antigenic component to a covalent binding reaction with the particle protein component via the binding peptide 1 and the binding peptide 2, and mixing with a proper adjuvant to obtain an immune composition product. The amount of the immunogenic complex is 0.25-100 μg/dose, preferably 0.5-50 μg/dose, more preferably 0.5 μg/dose, 1 μg/dose, 2 μg/dose, 3 μg/dose, 4 μg/dose, 5 μg/dose, 10 μg/dose, 15 μg/dose, 20 μg/dose, 25 μg/dose, 30 μg/dose, 35 μg/dose, 40 μg/dose, 45 μg/dose, or 50 μg/dose.

In the method for preparing the vaccine for preventing or treating the disease related to *N. meningitidis* group B described herein, the water/oil adjuvant is a squalene-based adjuvant, and the saponin adjuvant comprises QS-21. The drug substance of the vaccine is diluted with a buffer (e.g., TBS) according to dose and then mixed with the adjuvant in a 1:1 volume ratio.

The present disclosure further provides a kit, comprising an *N. meningitidis* group B vaccine, and a device and a container required for administering the vaccine, specifically including: needle/syringe devices, powder containers, and solvent containers. Preferably, the commercial form of the vaccine of the present disclosure is a pre-filled needle with the immune composition being pre-mixed with the adjuvant.

**H. Medical use**

The present disclosure provides medical use of the fHBP protein, the immunogenic complex, the immune composition, and the vaccine of *N. meningitidis* group B described above, comprising preventing or treating a disease related to *N. meningitidis* group B.

The present disclosure provides a pharmaceutical formulation of the fHBP protein, the immunogenic complex, the immune composition, and the vaccine of *N. meningitidis* group B described above for use in preventing or treating a disease related to *N. meningitidis* group B.

The disease related *N. meningitidis* group B in the present disclosure includes, but is not limited to: meningococcal meningitis, septicemia, septic shock, arthritis, myocarditis, pericarditis, endophthalmitis, meningitis, hemorrhagic dermatosis, activated fibrinolysis and blood coagulation, organ dysfunction such as kidney, lung, and heart failure, adrenal hemorrhage and muscle infarction, capillary leakage, edema, peripheral limb ischemia, respiratory distress syndrome.

Preferably, the disease related to *N. meningitidis* group B is meningococcal meningitis.

**I. Beneficial effects**

Compared with the prior art, the present disclosure has the following beneficial effects:

(1) It was found that the fusion proteins of characteristic amino acid sequences of three fHBP variants may have fragment bands, and the molecular sizes of the two bands are close, which is not conducive to production and purification. The present disclosure solves the technical problem by structural optimization, while ensuring the effective immunogenicity, reducing the complexity that may be brought to the production process from the source, improving the production efficiency, and saving the cost of mass production.

(2) In a first aspect, the fusion proteins comprising characteristic amino acid sequences of the three fHBP variants (e.g., RD012-1) of the present disclosure induce superior anti-fHBP immunological effects to those of simple mixtures of the three fHBP variants (e.g., RD012-7, RD012-8, and RD012-9) of the present disclosure. Also, the fusion proteins comprising characteristic amino acid sequences of the three fHBP variants (e.g., RD012-1) of the present disclosure induce superior anti-fHBP immunological effects to those of simple mixtures of molecules RD012-13 and RD012-14 (as control molecules in the present disclosure) known in the art.

In a second aspect, the nanoparticle vaccine antigens constructed by binding the antigenic component to the NPM particle also feature better immunogenicity than simple recombinant protein antigens.

In a third aspect, it was found that the nanoparticle immunogenic complex (RD012-1 NPM) constructed by binding the fusion protein comprising characteristic amino acid sequences of three fHBP variants to the NPM particle exhibited the immunogenic effect elicited by a mixture of nanoparticle immunogenic complexes formed by separately combining the three fHBP variants (RD012-7, RD012-8, and RD012-9) with the NPM. Therefore, the preparation procedures and the production cost are reduced, and the production efficiency is improved.

(3) Compared with the two approved MenB vaccines, i.e., Bexsero and Trumenba, the nanoparticle vaccine product provided by the present disclosure can induce superior cellular immunity and antibody immune response and achieve similar effects at a significantly reduced dose.

(4) In the present disclosure, the particle protein is prepared by *E. coli* fermentation and chromatographic purification, and the fHBP antigen can be prepared in BL21(DE3) by cell reactor culture and chromatographic purification, which are both suitable for industrial mass production and possess the advantages of high expression level, stable process and yield, simple operation, and the like. The particle protein component produced in a single batch can be combined

with the fHBP antigen produced in multiple batches, thereby improving the production efficiency. Compared with common recombinant protein vaccines, the nanoparticle vaccine disclosed herein provides higher immunoprotection at the same or lower dose, and thus can save the cost of mass production.

(5) The method for preparing the recombinant particle protein product provided herein features ease to operate and reduced amount of organic solvents used in subsequent chromatographic purification, and thus reduced cost in industrial mass production and suitability for industrial production. The product prepared from the recombinant particle protein provided herein, the side effects caused by residues such as impurities, host proteins, organic solvents, exogenous DNAs, antibiotics, bacterial endotoxins, and other substances in the particles are effectively reduced, thereby improving the safety.

(6) The present disclosure also explored the effect of linker peptides (linkers) of different structures on the expression of fusion proteins.

In a fourth aspect, the fHBP fusion protein of the present disclosure can elicit a potent bactericidal response against strains of *N. meningitidis* group B, and the bactericidal activity of nanoparticle vaccines obtained after linkage to the NPM is further enhanced; that is, the vaccine of the present disclosure can induce the production of antibodies at high levels with a broad bactericidal spectrum.

## BRIEF DESCRIPTION OF THE DRAWINGS

To illustrate the technical solutions in specific embodiments of the present disclosure or in the prior art more clearly, the drawings used for illustrating the specific embodiments or examples are briefly described below.

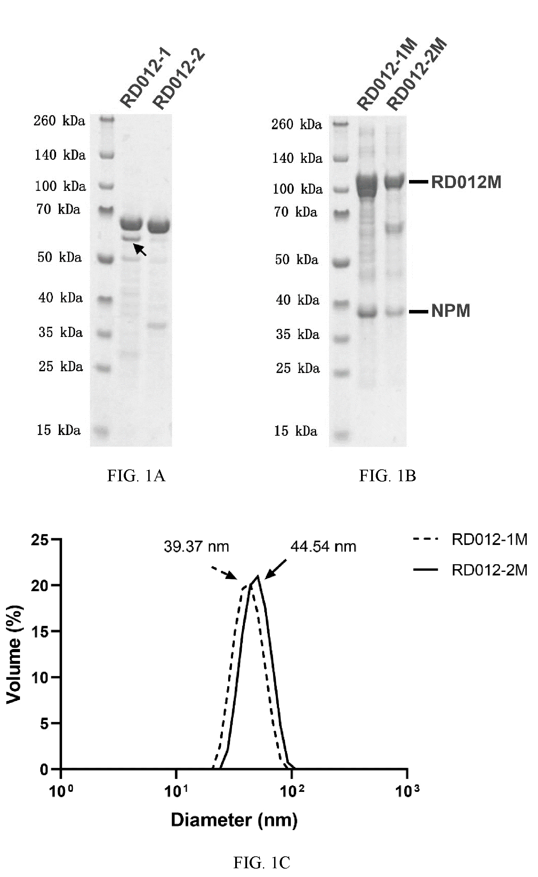

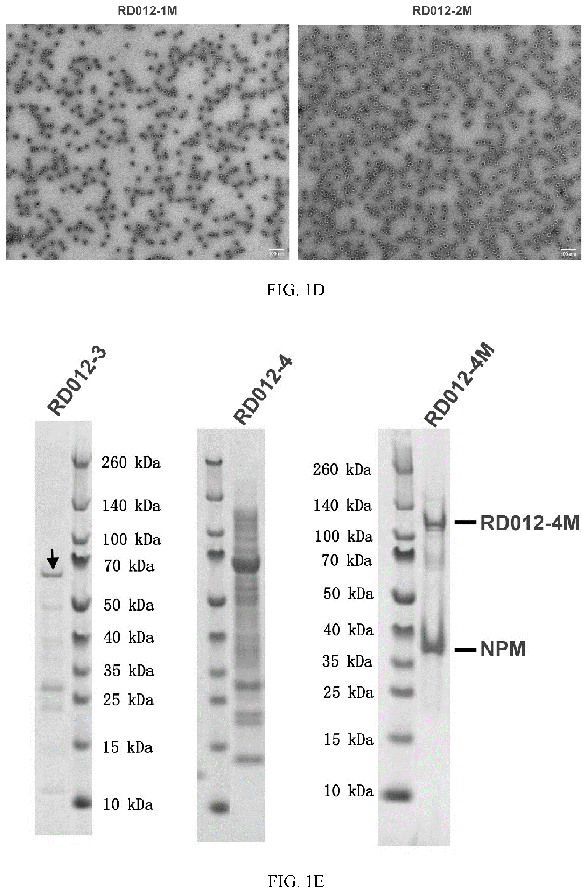

FIGs. 1A-1E: FIG. 1A shows the SDS-PAGE identification results of purified fusion proteins RD012-1 and RD012-2; FIG. 1B shows the SDS-PAGE identification results of purified RD012-1M and RD012-2M; FIG. 1C shows the particle size identification results of RD012-1M and RD012-2M; FIG. 1D shows the electron microscopic negative staining detection results of RD012-1M and RD012-2M; FIG. 1E shows the SDS-PAGE identification results of RD012-3, RD012-4, and RD012-4M;

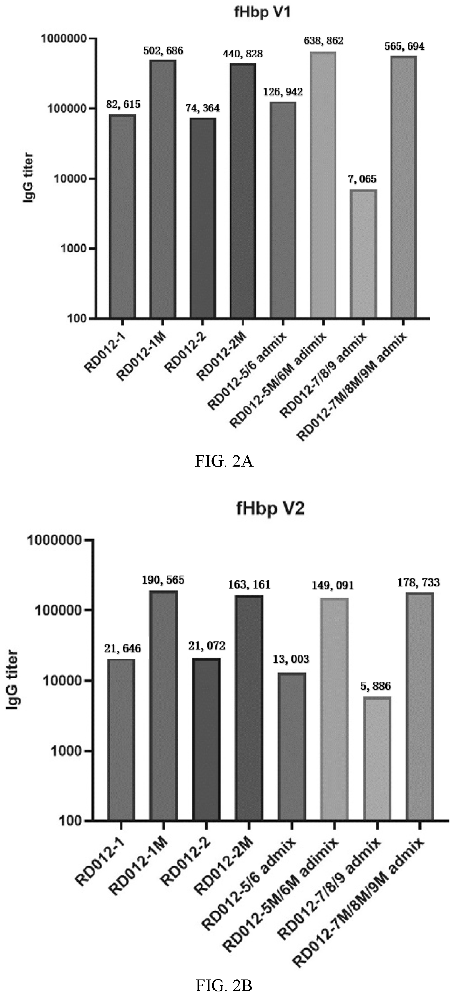

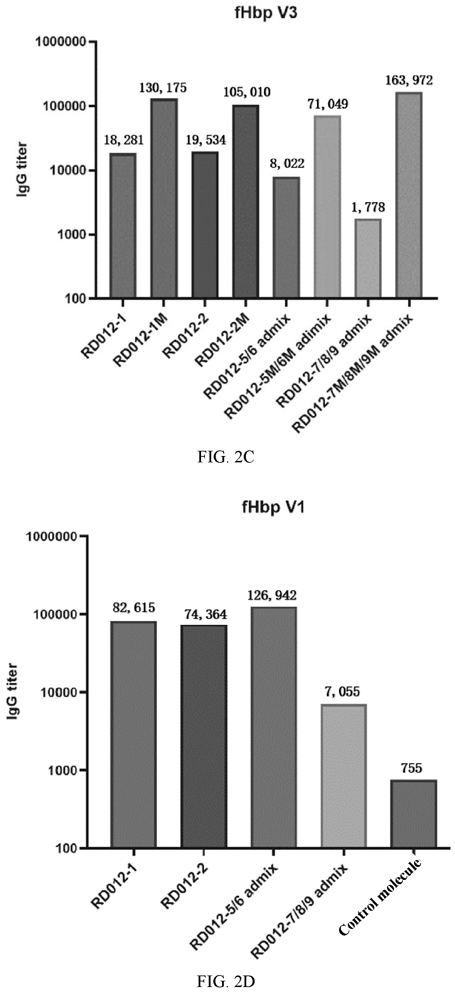

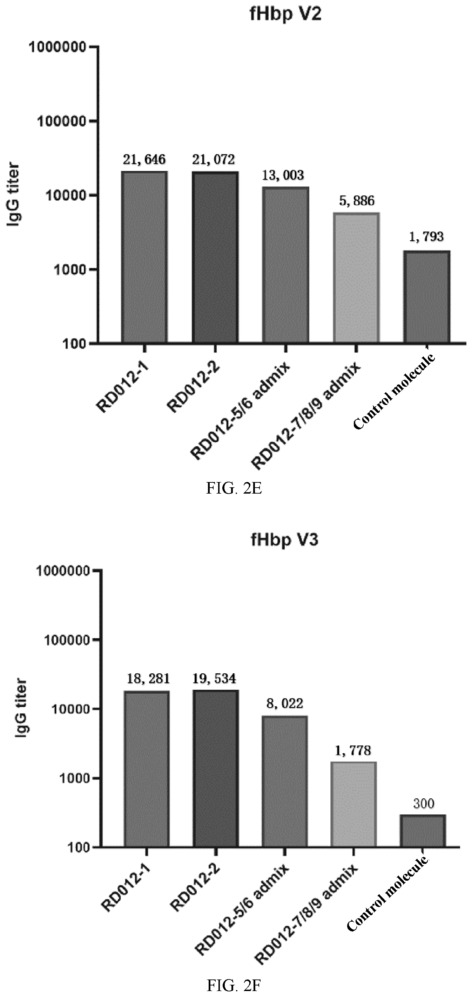

FIGs. 2A-2F: FIGs. 2A-2C show the induction of antibody titers against fHBP V1, V2, and V3 by vaccines RD012-1, RD012-1M, RD012-2, RD012-2M, RD012-5/6 admix, RD012-5M/M6 admix, RD012-7/8/9 admix, and RD012-7M/8M/9M admix; FIGs. 2D-2F show the induction of antibody titers against fHBP V1, V2, and V3 by vaccines RD012-1, RD012-2, RD012-5/6 admix, RD012-7/8/9 admix and control molecules;

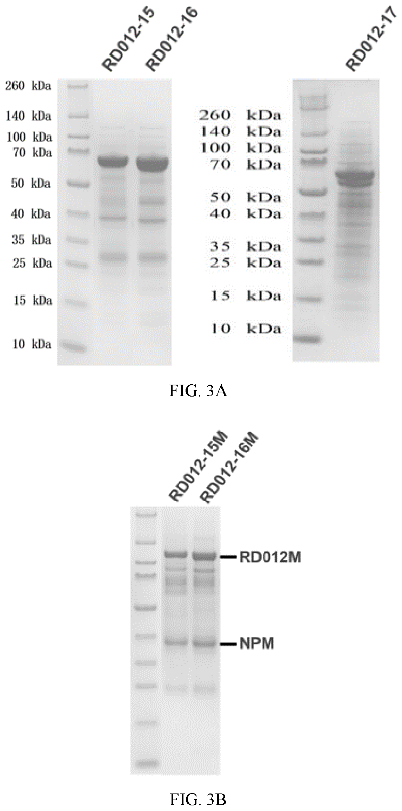

FIGs. 3A&3B: FIG. 3A shows the SDS-PAGE identification results of purified fusion proteins RD012-15, RD012-16, and RD012-17; FIG. 3B shows the SDS-PAGE identification results of purified RD012-15M and RD012-16M;

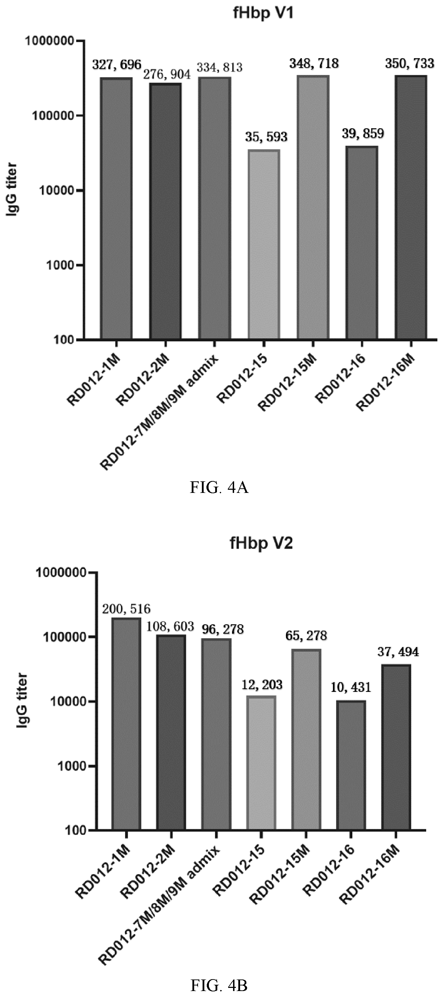

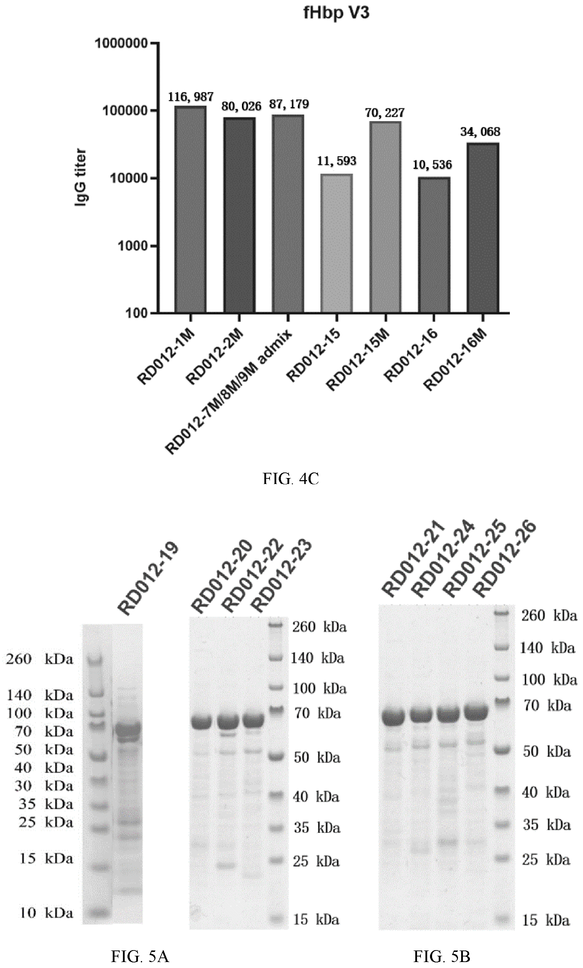

FIGs. 4A-4C: Schematics of the induction of antibody titers against fHBP V1, V2, and V3 by vaccines RD012-1M, RD012-2M, RD012-7M/8M/9M admix, RD012-15, RD012-15M, RD012-16, and RD012-16M;

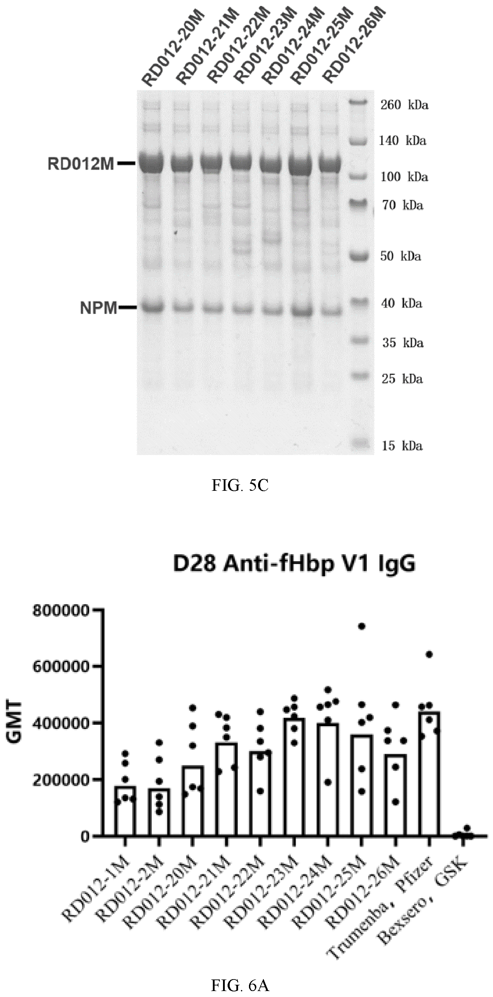

FIGs. 5A-5C: FIG. 5A shows the SDS-PAGE identification results of purified fusion proteins RD012-19, RD012-20, RD012-22, and RD012-23; FIG. 5B shows the SDS-PAGE identification results of purified fusion proteins RD012-21, RD012-24, RD012-25, and RD012-26; FIG. 5C shows the SDS-PAGE identification results of purified fusion proteins RD012-20M, RD012-21M, RD012-22M, RD012-23M, RD012-24M, RD012-25M, and RD012-26M;

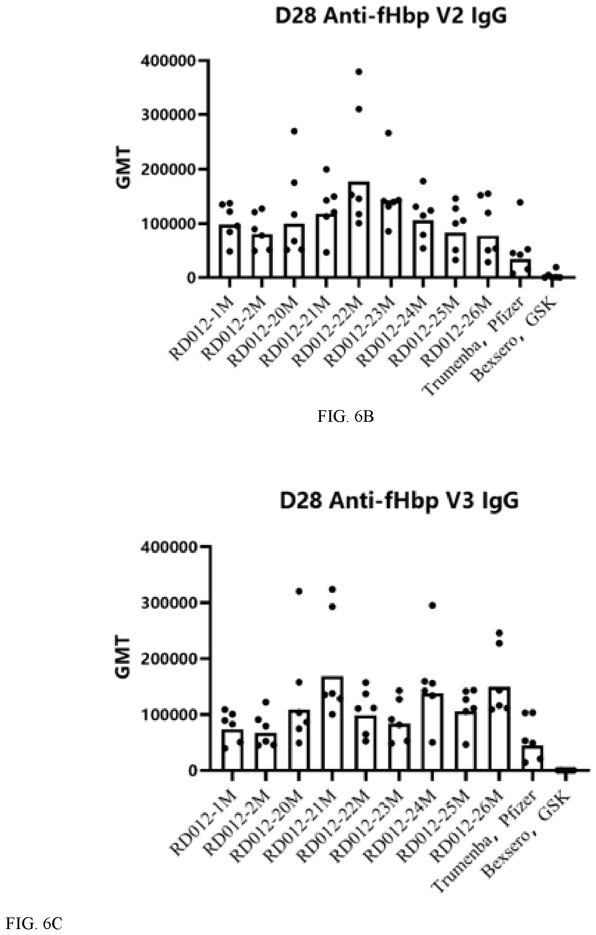

FIGs. 6A-6C: Schematics of the induction of antibody titers against fHBP V1, V2, and V3 by vaccines RD012-1M, RD012-2M, RD012-20M, RD012-21M, RD012-22M, RD012-23M, RD012-24M, RD012-25M, RD012-26M, Trumenba, and Bexsero.

## DETAILED DESCRIPTION

The principles and features disclosed herein are described with reference to the following examples, and the examples provided are only intended to explain the present disclosure and are not intended to limit the scope disclosed herein. Before the detailed description disclosed herein is further provided, it will be appreciated that the protection scope disclosed herein is not limited to the specific embodiments described below; it will also be appreciated that the terms used in the examples herein are intended to describe specific embodiments, rather than limit the protection scope disclosed herein. Procedures without specified conditions in the following examples are generally conducted according to conventional conditions or according to conditions recommended by manufacturers. When numerical ranges are given in the examples, it will be appreciated that, unless otherwise specified in the present disclosure, both endpoints of each of the numerical ranges and any numerical value between the two endpoints can be selected. Unless otherwise defined, all technical and scientific terms used herein have the same meaning as commonly understood by those skilled in the art. In addition to the specific methods, equipment, and materials used in the examples, any methods, equipment, and materials similar or equivalent to those described in the examples herein can also be used to implement the present disclosure, based on the understanding of the prior art by those skilled in the art and the disclosure disclosed herein. The experimental

materials used in the following examples were purchased from conventional reagent suppliers, unless otherwise specified.

**Example 1: Expression and purification of fHBP fusion protein molecules**

1. Materials:

Capsule filter (Bricap C01:180cm2) purchased from Cobetter, membrane cassette purchased from Millipore, HisTrap excel purchased from Cytiva, and SEC column (Superdex 200pg 10/300 GL) purchased from Cytiva.

2. Procedures:

2.1. The fHBP protein molecules shown in Table 1 were prepared according to a conventional technique of molecular cloning.
2.2. Expression induction conditions: RD012 BL21(DE3) monoclonal strain was seeded into 800 mL of LB (Amp+) culture medium and cultured at 37 °C and 220 rpm for 4-5 h. When the OD600 of the culture was about 0.6-0.8, the culture was cooled to 18 °C, IPTG was added at a final concentration of 0.5 mM, and the protein expression was induced at 200 rpm for 16 h.
2.3. Centrifugation for bacterial cell collection: The culture was centrifuged at 7000 g and room temperature to collect the bacterial cells, the medium was discarded, and the bacterial cells were resuspended in an 8 0 mL solution containing 150 mM NaCl, 20 mM Tris, pH 7.4.
2.4. Ultrasonic lysis: The resuspended bacterial cells were ultrasonicated in an ice-water bath for lysis. The sonication was conducted using a No. 2 ultrasonic horn at 50% power, with a cycle of 3 s on and 7 s off. The total sonication duration was 12 min.
2.5. Centrifugal collection of target protein: 13000 g, 4 °C, 30 min. The cell lysate supernatant was collected.
2.6. Histrap purification of target protein: The wash buffer was 20 mM Tris-HCl, 150 mM NaCl, pH 7.4; the elution buffer was 20 mM Tris-HCl, 150 mM NaCl, 500 mM imidazole, pH 7.4. The purification was performed on the Histrap excel-5ml NI column. The Histrap excel-5ml column was equilibrated with 10 CV of Wash buffer before sample loading. After the sample loading was completed, the column was washed with 10 CV of Wash buffer and washed with 10 CV of 2% elution buffer to remove impurity proteins. The target protein was linearly eluted with 15 CV of 2%-100% elution buffer. After the elution was completed, the protein purity was detected by SDS-PAGE.
2.7. SEC purification of target protein: The target protein purified by nickel column chromatography was collected, concentrated to 1 mL in a concentration tube, and then separated and purified on a SEC column (Superdex 200 pg 10/300 GL). The purification buffer was 20 mM Tris-HCl, 150 mM NaCl, pH 7.4. After elution, the protein purity was detected by SDS-PAGE. The SDS-PAGE identification results of purified RD012-1 and RD012-2 are shown in FIG. 1A. The protein concentration was determined by the BCA method, and the protein was stored at an appropriate temperature for subsequent binding reactions.

**Example 2: Expression and purification of binding peptide 2-NPM fusion protein**

The nanoparticle protein NPM (set forth in SEQ ID NO: 51) was linked, at its N-terminus, to the binding peptide 2 (set forth in SEQ ID NO: 48) via the linker peptide 2 (set forth in SEQ ID NO: 50) to form a binding peptide 2-NPM fusion protein, i.e., NPM-4C (set forth in SEQ ID NO: 52), as the particle protein component. The related sequences of NPM and NPM-4C are shown in Table 4.

Table 4: Sequences of NPM and NPM-4C fusion protein in examples of the present application

| Polypeptide name | Sequence |
|---|---|
| NPM | MKMEELFKKHKIVAVLRANSVEEAKKKALAVFLGGVHLIEITFTVPDADTVIKELSFLKEMGAIIGAGT VTSVEQARKAVESGAEFIVSPHLDEEISQFAKEKGVFYMPGVMTPTELVKAMKLGHTILKLFPGEVVG PQFVKAMKGPFPNVKFVPTGGVNLDNVCEWFKAGVLAVGVGSALVKGTPVEVAEKAKAFVEKIRGC TE (SEQ ID NO: 51) |
| NPM-4C | MDSATHIKFSKRDEDGKELAGATMELRDSSGKTISTWISDGQVKDFYLYPGKYTFVETAAPDGYEVAT AITFTVNEQGQVTVNGKATKGDAHIGGSGGSGGSGGSMKMEELFKKHKIVAVLRANSVEEAKKKALA VFLGGVHLIEITFTVPDADTVIKELSFLKEMGAIIGAGTVTSVEQARKAVESGAEFIVSPHLDEEISQFAK EKGVFYMPGVMTPTELVKAMKLGHTILKLFPGEVVGPQFVKAMKGPFPNVKFVPTGGVNLDNVCEW FKAGVLAVGVGSALVKGTPVEVAEKAKAFVEKIRGCTE (SEQ ID NO: 52) |

The coding gene of the fusion protein was expressed in *E. coli*. The cells were harvested and then lysed by high-pressure homogenization to release the target protein, and the feed liquid was clarified to remove bacterial debris and protein impurities. The clarification of the feed liquid was mainly achieved by heating treatment. The heating treatment was performed using a two-step heating method. The *E. coli* lysate supernatant was subjected to first-step heating and second-step heating (i.e., "two-step heating"). The impurity removal effect and the purity of the recombinant particle protein component in the two-step heating process were measured.
60 g of wet *E. coli* cells collected by centrifugation were taken, resuspended in 240 mL of buffer (20 mM Tris-HCl, 2 mM PMSF, pH = 9.0), and lysed using a high-pressure homogenizer at 1000 bar. After centrifugation, 280 mL of supernatant was collected. 40 mL of the resulting supernatant was then taken and subjected to the two-step heating operation. The lysate supernatant, the supernatant obtained after the first-step heating and centrifugation, and the resuspension of the pellet obtained after the second-step heating and centrifugation were subjected to SDS-PAGE analysis.
As shown in Table 5, in the first-step heating treatment, the pH was adjusted to 9.0, and the sample was heated in a water bath at 80 °C for 1 h. After returning to room temperature, centrifugation was performed to collect a supernatant (about 35 mL). In the second-step heating treatment, 35 mL of a buffer containing 100 mM Tris-HCl, 5 mM EDTA, pH 7.4, and 4% Triton was added, and then 7 mL of 1 M Tris-HCl, pH 7.4 was added. The mixture was thoroughly mixed, heated in a water bath at 60 °C for 10 min, and then immediately centrifuged to collect a pellet. The pellet was then redissolved in a buffer containing 20 mM Tris-HCl, 5 mM EDTA, pH 9.0.

Table 5: Two-step heating extraction process for recombinant particle protein component product

| Procedures | Reaction condition | Operational value |
|---|---|---|
| First pelleting and centrifugation | Heating temperature | 80 °C |
| | Heating duration | 60 min |
| | Centrifugation rotation speed | 12000 g |
| | Centrifugation temperature | 4 °C |
| | Centrifugation duration | 30 min |
| Supernatant dilution | Dilution buffer | 100 mM Tris-HCl, 5 mM EDTA, 4% Triton, pH 7.4 |
| | Volume of dilution buffer | 1:1 (v/v) |
| | pH buffer | 1 M Tris-HCl, pH 7.4 |
| | Volume of pH buffer | 10% of the total volume |
| Second pelleting and centrifugation | Heating temperature | 60 °C |
| | Heating duration | **10** min |
| | Centrifugation rotation speed | 6000 g |
| | Centrifugation temperature | 30 °C |
| | Centrifugation duration | **10** min |
| Pellet resuspending | Resuspension buffer | 20 mM Tris-HCl, 5 mM EDTA, pH 9.0 |
| | Volume of resuspension buffer | Resuspending to pre-centrifugation volume |
| | pH | 9.0+0.1 |
| | Filtration | 0.22 μm |

After the two-step heating process, adding urea and sodium chloride at different concentrations before chromatographic purification could significantly reduce the presence of unidentified substances near the target recombinant particle protein bands. The preferred pretreatment condition for the recombinant particle protein before Fractogel DEAE M chromatography was soaking in 8 M urea and 50-200 mM sodium chloride.
The above recombinant particle protein component sample solution was refined by ion exchange chromatography and hydrophobic chromatography. The first-step chromatographic purification was performed using the Fractogel DEAE M chromatography process for chromatographic purification. The specific procedures and parameters are shown in Table 6. The collected Fractogel DEAE M elution fraction sample was first diluted in a buffer, and then 50% (w/v) sucrose stabilizer was added to prevent pelleting of the recombinant particle protein during the next chromatography step. The specific parameters are shown in Table 7. Then, the sample was further refined using a hydrophobic chromatography

process with Octyl Bestarose 4FF (second-step chromatographic purification). The specific procedures and parameters are shown in Table 8.

Method for first-step chromatography: chromatography packing-Fractogel DEAE M, retention time-12.5 min.

Table 6. Procedures for first-step chromatography

| Chromatography procedures | Chromatography buffer/condition | Parameter |
|---|---|---|
| Equilibration buffer | 20 mM Tris-HCl, 5 mM EDTA, 8 M Urea, 50 mM NaCl, pH9.0 | 6 CV |
| pH after equilibration | 8.8±0.05 | 8.80 |
| Rinse buffer 1 | 20 mM Tris-HCl, 5 mM EDTA, 8 M Urea, 50 mM NaCl, pH9.0 | 1.5 CV |
| Rinse buffer 2 | 20 mM Tris-HCl, 5 mM EDTA, 8 M Urea, 2% Triton, pH9.0 | 5 CV |
| Rinse buffer 3 | 20 mM Tris-HCl, 8 M Urea, pH9.0 | 5 CV |
| Rinse buffer 4 | 20 mM Tris-HCl, 4 M Urea, pH9.0 | 5 CV |
| Elution buffer | 20 mM Tris-HCl, 4 M Urea, 150 mM, pH9.0 | 2 CV |
| Collection range | 50mAU-50mAU | Optical path length of 2 mm |

Table 7. Procedures for sample dilution before second-step chromatography

| Procedures | Buffer for dilution | Dilution volume |
|---|---|---|
| Collection of eluate from first-step chromatography | N/A | N/A |
| Buffer dilution | 20 mM Tris-HCl, 1 M NaCl, 50% (w/v) sucrose, pH 9.0 | 2× the volume of eluate |
| Buffer dilution | 20 mM Tris-HCl, 2 M NaCl, pH 9.0 | 1× the volume of eluate |

Method for second-step chromatography: chromatography packing-Octyl Bestarose 4FF, retention time-12.5 min

Table 8. Procedures for second-step chromatography

| Chromatography procedures | Chromatography buffer/condition | Parameter |
|---|---|---|
| Equilibration buffer | 20 mM Tris-HCl, 1 M NaCl, 25% (w/v) sucrose, pH 9.0 | 2 CV |
| Rinsing buffer | 20 mM Tris-HCl, 1 M NaCl, 25% (w/v) sucrose, pH 9.0 | 1.5 CV |
| Elution buffer | 20 mM Tris-HCl, 25% (w/v) sucrose, pH 9.0 | 3 CV |
| Collection range | 50 mAU-50 mAU | Optical path length of 2 mm |

Results and analysis:
Through purity testing, it was found that after further refining using the above chromatography medium combination, the purity of the resulting product reached 99.0% or higher.

**Example 3: Binding of fHBP to NPM and particle characterization**

I Binding of fHBP to NPM and product purification:

1. Procedures:

RD012-1 and RD012-2 as MenB antigens were mixed with NPM-4C as the carrier in a BCA protein concentration ratio of 6:1, a 50% sucrose stock solution was added at a final sucrose concentration of about 25%, and a 1 M Tris-HCl

stock solution pH 7.4 was added at 10% of the total reaction volume to stabilize the pH. The binding reaction was carried out at 22 °C for 24 h. The bound product was separated and purified on a SEC column (the purification buffer was 20 mM Tris-HCl, 150 mM NaCl, 25% Sucrose, pH 7.4), and the RD012-NPM component was collected. The purified RD012-NPM was identified by SDS-PAGE. The particle size of RD012-NPM was measured, and the negative staining results were obtained under an electron microscope.

The specific procedures are as follows:

(1) TEM examination

Negative staining samples were prepared using the floating method. A 400-mesh grid with a support film was selected and pre-treated to render it hydrophilic. Deionized water and a 2% uranyl formate negative staining solution were prepared. 3 μL of the prepared protein sample (0.12 mg/mL) was directly dropwise added to one side of the grid where the support film was located. After 1 minute, excess liquid was removed by touching the edge of the grid with clean filter paper. The grid was then briefly air-dried before being sequentially and rapidly rinsed twice on droplets of deionized water. This was followed by a single rinse with 5 μL of negative staining solution. Finally, 5 μL of negative staining solution was dripped to the grid and the grid was allowed to stand for 1 min. Then, the grid was held with tweezers, and the staining solution was removed using filter paper, leaving a thin layer to air-dry naturally before examination. The grid was examined under a 120 kV transmission electron microscope (FERRITINI Tecnai Spirit). The overall staining of the grid was assessed at low magnification. Holes with suitable thickness were selected for observation, and appropriate areas were chosen for photographing and data collection at high magnification.

(2) SDS-PAGE procedures

The SDS-PAGE test samples were prepared by adding a reductant DTT to the LDS sample loading buffer (4×). The sample loading buffer was heated at 70 °C for 5 min, cooled to room temperature, centrifuged at 10,000 rpm for 20 s, and thoroughly mixed by vortex, and the final loading amount was 5 μg. Both the test sample and the non-prestained protein molecular weight standard were loaded onto a 4-12% Bis-Tris gel, and the MES running buffer (lane 1) was applied. Electrophoresis was performed at a voltage of 150 V for approximately 60 min. After electrophoresis, the gel was removed and placed in a clean container. An appropriate amount of Coomassie brilliant blue staining solution was added to submerge the gel, followed by staining for 2 h on a shaker. After staining, the staining solution was discarded, and the gel was soaked in purified water for destaining on a shaker until the background was clear. The gel was then photographed using a GelDoc Go gel imager.

(3) DLS procedures

The purified and prepared test samples were diluted to 0.25 mg/mL. A Zetasizer Lab instrument was used, ≥ 1 mL of the test sample was injected into a sample cell, and the instrument was run for measurement. Data analysis was performed based on the Z-average (nm), polydispersity index (PI) value, and the distribution profiles of the Size Distribution by Intensity/Volume. The results were then documented.

2. Results:

The purified NPM VLPs of RD012-1 and RD012-2 were identified by SDS-PAGE as shown in FIG. 1B. The particle size and electron microscopic negative staining results of the NPM VLPs of RD012-1 and RD012-2 are shown in FIGs. 1C and 1D. The results suggest that in the RD012-1 fusion protein, the removal of the 4T sequence and the change of the His tag from the C-terminus to the N-terminus, i.e., RD012-3, result in a significantly reduced expression level, as shown in FIG. 1E. In addition, when the 4T sequence was placed between the two chimeric proteins, i.e., RD012-4, the binding rate to the VLP carrier NPM-4C was significantly reduced. The results are shown in FIG. 1E. Therefore, RD012-1 and RD012-2 molecules were determined as optimal fusion protein molecules.

**Example 4: Animal immunization study**

(1) Materials

Mice: female BALB/c mice, aged 5-6 weeks (purchased from Guangdong Vital River Laboratory Animal Technology Co., Ltd.)

Adjuvant: Aluminum hydroxide adjuvant

The other reagents and consumables are all commercially available, conventional reagents and consumables.

(1) Procedures

As shown in Table 9, the mice were immunized with vaccines of the same construct design by intramuscular injection on days 0 and 14 at a dose of 9 μg/mouse, with each vaccine dose containing 75 μg of aluminum hydroxide adjuvant. The serum was isolated from the blood collected on day 28 for the detection of bound antibodies.

Table 9. Animal immunization regimen 1

| Group (n=6) | Antigen | Adjuvant | Dose | Day 0 | Day 14 | Day 28 |
|---|---|---|---|---|---|---|
| 1 | RD012-1 | Alhydrogel | 9 μg | Primary immunization | Secondary immunization | Blood sampling at end-point |
| 2 | RD012-1M | | | | | |
| 3 | RD012-2 | | | | | |
| 4 | RD012-2M | | | | | |
| 5 | RD012-5/6 admix | | | | | |
| 6 | RD012-5M/6M admix | | | | | |
| 7 | RD012-7/8/9 admix | | | | | |
| 8 | RD012-7M/8M/9M admix | | | | | |
| 9 | control molecule | | | | | |

(3) Binding antibody assay:

ELISA plates were coated with three variant fHBP proteins, and the coated ELISA plates were blocked in the Blocker Casein in PBS blocking solution (purchased from ThermoFisher) for 1-4 h. Serum samples collected from mice in the treatment groups at the end of immunization in Table 9 were subjected to serial three-fold dilution from 1:300 to 656100. The dilutions were added to the wells, and the negative control was the sample diluent. The plate was incubated for 2-3 h, incubated with an HRP-conjugated goat anti-mouse secondary antibody for 1 h, and then subjected to a chromogenic reaction with a TMB substrate. After the chromogenesis, the reaction was stopped with 1 M hydrochloric acid, and the absorbance value (OD) was measured on a microplate reader with the primary wavelength being 450 nm and the reference wavelength being 620 nm. The OD value of the sample = OD450 - OD620. The measurement was completed within 5 min after the termination.

The results are shown in FIGs. 2A-F.

The results of the mouse immunization study showed that RD012-1/2 fusion protein induced about 10.5-fold, 3.6-fold, and 10.3-fold higher antibody titers against fHBP variants V1, V2, and V3 than equal-ratio mixtures of the three fHBP variants (RD012-7/8/9 admix). Therefore, the fusion protein comprising characteristic amino acid sequences of the three fHBP variants induced anti-fHBP antibody titers better than a simple mixture of the three fHBP variants, as shown in FIGs. 2A-F. In addition, the nanoparticle vaccine RD012-1M/2M antigens constructed by binding RD012-1/2 to NPM particles also demonstrated superior immunogenicity to the simple recombinant protein antigen (i.e., RD012-1/2), as shown in FIGs. 2A-2C.

**Example 5: Optimization of RD012 fusion protein molecule design**

I. Procedures:

According to the results of Example 4 above, it was found that the fusion protein antigen comprising the characteristic amino acid sequences of three fHBP variants constructed in the present disclosure has a broad spectrum and good efficacy. However, it was found in the results in FIG. 1A that RD012-1 protein had fragment bands, which may complicate the product production process. According to previous study data, the tandem orders of the two chimeric proteins RD012-1/2 were interchanged to obtain two molecules RD012-15/16, and the N-terminal amino acid portion of RD012-1 was truncated to obtain molecule RD012-17. The recombinant protein antigens and VLP particle antigens of

RD012-15/16/17 were prepared according to the method in Examples 1 and 2. The results are shown in FIG. 3. No cleavage bands were observed in the RD012-15/16 recombinant protein antigens and the VLP particle antigens, and cleavage bands were still observed in the RD012-17 fusion protein. The immunogenicities of RD012-15/16 and RD012-1/2 were compared according to the method in Example 4. The animal immunization regimen is shown in Table 10. The binding antibody titer determined by ELISA is shown in FIGs. 4A-C.

Table 10. Animal immunization regimen 2

| Group (n=6) | Antigen | Adjuvant | Dose | Day 0 | Day 14 | Day 28 |
|---|---|---|---|---|---|---|
| 1 | RD012-1M | Alhydrogel | 9μg | Primary immunization | Secondary immunization | Blood sampling at end-point |
| 2 | RD012-2M | | | | | |
| 3 | RD012-7M/8M/9M admix | | | | | |
| 4 | RD012-15 | | | | | |
| 5 | RD012-15M | | | | | |
| 6 | RD012-16 | | | | | |
| 7 | RD012-16M | | | | | |

II Results:

It can be seen from the experimental results that although the interchanged tandem orders of the two chimeric proteins in the fusion protein solved the molecular fragmentation problem of RD012-1, the induced titers of antibodies against fHBP variants V2 and V3 were also reduced.

**Example 6: Optimization of RD012 fusion protein molecule design**

I Procedures:

To solve the problem of fragment bands in the RD012-1 fusion protein, the complete molecular weights of the target molecule and the fragment molecules were detected by LC-MS, and the fragment amino acid sequences were determined by MS by comparing the two peptide maps. It was found in the previous study data that the fragment sequences did not affect the immunogenicity. Therefore, the fragment amino acids were directly truncated to obtain molecule RD012-18. The recombinant protein antigen RD012-18 was prepared and found to have a single target band, but the protein expression level was reduced by more than half. Subsequently, the tandem flexible linker between the two chimeric proteins of the fusion protein molecule was replaced with a semi-rigid linker: GGSGGEAAAK and a rigid linker of different lengths: (EAAAK)n to obtain molecule RD012-19/20/22/23. The results are shown in FIG. 5A. After the flexible linker was replaced with the rigid linker, almost no fragment bands were generated. In addition, the prevalent strains of meningococcus group B in China mainly express fHBP V2. To further optimize the antigen-induced antibody titer against the fHBP variant V2, the fHBP V3 domain 1 and fHBP V2 domain 2 in the fusion protein molecule RD012-1 chimeric protein were replaced by the fHBP V2 domain 1 and fHBP V3 domain 2 in the same tandem order to obtain fusion protein antigen molecules RD012-21/24/25/26. The prepared recombinant protein antigens are shown in FIG. 5B. The VLP particle antigens of the fusion protein molecules described above were prepared according to the method in Example 1, and the results are shown in FIG. 5C. The immunogenicities of the optimized molecules described above and RD012-1/2 were compared according to the method in Example 4. The animal immunization regimen is shown in Table 11.

Preparation of Trumenba vaccine: 60 μg of lipidated fHBP V1 protein and 60 μg of lipidated fHBP V3 protein were adhered onto an aluminum phosphate adjuvant.

Preparation of Bexsero vaccine: 50 μg of fHBP V1 fusion protein, 50 μg of NHBA fusion protein, 50 μg of NadA protein, and 25 μg of outer membrane vesicles (OMVs) were adhered onto an aluminum hydroxide adjuvant.

Table 11. Animal immunization regimen 3

| Group (n=6) | Antigen | Adjuvant | Dose | Day 0 | Day 14 | Day 28 |
|---|---|---|---|---|---|---|
| 1 | RD012-1M | | | | | |
| 2 | RD012-2M | | | | | |

(continued)

| Group (n=6) | Antigen | Adjuvant | Dose | Day 0 | Day 14 | Day 28 |
|---|---|---|---|---|---|---|
| 3 | RD012-20M | Alhydrogel | 5μg | Primary immunization | Secondary immunization | Blood sampling at end-point |
| 4 | RD012-21M | | | | | |
| 5 | RD012-22M | | | | | |
| 6 | RD012-23M | | | | | |
| 7 | RD012-24M | | | | | |
| 8 | RD012-25M | | | | | |
| 9 | RD012-26M | | | | | |
| 10 | Trumenba, Pfizer | Aluminium phosphate | 12 μg | | | |
| 11 | Bexsero, GSK | Aluminium hydroxide | 17.5 μg | | | |

II. Results:

The results, as shown in FIGs. 6A-6C, indicated that RD012-20M/22M/23M induced increased antibody titers against the three variants fHBPV1, V2, and V3 compared with RD012-1M/2M, suggesting that the immunogenicity of the fusion proteins can be improved by replacing the tandem flexible linker between the two chimeric proteins with a rigid linker. In addition, RD012-21M/24M/25M/26M fusion protein antigen molecules obtained by exchanging fHBP V3 domain 1 and fHBP V2 domain 2 in the fusion protein molecule RD012-1 chimeric protein with fHBP V2 domain 1 and fHBP V3 domain 2 in the same tandem order induced significantly increased antibody titer against fHBP as compared to RD012-1M/2M. In general, the immune effects of the vaccines RD012-1M/2M/20M/21M/22M/23M/24M/25M/26M of the present disclosure were superior to those of the commercially available control vaccines.

**Example 7: Serum bactericidal assay (SBA)**

The MenB strain was applied to a chocolate agar plate and cultured overnight at 37 °C with 5% $CO_2$. A single colony was seeded onto a Mueller-Hinton medium with the initial $OD_{600}$ of the bacterial solution being controlled at 0.05-0.08. The bacterial solution was cultured on a shaker at 37 °C until the $OD_{600}$ reached 0.23-0.24, and the bacterial viability was determined. The test serum of the immunization groups RD012-24M, Trumenba, and Bexsero in Example 6 was inactivated by heating at 56 °C for 1 h. The total volume in each well was 50 μL, including 25 μL of serial two-fold dilutions of the test serum, 12.5 μL of the bacterial working solution, and 12.5 μL of rabbit complements. The controls included: serum incubated with complement serum, immune serum incubated with bacteria, and inactivated complement. After shaking and mixing thoroughly, the cells were cultured on a shaker at 37 °C for 2-4 h. 10 μL of each sample was taken and added onto a Mueller-Hinton agar plate, and the plate was incubated at 37 °C overnight. The agar plates cultured overnight were subjected to chromogenesis with TTC, bacterial colonies were counted, and bactericidal titers were calculated. The results are shown in Table 12. The vaccine RD012-24M of the present disclosure exhibited superior bactericidal activity against V2 subtype strains to those of the commercially available vaccines, and comparable bactericidal activities against V1 and V3 subtype strains to those of commercially available vaccines. It can be seen that RD012-24M has a broader spectrum of immune effect and better bactericidal activity than its commercially available counterparts.

Table 12. Bactericidal activity of serum from immunized mice against different fHBP variant strains

| Vaccines / Target bacterium | RD012-24M | Trumenba | Bexsero | fHbp subtype of strain |
|---|---|---|---|---|
| **1** | 1:64 | 1:32 | 1:32 | 2.101 |
| **2** | 1:128 | <16 | <16 | 2.101 |
| **3** | 1:256 | 1:64 | 1:64 | 2.16 |
| **4** | 1:512 | 1:512 | 1:512 | 1.13 |
| **5** | 1:32 | 1:32 | 1:32 | 3.1239 |

According to the comprehensive analysis of the above results, the RD012 series of proteins, immunogenic complexes, immune compositions, and vaccines have good immunogenicity and bactericidal activity, and have a broad spectrum of immune effects.

In summary, the above examples and drawings are only for the purpose of illustrating preferred examples disclosed herein, and are not intended to limit the protection scope of the present disclosure. Any modifications, equivalent substitutions, improvements, and the like made without departing from the spirit and principle of the present disclosure shall all fall within the protection scope of the present disclosure.

## Claims

**1.** An fHBP protein, wherein the fHBP protein is as shown in any one of the following (1) or (2):

(1) an fHBP chimeric protein, comprising different domains of different fHBP variants, wherein the domain is selected from domain 1 or domain 2 of fHBP variant 1 (fHBP V1), domain 1 or domain 2 of fHBP variant 2 (fHBP V2), or domain 1 or domain 2 of fHBP variant 3 (fHBP V3); and
(2) an fHBP fusion protein, comprising two different fHBP chimeric proteins, wherein the two fHBP chimeric proteins may be linked in tandem via a linker, and the fHBP fusion protein may simultaneously induce antibodies against fHBP V1, V2, and V3;

the fHBP V1 is selected from v1.1, v1.4, v1.13, v1.15, v1.14, v1.10, v1.260, v1.510, v1.90, v1.275, v1.697, v1.226, v1.110, v1.249, v1.108, v1.227, v1.215, and v1-2,3.x; the fHBP V2 is selected from v2.16, v2.19, v2.21, v2.22, v2.24, and v1-2,3.x; the fHBP V3 is selected from v3.116, v3.28, v3.31, v3.45, v3.42, and v1-2,3.x.

**2.** The fHBP protein according to claim 1, wherein the fHBP chimeric protein is shown in any one of (A)-(D) below:

(A) comprising fHBP V3 domain 1 and fHBP V1 domain 2;
(B) comprising fHBP V1 domain 1 and fHBP V2 domain 2;
(C) comprising fHBP V2 domain 1 and fHBP V1 domain 2; and/or
(D) comprising fHBP V1 domain 1 and fHBP V3 domain 2;

preferably, the fHBP fusion protein comprises the fHBP chimeric proteins shown in (A) and (B), and/or comprises the fHBP chimeric proteins shown in (C) and (D).

**3.** The fHBP protein according to claim 1 or 2, wherein

the amino acid sequence of fHBP V1 domain 1 has 40% or higher, 50% or higher, 60% or higher, 70% or higher,

80% or higher, or 90% or higher identity to SEQ ID NO: 54, 55, or 56;
the amino acid sequence of fHBP V1 domain 2 has 40% or higher, 50% or higher, 60% or higher, 70% or higher, 80% or higher, or 90% or higher identity to SEQ ID NO: 57;
the amino acid sequence of fHBP V2 domain 1 has 40% or higher, 50% or higher, 60% or higher, 70% or higher, 80% or higher, or 90% or higher identity to SEQ ID NO: 58;
the amino acid sequence of fHBP V2 domain 2 has 40% or higher, 50% or higher, 60% or higher, 70% or higher, 80% or higher, or 90% or higher identity to SEQ ID NO: 59;
the amino acid sequence of fHBP V3 domain 1 has 40% or higher, 50% or higher, 60% or higher, 70% or higher, 80% or higher, or 90% or higher identity to SEQ ID NO: 60, 61, 62, or 63;
and/or the amino acid sequence of fHBP V3 domain 2 has 40% or higher, 50% or higher, 60% or higher, 70% or higher, 80% or higher, or 90% or higher identity to SEQ ID NO: 64.

**4.** The fHBP protein according to any one of claims 1-3, wherein the amino acid sequence of the fHBP V1 domain 1 is selected from SEQ ID NO: 54, 55, or 56, the amino acid sequence of the fHBP V1 domain 2 is selected from SEQ ID NO: 57, the amino acid sequence of the fHBP V2 domain 1 is selected from SEQ ID NO: 58, the amino acid sequence of the fHBP V2 domain 2 is selected from SEQ ID NO: 59, the amino acid sequence of the fHBP V3 domain 1 is selected from SEQ ID NO: 60, 61, 62, or 63, the amino acid sequence of the fHBP V3 domain 2 is selected from SEQ ID NO: 64; optionally, the linker is selected from the amino acid sequences of $G_n$, GSGGGG, $(EAAAK)_n$, or GGSGGEAAAK, wherein n may be an integer greater than 0 and less than or equal to 10, and preferably, n is 1, 2, 3, or 4.

**5.** An immunogenic complex, comprising:

(1) an fHBP antigenic component, comprising the fHBP protein according to any one of claims 1-4 or comprising an fHBP variant, wherein the fHBP variant is selected from fHBP V1, fHBP V2, or fHBP V3; and
(2) a particle protein component, comprising a nanoparticle protein, wherein

preferably, the antigenic component further comprises a binding peptide 1, and the particle protein component further comprises a binding peptide 2; the fHBP protein and the binding peptide 1 form a fusion protein, and the nanoparticle protein and the binding peptide 2 form a fusion protein; the antigenic component and the particle protein component are covalently bound to each other via the binding peptide 1 and the binding peptide 2 to form the immunogenic complex;
preferably, the binding peptide 1 comprises the amino acid sequence as set forth in SEQ ID NO: 53, and the binding peptide 2 comprises the amino acid sequence as set forth in SEQ ID NO: 54.

**6.** The immunogenic complex according to claim 5, wherein the antigenic component further comprises a linker peptide 1, and the particle protein component further comprises a linker peptide 2; the antigenic component is formed by fusing the fHbp protein, at the C-terminus, with the binding peptide 1 via the linker peptide 1; the particle protein component is formed by fusing the nanoparticle protein, at the N-terminus, with the binding peptide 2 via the linker peptide 2;

optionally, the linker peptide 1 is selected from the amino acid sequences of $(GGGGS)_n$, $(EAAAK)_n$, $(GSGGSG)_n$, $(GGS)_n$, or $(GSG)_n$, wherein n may be an integer greater than 0 and less than or equal to 5; the linker peptide 2 is selected from the amino acid sequences of $(GGS)_n$, $(GGGGS)_n$, $(EAAAK)_n$, or $(GSGGSG)_n$, wherein n may be an integer greater than 0 and less than or equal to 10;
optionally, both the antigenic component and the particle protein component comprise a histidine tag.

**7.** The immunogenic complex according to claim 5 or 6, wherein the amino acid sequence of the fHBP antigenic component is selected from any one of SEQ ID NOs: 1-9 and 12-23; preferably, the amino acid sequence of the fHBP antigenic component is selected from any one of SEQ ID NOs: 1, 2, 5-9, and 20-23.

**8.** The immunogenic complex according to any one of claims 5-7, wherein the nanoparticle protein is NPM, AP205, or ferritin protein; preferably, the amino acid sequence of NPM is set forth in SEQ ID NO: 52.

**9.** A method for preparing an immunogenic complex for preventing or treating a disease related to *Neisseria meningitidis* group B, comprising:

(1) separately ligating coding genes of the fHbp antigenic component and the particle protein component as defined in any one of claims 5-8 into expression vectors to construct recombinant expression plasmids;
(2) constructing a recombinant strain capable of expressing the fHbp antigenic component and the particle protein

component in the host cell;
(3) expressing fusion proteins using the recombinant strain and purifying the recombinant fusion proteins; and
(4) subjecting the antigenic component and the particle protein component purified in step (3) to a covalent binding reaction to obtain the immunogenic complex, wherein

preferably, in step (1), the plasmid expressing the antigenic component is selected from pcDNA3.4, and the vector expressing the particle protein may be selected from pET-28a(+) or pET-30a(+);
optionally, in step (2), the host cell expressing the antigenic component is CHO, and the host cell expressing the particle protein is *E. coli*.

**10.** An immune composition, as shown in any one of the following:

(1) comprising one or more of the fHBP proteins according to any one of claims 1-4; or
(2) comprising one or more of the immunogenic complexes according to any one of claims 5-8, wherein optionally, the immune composition further comprises a pharmaceutically acceptable carrier.

**11.** The immune composition according to claim 10, comprising any two of the fHBP chimeric proteins (A)-(D) as defined in claim 2, wherein
alternatively, the immune composition comprises three fHBP variants: fHBP V1, fHBP V2, and fHBP V3; optionally, the amino acid sequence of fHBP V1 is amino acids 1-259 of SEQ ID NO: 7, the amino acid sequence of fHBP V2 is amino acids 1-253 of SEQ ID NO: 8, and the amino acid sequence of fHBP V3 is amino acids 1-260 of SEQ ID NO: 9.

**12.** The immune composition according to claim 10 or 11, wherein the pharmaceutically acceptable carrier comprises a stabilizer, an excipient, a surfactant, a buffering agent, and a pH regulator; the stabilizer is sucrose and/or arginine, the excipient is mannitol, the surfactant is Tween 80, the buffering agent is disodium hydrogen phosphate dihydrate and/or sodium dihydrogen phosphate dihydrate, and the pH regulator is hydrochloric acid.

**13.** A vaccine for preventing or treating a disease related to *N. meningitidis* group B, comprising the immune composition according to any one of claims 10-12 and an adjuvant.

**14.** The vaccine according to claim 13, wherein the adjuvant is selected from at least one of an aluminum salt adjuvant, Freund's complete adjuvant, a propolis adjuvant, an oil-in-water adjuvant, a cytokine, CpGDNA, flagellin, a genetically engineered toxoid, an immune-stimulating complex, a liposome, a saponin, and a Poly(I:C) adjuvant; preferably, the aluminum salt adjuvant is aluminum hydroxide, the oil-in-water adjuvant is a squalene-based adjuvant, and the saponin adjuvant comprises QS-21;
optionally, the adjuvant comprises 3%-5% squalene, 0.4%-1% Span 85, 0.4%-1% Tween 80, and 10 mM citrate buffer or 0.1%-0.5% sodium citrate and 0.01%-0.05% citric acid (w/v), wherein the squalene is preferably 3.5%-4.5% (w/v), and the squalene-based adjuvant may be MF59 or SWE; preferably, the adjuvant comprises the following components: 0.5% Span 85, 0.5% Tween 80, 4.2% squalene, 0.264% sodium citrate, and 0.016% citric acid.

**15.** Use of the fHBP protein according to any one of claims 1-4, the immunogenic complex according to any one of claims 5-8, the immune composition according to any one of claims 10-12, or the vaccine according to claim 13 or 14 in preparing a medicament for preventing or treating a related disease caused by *N. meningitidis* group B.

RD012-1
RD012-2
260 kDa
140 kDa
100 kDa
70 kDa
50 kDa
40 kDa
35 kDa
25 kDa
15 kDa
RD012-1M
RD012-2M
RD012M
NPM

FIG. 1A

FIG. 1B

39.37 nm
44.54 nm
RD012-1M
RD012-2M
Volume (%)
25
20
15
10
5
0
$10^0$
$10^1$
$10^2$
$10^3$
Diameter (nm)

FIG. 1C

RD012-1M
RD012-2M

FIG. 1D

RD012-3
RD012-4
RD012-4M
260 kDa
140 kDa
100 kDa
70 kDa
50 kDa
40 kDa
35 kDa
25 kDa
15 kDa
10 kDa
RD012-4M
NPM

FIG. 1E

fHbp V1
IgG titer
1000000
100000
10000
1000
100
82, 615
502, 686
74, 364
440, 828
126, 942
638, 862
7, 065
565, 694
RD012-1
RD012-1M
RD012-2
RD012-2M
RD012-5/6 admix
RD012-5M/6M adimix
RD012-7/8/9 admix
RD012-7M/8M/9M admix

FIG. 2A

fHbp V2
IgG titer
1000000
100000
10000
1000
100
21, 646
190, 565
21, 072
163, 161
13, 003
149, 091
5, 886
178, 733
RD012-1
RD012-1M
RD012-2
RD012-2M
RD012-5/6 admix
RD012-5M/6M adimix
RD012-7/8/9 admix
RD012-7M/8M/9M admix

FIG. 2B

fHbp V3
IgG titer
1000000
100000
10000
1000
100
18, 281
130, 175
19, 534
105, 010
8, 022
71, 049
1, 778
163, 972
RD012-1
RD012-1M
RD012-2
RD012-2M
RD012-5/6 admix
RD012-5M/6M adimix
RD012-7/8/9 admix
RD012-7M/8M/9M admix

FIG. 2C

fHbp V1
IgG titer
1000000
100000
10000
1000
100
82, 615
74, 364
126, 942
7, 055
755
RD012-1
RD012-2
RD012-5/6 admix
RD012-7/8/9 admix
Control molecule

FIG. 2D

fHbp V2
1000000
100000
10000
1000
100
IgG titer
21, 646
21, 072
13, 003
5, 886
1, 793
RD012-1
RD012-2
RD012-5/6 admix
RD012-7/8/9 admix
Control molecule

FIG. 2E

fHbp V3
1000000
100000
10000
1000
100
IgG titer
18, 281
19, 534
8, 022
1, 778
300
RD012-1
RD012-2
RD012-5/6 admix
RD012-7/8/9 admix
Control molecule

FIG. 2F

RD012-15
RD012-16
RD012-17
260 kDa
140 kDa
100 kDa
70 kDa
50 kDa
40 kDa
35 kDa
25 kDa
15 kDa
10 kDa
260 kDa
140 kDa
100 kDa
70 kDa
50 kDa
40 kDa
35 kDa
25 kDa
15 kDa
10 kDa

FIG. 3A

RD012-15M
RD012-16M
RD012M
NPM

FIG. 3B

fHbp V1
1000000
100000
10000
1000
100
IgG titer
327, 696
276, 904
334, 813
35, 593
348, 718
39, 859
350, 733
RD012-1M
RD012-2M
RD012-7M/8M/9M admix
RD012-15
RD012-15M
RD012-16
RD012-16M

FIG. 4A

fHbp V2
1000000
100000
10000
1000
100
IgG titer
200, 516
108, 603
96, 278
12, 203
65, 278
10, 431
37, 494
RD012-1M
RD012-2M
RD012-7M/8M/9M admix
RD012-15
RD012-15M
RD012-16
RD012-16M

FIG. 4B

fHbp V3
IgG titer
1000000
100000
10000
1000
100
116, 987
80, 026
87, 179
11, 593
70, 227
10, 536
34, 068
RD012-1M
RD012-2M
RD012-7M/8M/9M admix
RD012-15
RD012-15M
RD012-16
RD012-16M

FIG. 4C

RD012-19
RD012-20
RD012-22
RD012-23
RD012-21
RD012-24
RD012-25
RD012-26
260 kDa
140 kDa
100 kDa
70 kDa
50 kDa
40 kDa
30 kDa
35 kDa
25 kDa
15 kDa
10 kDa

FIG. 5A

FIG. 5B

RD012-20M
RD012-21M
RD012-22M
RD012-23M
RD012-24M
RD012-25M
RD012-26M
RD012M
NPM
260 kDa
140 kDa
100 kDa
70 kDa
50 kDa
40 kDa
35 kDa
25 kDa
15 kDa

FIG. 5C

D28 Anti-fHbp V1 IgG
GMT
800000
600000
400000
200000
0
RD012-1M
RD012-2M
RD012-20M
RD012-21M
RD012-22M
RD012-23M
RD012-24M
RD012-25M
RD012-26M
Trumenba, Pfizer
Bexsero, GSK

FIG. 6A

D28 Anti-fHbp V2 IgG
400000
300000
200000
100000
0
GMT
RD012-1M
RD012-2M
RD012-20M
RD012-21M
RD012-22M
RD012-23M
RD012-24M
RD012-25M
RD012-26M
Trumenba, Pfizer
Bexsero, GSK

FIG. 6B

D28 Anti-fHbp V3 IgG
400000
300000
200000
100000
0
GMT
RD012-1M
RD012-2M
RD012-20M
RD012-21M
RD012-22M
RD012-23M
RD012-24M
RD012-25M
RD012-26M
Trumenba, Pfizer
Bexsero, GSK

FIG. 6C

**INTERNATIONAL SEARCH REPORT**

International application No.
**PCT/CN2024/129236**

**A. CLASSIFICATION OF SUBJECT MATTER**

C07K 19/00(2006.01)i; A61K 39/095(2006.01)i; C12N 15/31(2006.01)i; C12N 1/21(2006.01)i

According to International Patent Classification (IPC) or to both national classification and IPC

**B. FIELDS SEARCHED**

Minimum documentation searched (classification system followed by classification symbols)

IPC:C07K A61K C12N

Documentation searched other than minimum documentation to the extent that such documents are included in the fields searched

Electronic data base consulted during the international search (name of data base and, where practicable, search terms used)

CNTXT, ENTXTC, VEN, DWPI, SIPOABS, CNABS, CNKI, NCBI, ISI web of knowledge: 申请人, 发明人, 脑膜炎奈瑟菌, 脑膜炎球菌, 脑脊髓膜炎, H结合蛋白, 疫苗, 序列检索, neisseria meningitidis, MenB, fHBP, GNA1870, GNA 1870, ORF2086, LP2086, vaccine.

**C. DOCUMENTS CONSIDERED TO BE RELEVANT**

| Category* | Citation of document, with indication, where appropriate, of the relevant passages | Relevant to claim No. |
|---|---|---|
| X | US 2012107339 A1 (GRANOFF DAN M. et al.) 03 May 2012 (2012-05-03)<br>the claims, and figures 15-16 | 1-15 |
| X | US 2013004530 A1 (POOLMAN JAN. et al.) 03 January 2013 (2013-01-03)<br>the claims | 1-15 |
| X | CN 103002910 A (GLAXOSMITHKLINE BIOLOGICALS S.A.) 27 March 2013 (2013-03-27)<br>the claims, and abstract | 1-15 |
| A | CN 110804101 A (SUZHOU WEICHAO BIOTECHNOLOGY CO., LTD.) 18 February 2020 (2020-02-18)<br>entire document | 1-15 |
| A | CN 110804102 A (SUZHOU WEICHAO BIOTECHNOLOGY CO., LTD.) 18 February 2020 (2020-02-18)<br>entire document | 1-15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 January 2025** | **23 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/129236**

**C. DOCUMENTS CONSIDERED TO BE RELEVANT**

| Category* | Citation of document, with indication, where appropriate, of the relevant passages | Relevant to claim No. |
|---|---|---|
| A | CN 110903400 A (SUZHOU WEICHAO BIOTECHNOLOGY CO., LTD.) 24 March 2020 (2020-03-24) entire document | 1-15 |

Form PCT/ISA/210 (second sheet) (July 2022)

**INTERNATIONAL SEARCH REPORT**

International application No.
**PCT/CN2024/129236**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/129236**

| Patent document cited in search report | | | Publication date (day/month/year) | Patent family member(s) | | | Publication date (day/month/year) |
|---|---|---|---|---|---|---|---|
| US | 2012107339 | A1 | 03 May 2012 | AU | 2009223613 | A1 | 17 September 2009 |
| | | | | AU | 2009223613 | B2 | 25 September 2014 |
| | | | | ES | 2557282 | T3 | 25 January 2016 |
| | | | | WO | 2009114485 | A2 | 17 September 2009 |
| | | | | WO | 2009114485 | A3 | 22 April 2010 |
| | | | | US | 9511131 | B2 | 06 December 2016 |
| | | | | EP | 2265640 | A2 | 29 December 2010 |
| | | | | EP | 2265640 | B1 | 04 November 2015 |
| | | | | EP | 2631245 | A1 | 28 August 2013 |
| | | | | CA | 2717870 | A1 | 17 September 2009 |
| US | 2013004530 | A1 | 03 January 2013 | | None | | |
| CN | 103002910 | A | 27 March 2013 | EP | 2544713 | A1 | 16 January 2013 |
| | | | | EP | 2544714 | A1 | 16 January 2013 |
| | | | | BR | 112012022676 | A2 | 24 September 2019 |
| | | | | US | 2017209562 | A1 | 27 July 2017 |
| | | | | US | 2013011429 | A1 | 10 January 2013 |
| | | | | JP | 2013521327 | A | 10 June 2013 |
| | | | | JP | 2013521770 | A | 13 June 2013 |
| | | | | US | 2013045231 | A1 | 21 February 2013 |
| | | | | US | 9567377 | B2 | 14 February 2017 |
| | | | | JP | 2017114874 | A | 29 June 2017 |
| | | | | US | 2013004530 | A1 | 03 January 2013 |
| | | | | WO | 2011110634 | A1 | 15 September 2011 |
| | | | | EP | 2544712 | A1 | 16 January 2013 |
| | | | | WO | 2011110635 | A1 | 15 September 2011 |
| | | | | WO | 2011110636 | A1 | 15 September 2011 |
| | | | | CA | 2792683 | A1 | 15 September 2011 |
| | | | | CA | 2792687 | A1 | 15 September 2011 |
| | | | | JP | 2013521326 | A | 10 June 2013 |
| | | | | CA | 2792689 | A1 | 15 September 2011 |
| | | | | BR | 112012022669 | A2 | 14 February 2017 |
| | | | | BR | 112012022688 | A2 | 22 May 2018 |
| CN | 110804101 | A | 18 February 2020 | | None | | |
| CN | 110804102 | A | 18 February 2020 | | None | | |
| CN | 110903400 | A | 24 March 2020 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9266942 B2

### Non-patent literature cited in the description

- *Computational and Structural Biotechnology Journal*, 18 April 2022, vol. 20, 2070-2081